# FASCICULE DE BREVET EUROPEEN

(11) **EP 2 961 423 B1**
(45) Date de publication et mention de la délivrance du brevet: **26.07.2017**
(21) Numéro de dépôt: 14706650.0
(22) Date de dépôt: 26.02.2014
(51) Int. Cl.: A61K 39/00, A61K 47/14, A61K 9/107

(54) **COMPOSITION IMMUNOGÈNE SOUS FORME D'ÉMULSION**
IMMUNOGENE ZUSAMMENSETZUNG IN EMULSIONSFORM
IMMUNOGENIC COMPOSITION IN EMULSION FORM

(30) Priorité: 26.02.2013 FR 1351687
(43) Date de publication de la demande: 06.01.2016
(73) Titulaire: Commissariat à l'Energie Atomique et aux Energies Alternatives, 75015 Paris (FR)
(72) Inventeur: NAVARRO Y GARCIA, Fabrice, F-38600 Fontaine (FR); COURANT, Thomas, F-38240 Meylan (FR); TEXIER-NOGUES, Isabelle, F-38000 Grenoble (FR); MARCHE, Patrice, F-38240 Meylan (FR)
(74) Mandataire: Lavoix
(86) Numéro de dépôt international: PCT/EP2014/053772
(87) Numéro de publication internationale: WO 2014/131809

(56) Documents cités:
- WO-A1-2010/018223
- WO-A2-2007/015167
- US-A- 6 080 725
- ALMEIDA ANTONIO J ET AL: "Peptide-loaded solid lipid nanoparticles (SLN): Influence of production parameters", INTERNATIONAL JOURNAL OF PHARMACEUTICS (AMSTERDAM), vol. 149, no. 2, 1997, pages 255-265, XP002715515, ISSN: 0378-5173 cité dans la demande

## Description

La présente invention concerne une composition immunogène sous forme d'émulsion comprenant une phase aqueuse continue et une phase lipidique dispersée sous forme de gouttelettes et comprenant un antigène lié de façon covalente auxdites gouttelettes, son procédé de préparation et ses utilisations, notamment pour la production d'anticorps, en tant que médicament ou dans une méthode d'immunisation.

L'utilisation d'émulsions ou de nanoparticules lipidiques solides pour la délivrance d'agents thérapeutiques est connue de la littérature.

En particulier, la demande de brevet WO 2010/018223 décrit l'utilisation d'une formulation sous forme de nanoémulsion, comprenant une phase aqueuse continue et au moins une phase dispersée comprenant un lipide amphiphile, un lipide solubilisant, un agent thérapeutique et un co-tensioactif comprenant au moins une chaîne composée de motifs d'oxyde d'alkylène, pour la délivrance d'un agent thérapeutique amphiphile ou lipophile. L'agent thérapeutique peut éventuellement être greffé au co-tensioactif. L'agent thérapeutique peut notamment être un agent immunologique ou un vaccin. Un mode de réalisation décrivant spécifiquement l'utilisation d'un agent immunologique greffé au co-tensioactif ou d'un vaccin greffé au co-tensioactif n'est toutefois pas décrit. De plus, les présents inventeurs ont mis en évidence que toutes les formulations décrites dans WO 2010/018223 ne peuvent pas être adaptées facilement lorsque l'on souhaite greffer de façon covalente un antigène aux gouttelettes des émulsions, un tel greffage ne pouvant être effectué que dans des conditions spécifiques, en particulier avec un rapport molaire (co-tensioactif greffé par l'antigène) / (co-tensioactif greffé par l'antigène + co-tensioactif libre non greffé) spécifique.

Almeida et al. (Int. J. Pharm. 149 (1997) 255-265) suggère que les nanoparticules lipidiques solides pourraient être une bonne alternative comme vecteur d'antigènes pour la délivrance de vaccins. Pour étudier la faisabilité d'incorporer des antigènes de type protéiques, ils ont incorporés une protéine modèle (lysozyme) dans des nanoparticules lipidiques solides de 600 nm comprenant notamment des lipides solubilisants de type Witepsol E 85 et/ou Softisan 142, des lipides amphiphiles de type Tween 80 et des co-tensioactifs de type Superpolystate ou Poloxamer 182 ou 188.

Müller et al. (Eur. J. Pharm. Biopharm. 50 (2000) 161-177) est une revue sur l'utilisation de nanoparticules lipidiques solides pour la délivrance contrôlée d'agents thérapeutiques et comme adjuvant pour des vaccins.

Saraf et al. (Vaccine 24 (2006) 45-56) décrit une émulsion double eau dans l'huile dans l'eau de diamètre supérieur à 1µm et comprenant :
- un antigène de surface de l'hépatite B, soluble dans l'eau, et situé dans la phase aqueuse interne de l'émulsion double,
- de la lécithine de soja située dans la phase huileuse,
- éventuellement de la stéarylamine en tant que lipide cationique.

De Temmerman et al. (Drug Discovery Today 16 (2011) 13/14, 569-582) et Krishmamachari et al. (Parm. Res. 28 (2011) 215-236) sont des revues rapportant que des nanoémulsions peuvent encapsuler des antigènes et servir de vecteurs pour les vaccins. Dans la seconde publication, il est fait référence aux travaux de :
- Shi et al. qui ont préparé une nanoémulsion de 20-30 nm de diamètre et comprenant du CpG et l'antigène MG7, du Span 80 et du Tween 80 à titre de lipides amphiphiles et de l'huile de soja.
- Wei et al. qui ont préparé une nanoémulsion de 20 nm de diamètre et comprenant des antigènes associés aux tumeurs (TAA), MAGE-1 et/ou MAGE-3, une protéine de choc thermique HSP70, une entérotoxine staphylococcique A (SEA), du pluronic 88 en tant que co-tensioactif, du Span 20 comme lipide amphiphile et de l'huile de soja.

Dans ces articles, l'antigène (ou la protéine modèle dans Almeida et al.) est encapsulé dans les gouttelettes de l'émulsion et n'est pas lié de façon covalente aux gouttelettes de l'émulsion.

Or, une émulsion huile dans l'eau dont les gouttelettes de phase dispersée comprennent un antigène encapsulé peut :
- être difficile à préparer. En particulier, seuls des antigènes amphiphiles ou lipophiles peuvent être encapsulés facilement dans des gouttelettes, les antigènes hydrophiles nécessitant la réalisation d'une double émulsion avec risque de fuite vers la phase aqueuse continue. Il peut également être nécessaire d'adapter les composants lipidiques de l'émulsion pour chaque antigène que l'on souhaite encapsulé, ce qui est coûteux et est un frein à une utilisation industrielle,
- être difficilement utilisable pour la production d'anticorps, en tant que médicament ou dans une méthode d'immunisation, notamment à cause de problèmes de « burst release » difficilement contrôlables pour les antigènes hydrophiles (on entend par burst release » qu'une fraction non négligeable de l'antigène est libéré des gouttelettes par simple diffusion dans un milieu biologique) (Demento, Biomaterials, 33 (2012) 4957-4964).

Par conséquent, il existe un besoin de fournir des compositions immunogènes alternatives à celles existantes, et présentant au moins l'un des avantages suivants :
- pouvoir être facilement utilisées pour la production d'anticorps, en tant que médicament ou dans une méthode d'immunisation,
- être stables au stockage, notamment en évitant la fuite de l'antigène hors des gouttelettes,
- pouvoir être préparées facilement, notamment en utilisant des méthodes industrielles et sans qu'il soit nécessaire d'adapter le procédé de préparation et les composants de l'émulsion pour chaque antigène.
- posséder une petite taille (diamètre hydrodynamique < 200 nm) pour faciliter la capture cellulaire par les cellules immunes et leur drainage lymphatique (Bachmann et al., Nature Reviews Immunology, 2010, 10, 787).

### [Composition immunogène]

Selon un premier objet, l'invention concerne une composition immunogène comprenant une phase aqueuse continue et une phase dispersée sous forme de gouttelettes et comprenant :
- un lipide amphiphile,
- un lipide solubilisant comprenant au moins un glycéride d'acide gras,
- un co-tensioactif comprenant au moins une chaine composée de motifs d'oxyde d'alkylène,
- un tensioactif porteur d'un antigène de formule (I) suivante :

   (L₁-X₁-H₁-Y₁)ᵥ-G-Z₁-Ag (I),

   dans laquelle :
   - L₁ représente un groupe lipophile,
   - X₁, Y₁, Z₁ et G représentent indépendamment un groupe de liaison,
   - H₁ représente un groupe hydrophile comprenant une chaîne polyalcoxylée,
   - v est un nombre entier de 1 à 8, et
   - Ag représente un antigène,
dans laquelle le rapport molaire du tensioactif porteur d'un antigène de formule (I) sur la somme du co-tensioactif et du tensioactif porteur d'un antigène de formule (I) est de 0,01% à 5%.

Les inventeurs ont montré que la vectorisation d'un antigène par des gouttelettes selon l'invention permettait d'augmenter et d'améliorer la réponse immune dirigée contre ledit antigène. La réponse immune obtenue après injection d'un antigène vectorisé par les gouttelettes selon l'invention est notamment significativement plus importante et plus homogène que la réponse immune obtenue après injection de l'antigène seul. La composition immunogène peut donc être considérée comme une formulation adjuvante d'un antigène. L'utilisation des gouttelettes selon l'invention est donc particulièrement adaptée pour produire efficacement des anticorps dirigés contre un antigène, ainsi que dans le traitement de pathologies infectieuses, d'allergie ou de cancers, selon la nature de l'antigène inclus dans la composition immunogène.

La composition immunogène se présente sous forme d'une émulsion huile dans l'eau, de préférence une nanoémulsion huile dans l'eau. L'émulsion peut être simple ou multiple, notamment en comportant dans la phase dispersée une seconde phase aqueuse.

### • Définitions

Au sens de la présente demande, l'expression « phase dispersée » signifie les gouttelettes comprenant l'éventuelle huile/le lipide solubilisant/ le lipide amphiphile/le co-tensioactif/ l'éventuel agent d'intérêt lipophile/le tensioactif porteur d'un antigène de formule (I)/l'éventuel agent immunostimulant/l'éventuel ligand biologique de ciblage (libre ou greffé au co-tensioactif)/l'éventuel tensioactif cationique. La phase dispersée est généralement exempte de phase aqueuse. La composition immunogène est typiquement exempte de liposomes.

Le terme « gouttelette » englobe à la fois les gouttelettes d'huile liquide proprement dites ainsi que les particules solides issues d'émulsions de type huile-dans-eau dans lesquelles la phase dispersée est solide. On utilise également l'abréviation LNP pour désigner les gouttelettes lorsque leur taille est nanométrique (pour « lipidic nanoparticule »).

Les gouttelettes de la composition immunogène sont avantageusement monodisperses. L'écart type entre les diamètres minimum et maximum des gouttelettes par rapport au diamètre moyen est généralement inférieur ou égal à 30%, de préférence 20%. Le diamètre moyen des gouttelettes de la phase dispersée est de préférence de 20 à 200 nm, notamment de 40 à 150 nm et en particulier de 50 à 150 nm. Ces diamètres sont mesurés par diffusion quasi élastique de la lumière. On peut également obtenir la taille de gouttelettes par microscopie électronique en transmission (TEM), par cryomicroscopie électronique en transmission (cryoTEM) ou encore par microscopie à force atomique (AFM). Des diamètres inférieurs à 20 nm et supérieurs à 200 nm sont difficiles à atteindre en pratique.

Le terme « lipide » désigne dans le cadre de cet exposé l'ensemble des corps gras ou des substances contenant des acides gras présents dans les graisses d'origine animales et dans les huiles végétales. Ce sont de molécules hydrophobes ou amphiphiles principalement constituées de carbone, d'hydrogène et d'oxygène et ayant une densité inférieure à celle de l'eau. Les lipides peuvent être à l'état solide à température ambiante (25°C), comme dans les cires, ou liquide, comme dans les huiles.

Le terme « amphiphile » désigne une molécule possédant une partie hydrophobe et une partie hydrophile, par exemple une partie apolaire hydrophobe et une partie polaire hydrophile.

Le terme « phospholipide » vise des lipides possédant un groupe phosphate, notamment les phosphoglycérides. Le plus souvent, les phospholipides comportent une extrémité hydrophile formée par le groupe phosphate éventuellement substitué et deux extrémités hydrophobes formées par des chaînes d'acides gras. Parmi les phospholipides, on peut citer en particulier la phosphatidylcholine, la phosphatidyl éthanolamine, la phosphatidyl inositol, la phosphatidyl sérine et la sphingomyéline.

Le terme « lécithine » désigne la phosphatidylcholine, c'est-à-dire un lipide formé à partir d'une choline, d'un phosphate, d'un glycérol et de deux acides gras. Il couvre de manière plus large les phospholipides extraits du vivant, d'origine végétale ou animale, dans la mesure où ils sont majoritairement constitués de phosphatidylcholine. Ces lécithines constituent généralement des mélanges de lécithines portant différents acides gras.

On entend par le terme « tensioactif » des composés à structure amphiphile qui leur confère une affinité particulière pour les interfaces de type huile/eau et eau/huile ce qui leur donne la capacité d'abaisser l'énergie libre de ces interfaces et de stabiliser des systèmes dispersés.

On entend par le terme « co-tensioactif » un tensioactif agissant en plus d'un premier tensioactif (à savoir le lipide amphiphile) pour abaisser davantage l'énergie de l'interface.

On entend par agent d'intérêt « lipophile », un agent d'intérêt qui se situe majoritairement, de préférence totalement, dans la phase dispersée, à l'intérieur ou en surface des gouttelettes. Un agent d'intérêt lipophile a des affinités pour des composés huileux (graisses, huiles, cires...) et solvants apolaires (toluène, hexane...). Les forces permettant la solubilisation de l'agent d'intérêt lipophile sont majoritairement des forces de London (interactions de Van der Waals). Un agent d'intérêt lipophile présente un coefficient de partage huile/eau élevé.

On entend par agent d'intérêt « hydrophile », un agent d'intérêt qui se situe majoritairement, de préférence totalement, dans la phase aqueuse continue. Sa solubilité dans l'eau est généralement supérieure à 1% en poids. La solubilisation dans l'eau des agents d'intérêt hydrophiles provient généralement de liaisons hydrogène et/ou ioniques entre les agents d'intérêt hydrophiles et l'eau.

Par « composition immunogène », on entend une composition susceptible d'être administrée à l'homme ou à l'animal afin d'induire une réponse immunitaire. Ladite réponse immunitaire peut être une réponse immunitaire humorale, i.e. une réponse immunitaire qui se traduit par une production d'anticorps neutralisants, ou une réponse immunitaire cellulaire cytotoxique, i.e. une réponse immunitaire qui se traduit par une activation de certaines cellules, notamment les cellules présentatrices d'antigènes (par exemple les cellules dendritiques), les lymphocytes T, les lymphocytes B, les lymphocytes NK (pour « Natural Killer » en anglais).

Par « antigène » (« Ag » dans la présente demande), on entend tout antigène (y compris un épitope spécifique) susceptible d'être utilisé dans un vaccin, à savoir toute molécule qui peut être spécifiquement reconnue par les cellules du système immunitaire, telles que les cellules dendritiques, les cellules B, et/ou les cellules T (Pulendran et al., Nature Immunology, 2011, 12, 509-517).

Dans certains modes de réalisation, l'antigène est un vecteur comprenant un polynucléotide codant un polypeptide antigénique, ledit polynucléotide étant lié de manière opérationnelle à une ou plusieurs séquence(s) régulatrice(s) qui permettent la régulation de l'expression dudit polynucléotide.

Dans certains modes de réalisation, l'antigène est un allergène. Des exemples d'allergènes peuvent être, à titre non limitatif, des allergènes de pollen (d'arbres, de graminées, etc.), des allergènes d'acarien (de la poussière domestique ou de stockage), des allergènes d'insecte (hyménoptères, de blattes, etc.), des allergènes d'animaux (de chien, de chat, de cheval, de rat, de souris, etc.), des allergènes de moisissure et des allergènes alimentaires. Les allergènes alimentaires peuvent provenir du lait, des oeufs, des légumes (dont cacahuète et soja), des noix et noisettes, du blé, de crustacés, de poissons et de mollusques et des produits qui en sont dérivés. En particulier, les allergènes alimentaires peuvent être l'ovalbumine ou le gluten.

Dans certains modes de réalisation, l'antigène utilisé dans l'invention peut également être dérivé de n'importe quel organisme vivant ou non vivant ; de fragments cellulaires ; d'anatoxine. L'antigène peut également être dérivé d'un microorganisme naturel ou atténué, tel qu'un virus, une bactérie, un parasite ou une levure.

Dans certains modes de réalisation, l'antigène peut être par exemple une portion d'une molécule antigénique, ou une molécule synthétique ou une molécule obtenue par les technologies recombinantes.

Dans certains modes de réalisation, l'antigène est un polypeptide, un carbohydrate ou un lipide.

Des exemples non limitatifs d'antigènes sont des antigènes dérivés :
(i) de virus, tels que des antigènes dérivés du virus de l'immunodéficience acquise humaine de type 1 ou 2 (VIH ou HIV pour « Human immunodeficiency virus » en anglais) (e.g. tat, nef, gp120, gp160, gp40, p24, gag, env, vif, vpr, vpu, rev) ; du virus de l'herpès humain de type 1 ou 2 (HSV pour « Herpès Simplex Virus » en anglais) (e.g. gH, gL, gM, gB, gC, gK, gE, gD, ICP27 , ICP 47, IC P 4, ICP36 from HSV1 or HSV2) ; du cytomegalovirus tells que gB, du virus Epstein Barr (e.g. gp350) ; du virus de la varicelle (e.g. gpl, II, III et 1 E63) ; ou du virus de l'hépatite A, B (e.g. l'antigène de surface de l'hépatite B (« Hepatitis B Surface antigen » en anglais) ou l'antigène nucléocapsidique de l'hépatite (« Hepatitis core antigen » en anglais)) ; des paramyxoviruses tels que le virus respiratoire syncytial (« Respiratory Syncytial virus » en anglais), le parainfluenza virus, le virus de la rougeole, ou le virus des oreillons ; des papilloma virus (e.g. HPV6, 11, 16, 18, eg L1, L2, EI, E2, E3, E4, E5, E6, E7) ; des flavivirus tells que le virus de la fièvre jaune, le virus de la dengue, le virus de l'encéphalite de Saint-Louis, le virus de l'encéphalite japonaise ; le virus influenza (e.g. les proteins HA, NP, NA, ou M);
(ii) de bactéries, tels que des antigènes dérivés de bactéries du genre *Neisseria,* incluant *N. gonorrhea* et *N. meningitidis* (e.g, les proteins de liaison à la transferrine, les proteins de liaison à la lactoferrine, PilC, les adhésines); du genre *Streptococcus,* incluant *S. pyogenes* (e.g. les protéines M, la protease C5A), *S*. *pneumoniae* (e.g. PsaA, PspA, streptolysine, les protéines de liaison à la choline), *S. agalactiae* et *S. mutans;* du genre *Haemophilus,* incluant *H. ducreyi, H. influenzae* de type B (e.g. PRP), *H. influenzae* non typable (e.g. OMP26, les adhésines de haut poids moléculaire, P5, P6, protéine D, lipoprotéine D, la fibrine); du genre *Moraxella,* incluant *M catarrhalis* (e.g. les adhésines de haut et bas poids moléculaire et les invasines); du genre *Bordetella,* incluant *B. pertussis* (e.g. pertactine, la toxine pertussique, l'hemagglutinine fimlenteuse, l'adénylate cyclase), *B. parapertussis* et *B*. *bronchiseptica;* du genre *Mycobacterium,* incluant *M*. *tuberculosis* (e.g. ESAT6, l'antigène 85A, -B ou -C, MPT 44, MPT59, MPT45, HSP10, HSP65, HSP70, HSP75, HSP90, PPD 19kDa [Rv3763], PPD 38kDa [Rv0934]), *M. bovis, M. leprae, M. avium, M. paratuberculosis* et *M. smegmatis;* du genre *Legionella,* incluant *L. pneumophila;* du genre *Escherichia,* incluant *E. coli* entérotoxique (e.g. les facteurs de colonization, la toxine thermolabile ou la toxine thermostable), *E. coli* entérohémorragique, *E. coli* entéropathogénique (e.g. la vérotoxine); du genre *Vibrio,* incluant *V. cholera* (la toxine cholérique); du genre *Shigella,* incluant *S. sonnei, S. dysenteriae* et *S. flexnerii*; du genre *Yersinia,* incluant *Y. enterocolitica* (e.g. la protéine Yop), *Y. pestis, Y. pseudotuberculosis;* du genre *Campylobacter,* incluant *C. jejuni* (e.g. les toxines, adhésines et invasines) et *C. coli;* du genre *Salmonella,* incluant *S. typhi, S. paratyphi, S. choleraesuis, S. enteritidis;* du genre *Listeria,* incluant *L. monocytogenes;* du genre *Helicobacter,* incluant *H. pylon* (e.g. l'uréase, la catalase, la toxine vacuolaire); du genre *Pseudomonas,* incluant *P. aeruginosa;* du genre *Staphylococcus,* incluant *S. aureus, S. epidermidis;* du genre *Enterococcus,* incluant *E. faecalis, E. faecium;* du genre *Clostridium,* incluant *C. tetani* (e.g. la toxine tétanique), *C. botulinum* (e.g. la toxine botulique), *C. difficile* (e.g. les toxines A et B); du genre *Bacillus,* incluant *B*. *anthracis;* du genre *Corynebacterium,* incluant *C. diphtheriae* (e.g. la toxine diphtérique); du genre *Borrelia,* incluant *B*. *burgdorferi* (e.g. OspA, OspC, DbpA, DbpB), *B. garinii* (e.g. OspA, OspC, DbpA, DbpB), *B. afzelii* (e.g. OspA, OspC, DbpA, DbpB), *B. andersonii* (e.g. OspA, OspC, DbpA, DbpB), *B. hermsii;* du genre *Ehrlichia,* incluant *E. equi et* l'agent de l'Ehrlichiose granulocytaire humaine; du genre *Rickettsia,* incluant *R. rickettsii;* du genre *Chlamydia,* incluant *C. trachomatis* (e.g. MOMP, les protéines de liaison à l'héparine), *C. pneumoniae* (e.g. MOMP, les protéines de liaison à l'héparine), *C. psittaci;* du genre *Leptospira,* incluant *L. interrogans;* du genre *Treponema,* incluant *T. pallidum* (e.g. les protéines rares de la membrane externe), *T. denticola, T. hyodysenteriae;*
(iii) de parasites, tels que des antigènes dérivés de parasites du genre *Plasmodium,* incluant *P. falciparum* (e.g. RTS,S et TRAP); du genre *Toxoplasma,* incluant *T. gondii* (e.g. SAG2, SAG3, Tg34); du genre *Entamoeba,* incluant *E. histolytica;* du genre *Babesia,* incluant *B. microti;* du genre *Trypanosoma,* incluant *T. cruzi;* du genre *Giardia,* incluant *G. lamblia;* du genre *Leishmania,* incluant *L. major;* du genre *Pneumocystis,* incluant *P. carinii;* du genre *Trichomonas,* incluant *T. vaginalis;* du genre *Schisostoma,* incluant *S. mansoni,* ou
(iv) de levures du genre *Candida,* incluant *C. albicans;* du genre *Cryptococcus,* incluant *C*. *neoformans.*

Dans certains modes de réalisation, l'antigène est un antigène tumoral et peut être utile pour le traitement immunothérapeutique de cancers. Les antigènes tumoraux peuvent dériver de cancer de la prostate, du sein, du colon, du poumon, du foie, du pancréas, du rein, de la vessie, de mélanome, de carcinome, de sarcome. Des exemples non limitatifs d'antigènes tumoraux dérivé de mélanome, de carcinome (poumon, vessie), ou de sarcome sont MAGE 1, 3 et MAGE 4, PRAME, BAGE, Lage, SAGE, HAGE ou GAGE. Des exemples non limitatifs d'antigènes dérivés de cancer de la prostate sont l'antigène spécifique de la prostate (PSA pour « Prostate specific antigen » en anglais), PAP, PSCA, PSMA, P501S ou la prostase. Des exemples non limitatifs d'antigènes dérivés de cancer du sein sont Muc-1, Muc-2, EpCAM, her 2/ Neu, la mammaglobine. D'autres exemples d'antigènes tumoraux utiles dans le contexte de la présente invention sont Plu-1, HASH-1, HasH-2, Cripto, Criptin, tyrosinase, survivine.

Dans certains modes de réalisation, l'antigène comprend ou consiste en un fragment d'au moins 6, 7, 8, 10, 15, 20, 30, 40, 50, 75, 100, 150, 200, 300, 400, 500 acides aminés contiguës d'un antigène tel que défini ci-dessus.

Dans certains modes de réalisation, l'antigène est une protéine de fusion comprenant ou consistant en au moins 2 antigènes ou fragments d'antigène tels que définis ci-dessus.

On entend par « ligand biologique de ciblage » une molécule permettant d'augmenter la reconnaissance spécifique d'une cellule ou d'un organe que l'on souhaite cibler, notamment d'une cellule immunitaire, comme par exemple les lymphocytes T, les lymphocytes B, les lymphocytes NK, les cellules dendritiques, les macrophages, et de favoriser l'internalisation des gouttelettes par les cellules cibles. Le ligand biologique de ciblage peut notamment être choisi parmi les anticorps, peptides, saccharides, aptamères, oligonucléotides ou les composés comme l'acide folique.
De préférence, le ligand biologique de ciblage permet de cibler les cellules dendritiques. Selon ce mode de réalisation, le ligand biologique de ciblage peut donc être une molécule mannosylée, tel qu'un peptide ou un lipide mannosylé, un polymère de mannose, tel que le mannan, qui peut être reconnu par les récepteurs au mannose présents à la surface des cellules dendritiques, et/ou un anticorps, fragment d'anticorps ou un ligand reconnaissant spécifiquement les cellules dendritiques tel que anti-DC-SIGN anti-DEC-205, anti-CD-207.

On entend par « agent immunostimulant », aussi dénommé « adjuvant », une substance capable d'améliorer, ou d'augmenter, la réponse immune induite par l'antigène tel que défini ci-dessus. Des agents immunostimulants appropriés incluent des sels d'aluminium, des sels de calcium, magnésium, fer ou zinc, la saponine, le lipide A (aussi connu sous le nom de MPLA pour « Monophosphoryl Lipid A » en anglais) ou l'un de ses dérivés, un oligonucléotide immunostimulant, un alkyl phosphate glucosamide, des cytokines, des chimiokines ou une combinaison de ces composés. Des exemples de saponines sont Quil A et de ses fragments purifiés sont QS7 et QS21. Des exemples de cytokines sont l'interleukine 1 beta (IL-1β), l'interleukine 6 (IL-6), l'interféron gamma (IFN-γ), le facteur de nécrose alpha (« Tumor Necrosis Factor alpha » (TNF-α) en anglais). Des exemples de chimiokines sont MCP-1 (Monocyte chimoattractant protein 1, aussi connue sous la dénomination CCL-2), MIP-1 alpha (aussi connue sous la dénomination CCL-3) et MIP-1 beta (aussi connue sous la dénomination CCL-4).

On entend par le terme « acide gras » désigner des acides carboxyliques aliphatiques présentant une chaîne carbonée d'au moins 4 atomes de carbone. Les acides gras naturels possèdent une chaîne carbonée de 4 à 28 atomes de carbone (généralement un nombre pair). On parle d'acide gras à longue chaîne pour une longueur de 14 à 22 carbones et à très longue chaîne s'il y a plus de 22 carbones.

On entend par le terme « chaîne hydrocarbonée » désigner une chaîne composée d'atomes de carbone et d'hydrogène, saturée ou insaturée (double ou triple liaison). Les chaînes hydrocarbonées préférées sont les alkyle ou alcényle.

On entend par le terme « alkylène » désigner un groupe divalent aliphatique hydrocarboné, saturé, linéaire ou ramifié, de préférence linéaire.

Par « ester activé », on entend un groupe de formule -CO-LG, par « carbonate activé », on entend un groupe de formule -O-CO-LG, par « carbamate activé », on entend un groupe de formule -NH-CO-LG, où LG est un bon groupe partant notamment choisi parmi un brome, un chlore, un imidazolyle, un pentafluorophénolate, un pentachlorophénolate, un 2,4,5-trichlorophénolate, 2,4,6-trichlorophénolate, un groupe -O-succinimidyle, -O-benzotriazolyle, -O-(7-aza-benzotriazolyle) et -O-(4-nitrophényle).

Les modes de réalisation décrits pour chacun des composants de la composition immunogène peuvent bien sûr être combinés entre eux. De plus, lorsqu'un composant est constitué de plusieurs radicaux (par exemple le tensioactif porteur d'un antigène de formule (I) est composé de différents radicaux L₁-, -X₁-, -H₁-, -Y₁, -G-, -Z₁- et Ag), les différents modes de réalisation de chacun des radicaux peuvent bien sûr être combinés entre eux.

### • Tensioactif porteur d'un antigène de formule (I)

La composition immunogène selon l'invention comprend un tensioactif de formule (I) qui est porteur d'un antigène. Ce tensioactif permet de lier de façon covalente l'antigène Ag aux gouttelettes.

Le tensioactif de formule (I) se situe dans la couronne des gouttelettes de l'émulsion, le(s) groupe(s) L₁ étant dirigés vers l'intérieur des gouttelettes alors que l'antigène Ag est dirigé vers l'extérieur des gouttelettes, vers la phase aqueuse continue.

Le groupe X₁ est un groupe de liaison liant les groupes lipophile et hydrophobe. Le groupe G est un groupe de liaison entre les parties [lipophile-hydrophile] et l'antigène. Le groupe Y₁ est un groupe de liaison liant le groupe G aux parties [lipophile-hydrophile].

Dans un mode de réalisation, dans la formule (I) susmentionnée :
- L₁ est choisi parmi :
   - un groupe R ou R-(C=O)-, où R représente une chaîne hydrocarbonée linéaire comprenant de 7 à 23 atomes de carbone, de préférence de 11 à 23 atomes de carbone,
   - un ester ou un amide d'acides gras comprenant de 8 à 24 atomes de carbone (de préférence de 12 à 24 atomes de carbone) et de phosphatidyléthanolamine, tel que le distéaryl phosphatidyléthanolamine (DSPE), et
   - un poly(oxyde de propylène), et/ou
- X₁, Y₁ et Z₁ sont indépendamment choisis parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH,
   - un groupe Alk étant un alkylène comprenant de 1 à 8 atomes de carbone comprenant éventuellement un cycle (par exemple un radical 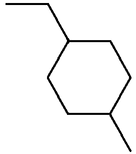 ), et
   - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents, et/ou
- H₁ est choisi parmi un poly(oxyde d'éthylène) comprenant typiquement de 3 à 500 unités oxyde d'éthylène, de préférence de 20 à 200 unités oxyde d'éthylène, et/ou
- G comprend au moins un groupe G' ayant l'une des formules suivantes (le(s) groupe(s) Y₁ et Z₁ pouvant être reliés à gauche ou à droite des formules décrites ci-dessous, par exemple, lorsque G est un groupe G' de formule (XI), le tensioactif de formule (I) peut avoir la formule la formule L₁-X₁-H₁-Y₁-CONH-Z₁-Ag ou L₁-X₁-H₁-Y₁-NHCO-Z₁-Ag) : 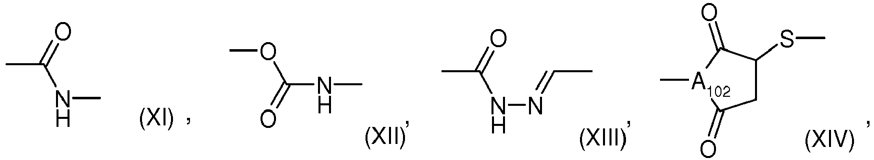 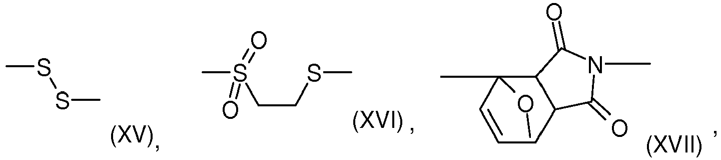 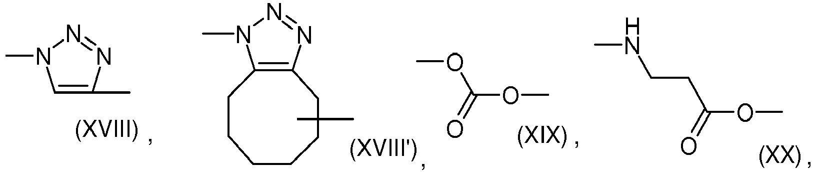 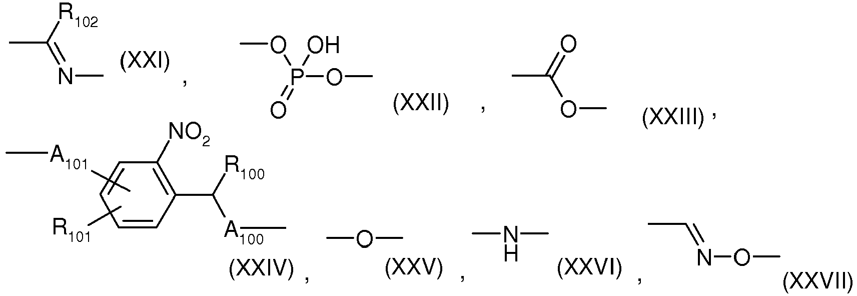 où A₁₀₂ représente CH ou N, R₁₀₂ représente H ou une chaîne hydrocarbonée linéaire comprenant de 1 à 6 atomes de carbone, A₁₀₁ représente -O-, -NH-(CO)- ou -O-(CO)-, R₁₀₀ représente H ou un méthyle, A₁₀₀ représente -O- ou -NH- et R₁₀₁ représente H, Me ou -OMe.
Par la formule 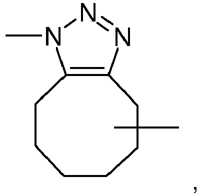 on entend que le groupe Z₁ ou le groupe Y₁ peut être relié à n'importe lequel des six atomes du cyclooctyle et par la formule 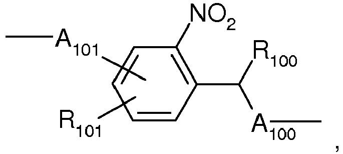 on entend que les groupe A₁₀₁ et R₁₀₁ peuvent être relié à n'importe lequel des quatre atomes du phényle.

Notamment, v représente 1 ou 2. v représente de préférence 1.

Le groupe G peut comprendre un ou plusieurs des groupes G' définis ci-dessus.

Ainsi, dans un premier mode de réalisation, le groupe G est constitué d'un groupe G'. Dans ce mode de réalisation, dans la formule (I), v représente 1.

Dans un second mode de réalisation, le groupe G répond à la formule -G'-Y₃-G'- dans laquelle :
- Y₃ représente un groupe de liaison, notamment choisi parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH,
   - un groupe Alk étant un alkylène comprenant de 1 à 6 atomes de carbone, et
   - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents
- chacun des G' représente indépendamment un groupe de formule (XI) à (XXVI) susmentionnés, et de préférence, les deux groupes G' de la formule -G'-Y₃-G'- sont identiques.
Ce mode de réalisation est particulièrement intéressant lorsque les deux groupes G' sont identiques et comprennent une fonction clivable. En effet, il suffit alors de cliver une seule des deux fonctions pour rompre les liaisons ovalentes entre les gouttelettes de la composition immunogène et l'antigène, ce qui améliore les chances de succès du clivage et donc la libération de l'antigène hors des gouttelettes après capture cellulaire.

Lorsque L₁ représente un groupe R-(C=O)-, où R représente une chaîne hydrocarbonée linéaire comprenant de 7 à 23 atomes de carbone, L₁ représente un groupe issu d'acide gras comprenant de 8 à 24 atomes de carbone.

Par «L₁ représente un ester ou un amide d'acides gras comprenant de 8 à 24 atomes de carbone et de phosphatidyléthanolamine», on entend qu'il représente un groupe de formule : 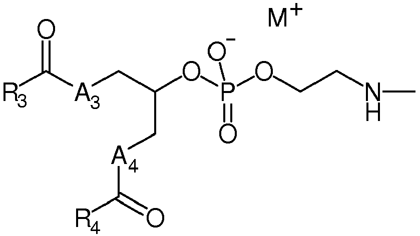 dans laquelle
- R₃ et R₄ représentent indépendamment une chaîne hydrocarbonée linéaire comprenant de 7 à 23 atomes de carbone, de préférence de 11 à 23 atomes de carbone,
- A₃ et A₄ représentent O ou NH, et
- M représente H ou un cation.

Dans un mode de réalisation, dans la formule (I) susmentionnée, le radical L₁-X₁-H₁- consiste en l'un des groupes de formules suivantes (le radical -Y₁-G-Z₁-Ag étant relié à droite des formules décrites ci-dessous): 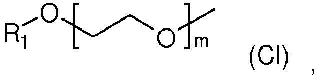 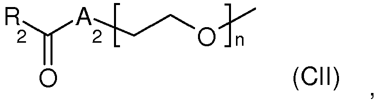 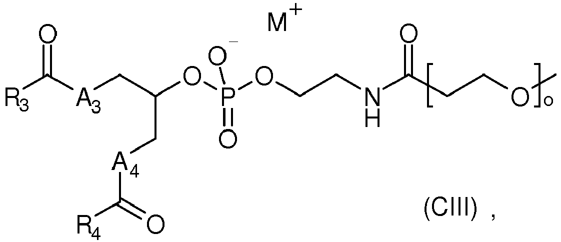 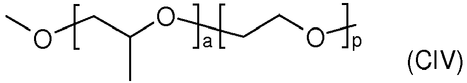 dans lesquelles :
- R₁, R₂, R₃ et R₄ représentent indépendamment une chaîne hydrocarbonée linéaire comprenant de 7 à 23 atomes de carbone, de préférence de 11 à 23 atomes de carbone,
- A₁, A₂, A₃ et A₄ représentent O ou NH,
- m, n, o et p représentent indépendamment des nombres entiers de 3 à 500, de préférence 20 à 200, et
- a représente un nombre entier de 20 à 120,
- M représente H ou un cation.

Le radical L₁-X₁-H₁- de formule (CII) est préféré. En effet, il est facile à préparer (notamment par formation d'un ester ou d'une amide entre un acide gras et un dérivé de poly(éthylène glycol)).

Le radical L₁-X₁-H₁- de formule (CII) avec A₂ représente NH sont particulièrement préférés, car les tensioactifs comprenant de tels radicaux permettent d'éviter la fuite des agents d'intérêts lipophiles et/ou ligand de ciblage et/ou agent immunostimulant éventuellement présents hors des gouttelettes de la composition immunogène plus efficacement que des tensioactifs comprenant un radical L₁-X₁-H₁- de formule (CII) avec A₂ représente O.

Dans un mode de réalisation, dans la formule (I) :
- v représente 1,
- L₁ est R-(C=O)-, où R représente une chaîne hydrocarbonée linéaire comprenant de 7 à 23 atomes de carbone, de préférence de 11 à 23 atomes de carbone,
- H₁ est un poly(oxyde d'éthylène) comprenant de 3 à 500 unités oxyde d'éthylène,
- X, représente -O- ou -NH-,
- G est constitué d'un groupe G' de formule (XIV) dans laquelle A₁₀₂ représente N,
- Y₁ représente -CH₂-CH₂-NH-CO-CH₂-CH₂- (groupe Alk-Z-Alk ci-dessus avec Alk représente -CH₂-CH₂- et Z représente -NH-(CO)-) et Z₁ représente 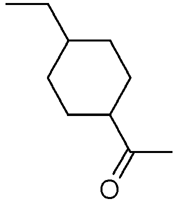 (groupe Alk-Z ci-dessus avec Alk représente 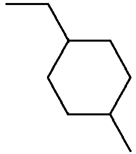 et Z représente (CO)),
   et le tensioactif de formule (I) de la composition immunogène a alors la formule (I') suivante : 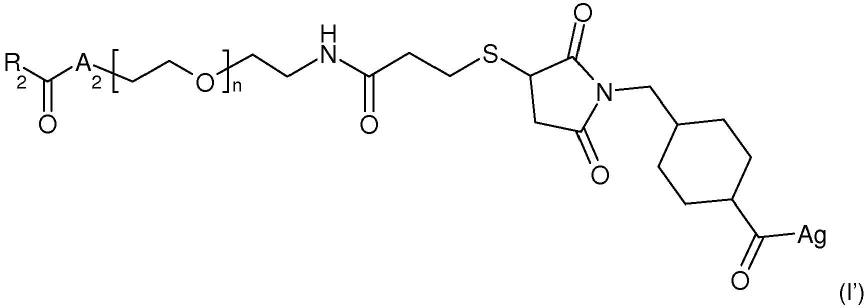 dans laquelle :
   - R₂ représente une chaîne hydrocarbonée linéaire comprenant de 7 à 23 atomes de carbone, notamment de 11 à 23 atomes de carbone, de préférence 17,
   - A₂ représente O ou NH, de préférence NH, et
   - n représente un nombre entier de 3 à 500, de préférence de 20 à 200, notamment 100,
   - Ag représente un antigène.

Dans un mode de réalisation, le groupe H₁ est choisi parmi un poly(oxyde d'éthylène) comprenant plus de 3 unités de poly(oxyde d'éthylène), voire plus de 20 unités, notamment plus de 50 (dans les formules susmentionnées, m, n, o ou p sont de préférence supérieurs à 3, voire 20, notamment plus de 50).

Dans un mode de réalisation, le groupe G du tensioactif de formule (I) de la composition immunogène comprend une fonction clivable, notamment chimiquement (lorsque le tensioactif de formule (I) est mis en contact avec un composé chimique capable de cliver la fonction du groupe G), électrochimiquement, à certains pH (pH basique ou acide), par des enzymes, par la lumière (lumière visible, ultraviolets ou infrarouge) et/ou au-delà de certaines températures. Généralement, le groupe G comprend alors un groupe G' comprenant une fonction clivable.

Ce mode de réalisation est intéressant car il peut permettre la délivrance de l'antigène Ag localisé à l'endroit souhaité où le stimulus chimique, électrochimique, de pH ou de température se produit. Par exemple, il est possible de délivrer l'antigène dans le compartiment intracellulaire des cellules cibles lorsque l'antigène est lié à la gouttelette par une liaison dithiol (-S-S-) (c'est-à-dire lorsque le groupe G comprend au moins un groupe G' comprenant un groupe (XV)). Une fois phagocyté, la particule porteuse d'antigène se retrouve dans l'endosome où la liaison (-S-S-) sera réduite par la gluthathione. L'antigène libre pourra alors s'échapper de l'endosome vers le cytosol pour y subir une présentation croisée, on parle alors d'échappement endosomal (Joffre et al., Nature Reviews Immunology 2012, 12, 557-569).

Par exemple :
- la fonction β-cétoaminoester du groupe G' de formule (XX) est clivable à pH acide (typiquement autour de 5),
- la fonction disulfure du groupe G' de formule (XV) est clivable aux ultraviolets, électrochimiquement, chimiquement (par exemple par mise en contact avec un agent réducteur, tel que la tris(2-carboxyéthyl)phosphine (TCEP) ou le dithiothréitol (DTT)), ou par des enzymes telles que des thioréductases,
- la fonction amide du groupe G' de formule (XI) est clivable par des enzymes telles que des protéases,
- la fonction phosphate du groupe G' de formule (XXII) est clivable par des enzymes telles que des phosphatases,
- la fonction imine des groupes G' de formules (XXI) et (XIII) sont clivables à pH acide ou au-delà de certaines températures,
- le cycle cyclohexène du groupe G' de formule (XVII) est clivable au-delà de certaines températures (par rétro Diels-Alder),
- la fonction carbonate du groupe G' de formule (XIX) et la fonction carbamate du groupe G de formule (XII) sont clivables à pH acide ou chimiquement (par exemple par réaction avec un agent nucléophile),
- la fonction orthonitrobenzyle du groupe G' de formule (XXIV) est clivable sous l'action de la lumière à 365 nm.

L'homme du métier, au vu de ses connaissances générales, sait quelles fonctions sont clivables et dans quelles conditions. Il est notamment à même de choisir la fonction du groupe G' du tensioactif de formule (I) pour qu'elle soit clivable dans les conditions rencontrées dans l'application souhaitée de la composition immunogène selon l'invention.

Dans un mode de réalisation, la composition immunogène selon l'invention comprend plusieurs tensioactifs de formule (I) (par exemple deux tensioactifs de formule (I)), chacun des tensioactifs de formule (I) étant porteur d'un antigène de nature différente. Certaines utilisations biologiques peuvent en effet nécessiter l'administration de plusieurs antigènes en même temps.

### • Lipide amphiphile

La composition immunogène comprend un lipide amphiphile à titre de tensioactif qui permet la formation des gouttelettes de la phase dispersée. La nature amphiphile du tensioactif assure la stabilisation des gouttelettes d'huile au sein de la phase continue aqueuse.

Les lipides amphiphiles comportent une partie hydrophile et une partie lipophile. Ils sont généralement choisis parmi les composés dont la partie lipophile comprend une chaîne saturée ou insaturée, linéaire ou ramifiée, ayant de 8 à 30 atomes de carbone. Ils peuvent être choisis parmi les phospholipides, les cholestérols, les lysolipides, les sphingomyélines, les tocophérols (non estérifiés), les glucolipides, stéarylamines, les cardiolipines d'origine naturelle ou synthétique ; les molécules composées d'un acide gras couplé à un groupement hydrophile par une fonction éther ou ester tels que les esters de sorbitan comme par exemple les monooléate et monolaurate de sorbitan vendus sous les dénominations Span^{®} par la société Sigma; les lipides polymérisés ; les lipides conjugués à de courtes chaînes d'oxyde de polyéthylène (PEG) tels que les tensioactifs non-ioniques vendus sous les dénominations commerciales Tween^{®} par la société ICI Americas, Inc. et Triton^{®} par la société Union Carbide Corp.; les esters de sucre tels que les mono- et di-laurate, mono- et di-palmitate, mono- et distéarate de saccharose; lesdits tensioactifs pouvant être utilisés seuls ou en mélanges.

Les phospholipides sont des lipides amphiphiles particulièrement préférés, notamment les phospholipides choisis parmi la phosphatidylcholine, la phosphatidyléthanolamine, la phosphatidylsérine, le phosphatidylglycérol, le phosphatidylinositol, le phosphatidyl-acide phosphatidique non-hydrogéné ou hydrogéné, notamment vendu par la société Lipoid.

La lécithine est le lipide amphiphile préféré.

Généralement, la phase dispersée comporte de 0,01 à 99% en poids, de préférence de 5 à 75% en poids, en particulier de 5 à 60% et tout particulièrement de 5 à 45% en poids de lipide amphiphile.

La quantité de lipide amphiphile contribue avantageusement à contrôler la taille des gouttelettes de la phase dispersée de la composition immunogène.

### • Lipide solubilisant

La composition immunogène comprend un lipide solubilisant, qui permet notamment :
- d'augmenter la stabilité physicochimique de la composition immunogène, et
- lorsque la composition immunogène comprend un agent d'intérêt lipophile et/ou un ligand de ciblage et/ou un agent immunostimulant encapsulé(s) dans les gouttelettes:
   - de solubiliser l'agent d'intérêt lipophile et/ou le ligand de ciblage et/ou l'agent immunostimulant, et
   - d'améliorer le contrôle du relargage de l'agent d'intérêt lipophile et/ou du ligand de ciblage et/ou de l'agent immunostimulant.

De préférence, le lipide solubilisant est solide à température ambiante (20°C).

Le lipide solubilisant peut notamment être constitué de dérivés du glycérol, et en particulier de glycérides obtenues par estérification de glycérol avec des acides gras, notamment dans le cas où le lipide amphiphile est un phospholipide.

Les lipides solubilisants préférés, en particulier pour les phospholipides, sont les glycérides d'acides gras, notamment d'acides gras saturés, et en particulier d'acides gras saturés comportant 8 à 18 atomes de carbone, encore préféré 12 à 18 atomes de carbone. Avantageusement, le lipide solubilisant est constitué d'un mélange complexe de différents glycérides. Par « mélange complexe », on entend un mélange de mono, di et triglycérides, comprenant des chaînes grasses de différentes longueurs, les dites longueurs s'étendant préférentiellement de C8 à C18, par exemple, en association, des chaînes en C8, C10, C12, C14, C16 et C18, ou de C10 à C18, comprenant par exemple en association, des chaînes en C10, C12, C14, C16 et C18.

Selon un mode de réalisation, lesdites chaînes grasses peuvent contenir une ou plusieurs insaturations.

De préférence, le lipide solubilisant est constitué d'un mélange de glycérides d'acides gras saturés comportant au moins 10% en poids d'acides gras en C12, au moins 5% en poids d'acides gras en C14, au moins 5% en poids d'acides gras en C16 et au moins 5% en poids d'acides gras en C18.

De préférence, le lipide solubilisant est constitué d'un mélange de glycérides d'acides gras saturés comportant 0% à 20% en poids d'acides gras en C8, 0% à 20% en poids d'acides gras en C10, 10% à 70% en poids d'acides gras en C12, 5% à 30% en poids d'acides gras en C14, 5% à 30% en poids d'acides gras en C16 et 5% à 30% en poids d'acides gras en C18.

Les mélanges des glycérides semi-synthétiques solides à température ambiante vendus sous la dénomination commerciale Suppocire^{®}NB par la société Gattefossé et approuvé pour un usage chez l'homme sont des lipides solubilisants particulièrement préférés. Les Suppocire^{®} de type N sont obtenues par estérification directe d'acides gras et de glycérol. Il s'agit de glycérides hémi-synthétiques d'acides gras saturés de C8 à C18, dont la composition quali-quantitative est indiquée dans le tableau 1 ci-dessous.

**Tableau 1 : Composition en acides gras du Suppocire^{®} NB de Gattefossé**

| Longueur de chaînes | [% en poids] |
|---|---|
| C8 | 0,1 à 0,9 |
| C10 | 0,1 à 0,9 |
| C12 | 25 à 50 |
| C14 | 10 à 24,9 |
| C16 | 10 à 24,9 |
| C18 | 10 à 24,9 |

Les lipides solubilisants précités permettent d'obtenir une composition immunogène avantageusement stable. Sans vouloir être lié à une théorie particulière, il est supposé que les lipides solubilisants précités permettent d'obtenir dans la composition immunogène des gouttelettes présentant un coeur amorphe. Le coeur ainsi obtenu présente une viscosité interne élevée sans pour autant présenter de cristallinité. Or, la cristallisation est néfaste pour la stabilité de la composition immunogène car elle conduit généralement à une agrégation des gouttelettes et/ou à une expulsion de l'agent d'intérêt lipophile et/ou du ligand de ciblage et/ou de l'agent immunostimulant, si présent(s), à l'extérieur des gouttelettes. Ces propriétés physiques favorisent la stabilité physique de la composition immunogène.

La quantité de lipide solubilisant peut varier largement en fonction de la nature et de la quantité de lipide amphiphile présent dans la phase dispersée. Généralement, la phase dispersée comporte de 1 à 99% en poids, de préférence de 5 à 80% en poids et tout particulièrement de 30 à 75% en poids de lipide solubilisant.

### • Co-tensioactif

La composition immunogène selon l'invention comprend un co-tensioactif. Ce co-tensioactif se situe partiellement dans la phase aqueuse continue et partiellement dans les gouttelettes de la phase dispersée.

De préférence, le co-tensioactif comporte au moins une chaîne composée de motifs d'oxyde d'éthylène ou d'oxyde d'éthylène et d'oxyde de propylène. Il peut être choisi en particulier parmi les composés conjugués polyéthylèneglycol/phosphatidyléthanolamine (PEG-PE), les éthers d'acide gras et de polyéthylèneglycol, les esters d'acide gras et de polyéthylèneglycol et les copolymères blocs d'oxyde d'éthylène et d'oxyde de propylène.

La chaîne polyalcoxylée du co-tensioactif comprend de préférence de 10 à 200, typiquement de 10 à 150, notamment de 20 à 100 motifs oxyde d'éthylène/oxyde de propylène.

A titre d'exemple de co-tensioactifs, on peut en particulier citer les composés conjugués à base de polyéthylèneglycol/phosphatidyl-éthanolamine (PEG-PE), les éthers d'acide gras et de polyéthylèneglycol tels que les produits vendus sous les dénominations commerciales Brij^{®} (par exemple Brij^{®} 35, 58, 78 ou 98) par la société ICI Americas Inc., les esters d'acide gras et de polyéthylèneglycol tels que les produits vendus sous les dénominations commerciales Myrj^{®} par la société ICI Americas Inc. (par exemple Myrj^{®} s20, s40 ou s100, anciennement nommés 49, 52 ou 59) et les copolymères blocs d'oxyde d'éthylène et d'oxyde de propylène tels que les produits vendus sous les dénominations commerciales Pluronic^{®} par la société BASF AG (par exemple Pluronic^{®} F68, F127, L64, L61, 10R4, 17R2, 17R4, 25R2 ou 25R4) ou les produits vendus sous la dénomination commerciale Synperonic^{®} par la société Unichema Chemie BV (par exemple Synperonic^{®} PE/F68, PE/L61 ou PE/L64).

Dans la composition immunogène selon l'invention, le rapport molaire du tensioactif porteur d'un antigène de formule (I) sur la somme du co-tensioactif et du tensioactif porteur d'un antigène de formule (I) est de 0,01 à 5%, notamment de 0,1 à 3%.

En effet, en dessous de 0,01%, et parfois en dessous de 0,1%, la quantité d'antigène est trop faible pour les applications de la composition explicitées ci-après.

Au-delà de 5%, et parfois au-delà de 3%, la composition immunogène est difficile à préparer et/ou est peu stable. En effet, comme explicité ci-après, la préparation de la composition immunogène nécessite une émulsion prémix comprenant un tensioactif (LI) comprenant un groupe G₁ fonctionnalisable. Pour obtenir une composition immunogène dans laquelle ledit rapport est supérieur à 5%, il est nécessaire de préparer une émulsion prémix dans laquelle le rapport molaire du tensioactif de formule (LI) sur la somme du co-tensioactif et du tensioactif de formule (LI) est supérieur à 5%, ce qui est difficile. En effet, les gouttelettes d'une telle émulsion ont une trop grande densité de surface de groupes fonctionnalisables G₁ et l'émulsion est donc peu stable. De plus, le greffage ultérieur de l'antigène sur l'émulsion prémix est difficile. Il n'a en effet pas été possible de formuler des émulsions pour des proportions molaires en tensioactif porteur d'un antigène de formule (I) sur la somme du co-tensioactif et du tensioactif porteur d'un antigène de formule (I) supérieur à 5%.

Généralement, la phase dispersée comporte de 0,01 à 3% en poids, de préférence de 0,1 à 1,3% en poids et tout particulièrement de 0,2 à 0,7% en poids de co-tensioactif.

Généralement, la fraction massique de lipide amphiphile par rapport au poids de co-tensioactif est de 0,005 % à 100 %, notamment de 0,01 % à 50 %, de préférence de 0,1 % à 30 %. En effet, en dessous de 0,005 % et au delà de 100 %, les gouttelettes de la phase dispersée ne sont souvent pas suffisamment stables et coalescent en quelques heures et il est souvent difficile d'obtenir des gouttelettes de diamètre inférieur à 200 nm.

Généralement, la composition immunogène ne comporte pas de tensioactifs supplémentaires : les seuls tensioactifs de la composition immunogène sont le lipide amphiphile, le co-tensioactif, le tensioactif de formule (I) et l'éventuel tensioactif cationique.

Dans un mode de réalisation, une proportion (100 - x) %, où 0 < x < 100, du co-tensioactif est greffé de façon covalente à un ligand biologique de ciblage.

La composition immunogène selon l'invention comprend toujours une proportion x non nulle de co-tensioactif « libre » (ne comportant pas de ligand biologique de ciblage greffé). Le co-tensioactif est constitué de x% de co-tensioactif « libre » et de (100-x)% de co-tensioactif sur lequel un ligand biologique de ciblage greffé. Dans ce mode de réalisation, par « le rapport molaire du tensioactif porteur d'un antigène de formule (I) sur la somme du co-tensioactif et du tensioactif porteur d'un antigène de formule (I) est de 0,01 à 5% », on entend que le rapport molaire du tensioactif porteur d'un antigène de formule (I) sur la somme du co-tensioactif « libre » et du tensioactif porteur d'un antigène de formule (I) est de 0,01 à 5%, c'est-à-dire que le rapport molaire du tensioactif porteur d'un antigène de formule (I) sur la somme de x% du co-tensioactif, et du tensioactif porteur d'un antigène de formule (I) est de 0,01 à 5%

Typiquement, le ligand biologique de ciblage a été greffé par liaison covalente au co-tensioactif tel que défini ci-dessus. Le greffage peut être réalisé avant ou après la formation de l'émulsion utilisée pour préparer la composition immunogène (émulsion prémix ci-après). Le dernier cas peut être préconisé lorsque les réactions chimiques employées sont compatibles avec la stabilité des émulsions, notamment en termes de pH. De préférence, le pH lors de la réaction de greffage est compris entre 5 et 11.

Généralement, ce greffage a été effectué à une extrémité de la ou des chaînes polyalcoxylée(s) du co-tensioactif, et le ligand biologique de ciblage est ainsi situé à la surface des gouttelettes de la phase dispersée de la composition immunogène.

### • Agent immunostimulant

Dans un mode de réalisation, la composition immunogène peut comprendre, en outre, un agent immunostimulant, qui permet d'améliorer ou d'augmenter la réponse immune.

L'agent immunostimulant est notamment choisi parmi un sel d'aluminium, un sel de calcium, magnésium, fer ou zinc, la saponine (e.g. Quil A et ses fragments purifiés tels que QS7 et QS21), le lipide A ou l'un de ses dérivés, un oligonucléotide immunostimulant, un alkyl phosphate glucosamide. De préférence, l'agent immunostimulant est le lipide A.

La proportion d'agent immunostimulant dans la phase dispersée dépend de la nature et de l'efficacité de l'agent immunostimulant et de la présence ou non d'un ligand biologique de ciblage dans la composition immunogène. Cette proportion peut facilement être déterminée par l'homme du métier.

### • Ligand biologique de ciblage

Dans un mode de réalisation, la composition immunogène peut comprendre un ligand biologique de ciblage.

Le ligand biologique de ciblage peut être greffé ou non sur le co-tensioactif, c'est à dire :
- sous forme libre dans les gouttelettes, c'est-à-dire qu'il est encapsulé dans les gouttelettes, soit dans la couronne s'il a un caractère amphiphile, soit dans le coeur s'il a un caractère lipophile, et/ou
- sous forme greffée de façon covalente au co-tensioactif, comme explicité ci-dessus.

Dans certains modes de réalisation, le ligand biologique de ciblage est un lipide mannosylé ou un anticorps anti-DC-sign.

La proportion de ligand biologique de ciblage dans la phase dispersée dépend de la nature et de l'efficacité du ligand biologique de ciblage. Cette proportion peut facilement être déterminée par l'homme du métier.

### • Agent d'intérêt

Dans un mode de réalisation, la composition immunogène peut comprendre un ou plusieurs agent(s) d'intérêt.

L'agent d'intérêt peut être de nature très variée. Ainsi, l'agent d'intérêt peut être :
- un agent optique tel que un colorant, un chromophore, un fluorophore, par exemple le perchlorate 1,1'-dioctadecyl 3,3,3',3'-tetramethylindodicarbocyanine (DiD), le iodure de 1,1'-dioctadecyl 3,3,3',3'-tetramethylindotricarbocyanine (DiR), le vert d'indocyanine (ICG), ou encore des composants ayant des propriétés optoélectronique, tels que les saturants ou les absorbants optiques,
- un agent physique, tel qu'un isotope radioactif ou un photo-sensibilisateur,
- un agent d'imagerie, notamment pour l'IRM (imagerie par résonance magnétique), le PET (en anglais Positron Emission Tomography), le SPECT (Single Photon Emission Computed Tomography), l'échographie ultrasonore, la radiographie, la tomographie X et l'imagerie optique (fluorescence, bioluminescence, diffusion...). Ces agents peuvent permettre de suivre la position des gouttelettes (et donc de l'antigène) après administration de la composition immunogène au patient, et/ou
- un agent thérapeutique, notamment choisi parmi un antibiotique, un anticancéreux, un antiviral, un antiparasitaire, une protéine thérapeutique (telle qu'une cytokine ou une chimiokine) et un mélange de ceux-ci.

Les quantités d'agent d'intérêt dépendent de l'application visée ainsi que de la nature des agents.

Lorsque la composition immunogène selon l'invention comprend un agent d'intérêt, elle contient le plus souvent une quantité de 0,001 à 30% en poids, de préférence 0,01 à 20% en poids, et encore préférée 0,1 à 10% en poids d'agent d'intérêt.

L'agent d'intérêt peut être hydrophile (il est alors situé dans la phase aqueuse continue de la composition immunogène) ou lipophile (il est alors encapsulé dans les gouttelettes formant la phase dispersée de la composition immunogène ou se situe dans la couronne des gouttelettes, selon son affinité lipophile ou amphiphile).

### • Tensioactif cationique

La formulation selon l'invention peut comprendre un tensioactif cationique, notamment choisi parmi :
- le *N*[1-(2,3-dioléyloxy)propyl]-*N,N,N-*triméthylammonium (DOTMA),
- le 1,2-dioleyl-3-trimethylamonium-propane (DOTAP),
- le *N*-(2-hydroxyethyl)-*N,N-*dimethyl-2,3-bis(tetradecyloxy-1-propananium) (DMRIE),
- le 1-[2-(oleoyloxy)ethyl]-2-oleyl-3-(2-hydroxyethyl)imidazolinium (DOTIM), et
- le dioctadecylamidoglycylspermine (DOGS) (sous forme protonée),
et étant de préférence le 1,2-dioleyl-3-trimethylamonium-propane (DOTAP).

Le tensioactif cationique se situe dans la couronne des gouttelettes de la formulation. Le tensioactif cationique permet notamment d'améliorer, ou d'accélérer la capture des gouttelettes par les cellules immunitaires. Le tensioactif cationique a également pour effet de créer au site d'administration une légère inflammation locale qui attire les cellules immunitaires sur le site d'administration des gouttelettes, permettant ainsi une accélération de la capture des gouttelettes par les cellules immunitaires (Doroud et al. / Journal of Controlled Release 153 (2011) 154-162).

Généralement, la phase dispersée comporte de 0 à 25% en poids, de préférence de 0 à 7% en poids de tensioactif cationique. Ces proportions sont particulièrement adaptées pour améliorer, ou accélérer la capture des gouttelettes par les cellules immunitaires.

### • Huile

La phase dispersée de la composition immunogène selon l'invention peut également comporter une ou plusieurs huiles.

Les huiles utilisées présentent de préférence une balance hydrophile-lipophile (HLB) inférieure à 10 et encore plus préférentiellement de 3 à 9. Avantageusement, les huiles sont utilisées sans modification chimique ou physique préalablement à la formation de la composition immunogène.

Selon les applications envisagées, les huiles peuvent être choisies parmi les huiles biocompatibles, et en particulier parmi les huiles d'origine naturelle (végétale ou animale) ou synthétique. Parmi de telles huiles, on peut notamment citer les huiles d'origine naturelle végétale parmi lesquelles figurent notamment les huiles de soja, de lin, de palme, d'arachide, d'olives, de sésame, de pépin de raisins et de tournesol ; les huiles synthétiques parmi lesquelles figurent notamment les triglycérides, diglycérides et les mono glycérides ; et les huiles issues de graisses animales. Ces huiles peuvent être de premières expressions, raffinées ou inter-estérifiées.

L'huile préférée est l'huile de soja.

Généralement, si présente, l'huile est contenue dans la phase dispersée dans une proportion allant de 1 à 80% en poids, de préférence de 5 à 50 % en poids et tout particulièrement de 10 à 30% en poids par rapport au poids total de la phase dispersée.

### • Phase aqueuse

La phase aqueuse continue de la composition immunogène selon l'invention est de préférence constituée d'eau et/ou d'un tampon tel qu'un tampon phosphate comme par exemple du PBS ("Phosphate Buffer Saline") ou d'une solution saline, notamment de chlorure de sodium. Généralement, le pH de la phase aqueuse est de l'ordre du pH physiologique.

Selon un mode de réalisation, la phase continue comporte également un agent épaississant tel que du glycérol, un saccharide, oligosaccharide ou polysaccharide, une gomme ou encore une protéine, de préférence du glycérol. En effet, l'utilisation d'une phase continue de viscosité plus élevée facilite l'émulsification et permet de ce fait de réduire le temps de sonication.

La phase aqueuse comporte avantageusement de 0 à 50% en poids, de préférence de 1 à 30% en poids et tout particulièrement de 5 à 20% en poids d'agent épaississant.

Bien entendu, la phase aqueuse peut contenir en outre d'autres additifs tels que des colorants, stabilisants et conservateurs en quantité appropriée.

### • Composition immunogène sous forme de gel

Dans un mode de réalisation, la viscosité de la composition immunogène est supérieure à 1 poise (0,1 Pa.s) à 25°C.

Dans ce mode de réalisation, la composition immunogène se présente sous forme de « gel ». On entend par le terme « gel » habituellement un système biphasique solide-liquide thermodynamiquement stable, constitué d'un double réseau interpénétré continu tridimensionnel, l'un solide et le second liquide. Un tel gel est un système biphasique liquide-solide dont le réseau solide retient une phase liquide. Bien que les gels puissent être considérés comme solides, ils présentent des propriétés propres aux solides (rigidité structurelle, élasticité à la déformation) comme aux liquides (pression de vapeur, de compressibilité et conductivité électrique). Les interactions entre les gouttelettes peuvent être dues à des forces de Van der Waals, des liaisons électrostatiques, des liaisons hydrogènes ou des liaisons covalentes.

Dans le cas d'une nanoémulsion sous forme de gel, le réseau tridimensionnel est formé par les gouttelettes, les interstices entre gouttelettes étant remplis de phase continue. Les liaisons entre les unités du réseau, à savoir les gouttelettes, reposent principalement sur des interactions électrostatiques (paires d'ions). Ces interactions électrostatiques existent principalement entre les séquences nucléotidiques susceptibles de moduler des mécanismes endogènes d'ARN interférence et les tensioactifs cationiques de gouttelettes adjacentes.

Une nanoémulsion sous forme de gel montre donc une résistance à la pression et est capable de maintenir une forme définie, ce qui peut être avantageux selon la forme et/ou la voie d'administration souhaitée.

Pour mettre en évidence que la composition immunogène est sous forme de gel, on peut réaliser des études rhéologiques permettant d'évaluer les propriétés viscoélastiques, et/ou des études plus structurelles montrant les liaisons entre les gouttelettes formant le réseau tridimensionnel (diffraction aux rayons X, neutrons...). En effet, une nanoémulsion sous forme de gel possède une viscosité et un coefficient d'élasticité plus important qu'une nanoémulsion liquide. La nanoémulsion sous forme de gel peut, en fonction de la concentration de gouttelettes et donc de la fraction massique en phase dispersée, se trouver à l'état de liquide visqueux, de solide viscoélastique ou de solide élastique. Par rapport à la phase dispersante aqueuse, dont la viscosité est proche de celle de l'eau (1 mPa.s à 25°C), la nanoémulsion est considérée comme un liquide visqueux lorsque sa viscosité est 10 fois plus élevée que celle de l'eau, soit > 10 mPa.s à 25°C. Par ailleurs, lorsque l'on procède à la mesure rhéologique des modules de G' (module de conservation en cisaillement) et G" (module de perte en cisaillement), on considère que la nanoémulsion est sous forme d'un liquide visqueux lorsque G" >G'. Lorsque G' devient proche de G", la nanoémulsion est à l'état de solide viscoélastique. Lorsque G" < G', on est à l'état de solide élastique. Dans ce mode de réalisation, la nanoémulsion se présente de préférence à l'état liquide visqueux, de solide viscoélastique, ou solide. La viscosité et le coefficient d'élasticité peuvent être mesurés par un rhéomètre cône-plan ou par un rhéomètre Couette. La viscosité d'une nanoémulsion liquide est généralement inférieure à 1 poise, voire même souvent inférieure à 0,01 poise. La nanoémulsion utilisée dans ce mode de réalisation de l'invention a généralement une viscosité supérieure à 1 poise, et pourra avoir une viscosité allant jusqu'à celle d'un solide (plus de 1000 poises). Les études structurelles, notamment les diffractions aux rayons X ou aux neutrons, permettent également de différencier l'organisation d'une nanoémulsion liquide, de l'organisation d'une nanoémulsion sous forme de gel. En effet, les pics du diffractogramme obtenu pour une nanoémulsion liquide sont caractéristiques de la structure des gouttelettes de phase dispersée (grand angles de diffraction caractéristiques de distances courtes), alors que les pics du diffractogramme d'une nanoémulsion sous forme de gel sont caractéristiques non seulement de la structure des gouttelettes (grand angles de diffraction caractéristiques de distances courtes) mais aussi de l'organisation de ces gouttelettes en réseau tridimensionnel (faibles angles de diffraction caractéristiques de distances plus grandes).

La composition immunogène sous forme de gel est avantageusement sous forme de gel dispersible, c'est-à-dire que les gouttelettes formant le réseau tridimensionnel peuvent être relarguées dans la phase continue sous certaines conditions par « dégélification » du système gel, également dénommée « désagrégation » dans la présente demande. La désagrégation est observée par ajout de phase continue au gel, par mise en contact avec les fluides physiologiques lors de l'administration de la nanoémulsion ou par augmentation de la température. En effet, ajouter de la phase continue entraîne une différence de pression osmotique entre l'intérieur du gel et la phase continue. Le système tendra donc à diminuer, jusqu'à annuler, cette différence de pression osmotique en libérant les gouttelettes dans l'excès de phase continue, jusqu'à obtenir une concentration en gouttelettes homogène dans l'ensemble du volume de phase continue. La mise en contact avec les fluides physiologiques peut aussi induire une réaction chimique (exemple : clivage de liaisons covalentes de type pont disulfure ou liaison peptidique, et ainsi libérer les gouttelettes). De même augmenter suffisamment la température du système revient à donner aux différentes gouttelettes une énergie thermique supérieure aux énergies mises en jeu dans les liaisons, par exemple les liaisons hydrogène, et ainsi à rompre ces liaisons et libérer les gouttelettes du réseau tridimensionnel. Ces températures dépendent de la composition du gel et plus particulièrement de la taille des gouttelettes et de la longueur des chaînes polyalcoxylées du co-tensioactif. La désagrégation de la nanoémulsion sous forme de gel peut être suivie par diffraction aux rayons X, par calorimétrie différentielle à balayage (DSC) ou par résonance magnétique nucléaire (RMN).

### • Localisation des composants des gouttelettes

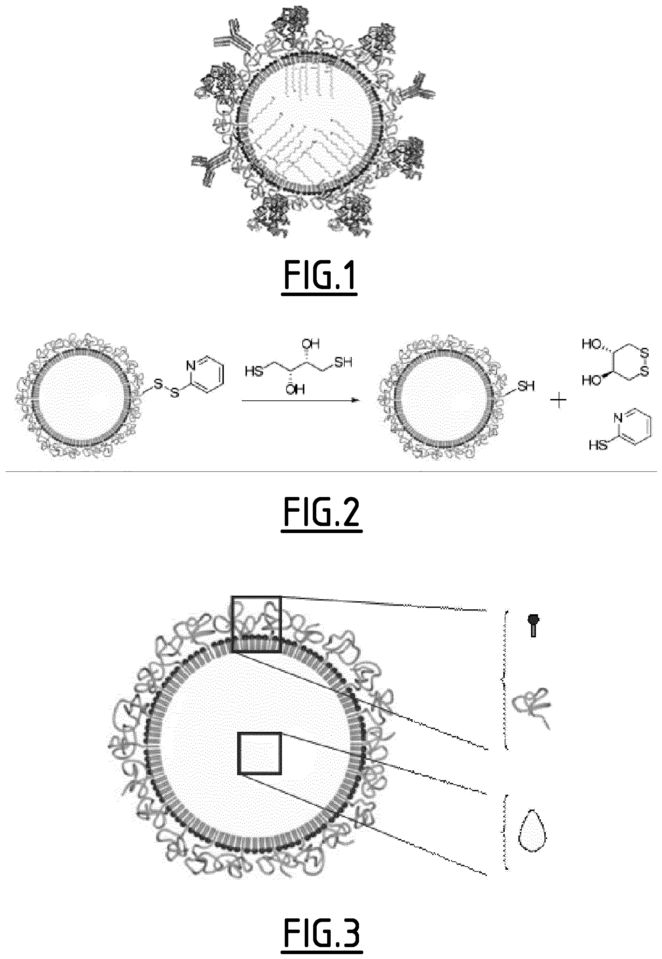

Comme illustré sur la figure 1, les gouttelettes de la composition immunogène selon l'invention s'organisent sous forme de coeur - couronne, où :
- le coeur comprend :
   - le lipide solubilisant,
   - l'éventuelle huile,
   - l'éventuel agent d'intérêt lipophile,
   - l'éventuel agent immunostimulant lipophile,
- la couronne comprend :
   - le lipide amphiphile,
   - le tensioactif porteur d'un antigène de formule (I),
   - l'éventuel tensioactif cationique,
   - le co-tensioactif (dont une proportion (1-x)% est éventuellement greffé avec un ligand de ciblage),
   - l'éventuel ligand biologique de ciblage amphiphile,
   - la séquence nucléotidique susceptible de moduler des mécanismes endogènes d'ARN interférence,
   - l'éventuel agent immunostimulant amphiphile,
   - l'éventuel agent d'intérêt amphiphile.

### • Autres propriétés de la composition immunogène

Grâce à sa formulation, la composition immunogène selon l'invention est stable et présente une excellente stabilité au stockage (supérieure à 5 mois voire à 8 mois). En particulier, du fait que l'antigène est lié de façon covalente aux gouttelettes, il ne migre pas dans la phase aqueuse continue, au contraire de compositions immunogènes dans lesquelles l'antigène est simplement encapsulé.

L'antigène greffé aux gouttelettes est également stabilisé par la composition immunogène, du fait que les co-tensioactifs et lipides amphiphiles le protègent.

Les chaînes polyalcoxylées du co-tensioactif et du tensioactif de formule (I), hydratées et non chargées, couvrant la surface des gouttelettes, écrantent les charges apportées par les lipides amphiphiles à la surface solide des gouttelettes. On se trouve donc dans le cas d'une stabilisation stérique des gouttelettes, et non une stabilisation électrostatique.

### [Procédé de préparation]

Selon un deuxième objet, l'invention concerne un procédé de préparation de la composition immunogène définie ci-dessus, comprenant la mise en contact :
- d'une émulsion prémix comprenant une phase aqueuse continue et une phase dispersée sous forme de gouttelettes, comprenant un lipide amphiphile, un lipide solubilisant comprenant au moins un glycéride d'acide gras, un co-tensioactif comprenant au moins une chaine composée de motifs d'oxyde d'alkylène et un tensioactif de formule (LI) suivante :

   L₁-X₁-H₁-Y₁-G₁ (LI),

   dans laquelle le rapport molaire du tensioactif de formule (LI) sur la somme du co-tensioactif et du tensioactif de formule (LI) est de 0,01% à 5%,
- avec un composé de formule (LII) suivante :

   G₂-Z₁-Ag (LII)
où L₁, X₁, H₁, Y₁, Z₁ et Ag sont tels que définis ci-dessus, et G₁ et G₂ sont des groupes susceptibles de réagir ensemble pour former le groupe G tel que défini ci-dessus,
dans des conditions permettant la réaction du tensioactif de formules (LI) avec le composé de formule (LII) pour former le tensioactif porteur d'un antigène de formule (I) tel que défini ci-dessus.

Lorsque le groupe G comprend un seul groupe G', les groupes G₁ et G₂ sont typiquement des groupes susceptibles de réagir l'un avec l'autre pour former le groupe G.

Lorsque le groupe G comprend plusieurs groupes G', on met généralement l'émulsion prémix et le composé de formule (LII) en contact avec un composé susceptible de réagir avec eux pour former le groupe G. Ce composé comprend typiquement au moins v fonctions G'₁ susceptibles de réagir avec le groupe G₁ et une fonction G'₂ susceptibles de réagir avec le groupe G₂.

Ainsi, dans le mode de réalisation dans lequel le groupe G répond à la formule -G'-Y₃-G'- définie ci-dessus, le procédé de préparation de la composition immunogène comprend typiquement la mise en contact :
- d'une émulsion prémix telle que définie ci-dessus,
- et du composé de formule (LII) tel que définie ci-dessus,
- avec un composé de formule G'₁-Y₃-G'₂ dans laquelle Y₃ est tel que défini ci-dessus, G'₁ est un groupe susceptible de réagir avec G₁ pour former le premier groupe G' tel que défini ci-dessus et G'₂ est un groupe susceptible de réagir avec G₂ pour former le second groupe G' tel que défini ci-dessus (de nature identique ou différente du premier groupe G'),
dans des conditions permettant la réaction du tensioactifs de formule (LI) et du composé de formule (LII) avec le composé de formule G'₁-Y₃-G'₂ pour former le tensioactif porteur d'un antigène de formule (I) dans lequel le groupe G répond à la formule -G'-Y₃-G'-définie ci-dessus.

### • Formation du tensioactif porteur d'antigène de formule (I) par réaction entre le tensioactif de formule (LI) et le composé de formule (LII)

L'émulsion prémix comprend un tensioactif de formule (LI) comprenant un groupe G₁ fonctionnalisable, qui se situe en surface des gouttelettes.

Avantageusement, une même émulsion prémix peut être utilisée pour greffer des anticorps de natures différentes, dès lors que le composé de formule (LII) comprend un groupe G₂ susceptible de réagir avec le groupe G₁ de l'émulsion prémix. Il n'est pas nécessaire d'adapter les composants de l'émulsion prémix et les conditions de greffage pour chaque antigène différent utilisé. Ainsi, le procédé de préparation de la composition immunogène peut être mis en oeuvre de façon industrielle et être automatisé.

La formation du tensioactif porteur d'antigène de formule (I) par réaction entre le tensioactif de formule (LI) et le composé de formule (LII) permet le greffage par liaison covalente de l'antigène Ag aux gouttelettes de l'émulsion prémix comprenant le tensioactif fonctionnalisable de formule (LI). L'antigène est lié aux gouttelettes de l'émulsion par une liaison covalente. Le greffage de l'anticorps aux gouttelettes de l'émulsion prémix est avantageusement indépendant du caractère hydrophile, amphiphile ou lipophile de l'antigène. N'importe quel type d'antigène peut donc être greffé, ce qui est un avantage par rapport aux compositions immunogènes à base d'émulsion et de nanoparticules lipidiques solides de l'art antérieur dans lesquelles l'antigène est encapsulé dans les gouttelettes (l'encapsulation n'étant possible que pour des antigènes amphiphiles ou lipophiles).

Au vu de ses connaissances générales en chimie, l'homme du métier est à même de choisir la nature des groupes G'₁, G'₂, Y₃, G₁ et G₂ à utiliser pour former le groupe G et les conditions permettant la réaction. Les réactions de chimie organique usuelles peuvent être suivies, notamment celles décrites dans « Comprehensive Organic Transformations: A Guide to Functional Group Preparations » de Richard C. Larock édité par John Wiley & Sons Inc, et les références qui y sont citées. Ainsi, les exemples de groupes G₁ et G₂ ci-dessous sont cités à titre illustratif et non limitatif.

Typiquement, lorsque le groupe G est constitué d'un groupe G', le groupe G₁ du tensioactif de formule (LI) et le groupe G₂ du composé de formule (LII) peuvent par exemple être choisis comme suit :
- l'un des G₁ et G₂ représente un thiol (-SH) et l'autre G₁ ou G₂ représente :
   - soit un maléimide, un tensioactif de formule (I) dans lequel G comprend un groupe G' représentant un groupe de formule (XIV) où A₁₀₂ représente N étant alors formé, la mise en contact de l'émulsion prémix et du composé de formule (LII) étant de préférence réalisée à une température de 0 °C à 15 °C, notamment de 0 à 10 °C, de préférence de 0 à 5 °C,
   - soit une vinylsulfone, un tensioactif de formule (I) dans lequel G comprend un groupe G' représentant un groupe de formule (XVI) étant alors formé,
   - soit un groupe -S-S-pyridinyle ou -SH, un tensioactif de formule (I) dans lequel G comprend un groupe G' représentant un groupe de formule (XV) étant alors formé,
- l'un des G₁ et G₂ représente un hydroxyle et l'autre G₁ ou G₂ représente -COOH ou un ester activé, un tensioactif de formule (I) dans lequel G comprend un groupe G' représentant un groupe de formule (XXIII) étant alors formé,
- l'un des G₁ et G₂ représente une amine -NH₂ et l'autre G₁ ou G₂ représente -COOH ou un ester activé, un tensioactif de formule (I) dans lequel G comprend un groupe G' représentant un groupe de formule (XI) étant alors formé,
- l'un des G₁ et G₂ représente un hydroxyle et l'autre G₁ ou G₂ représente un carbonate activé ou un carbamate activé, un tensioactif de formule (I) dans lequel G comprend un groupe G' représentant un groupe de formule (XIX) étant alors formé,
- l'un des G₁ et G₂ représente une amine -NH₂ et l'autre G₁ ou G₂ représente un carbonate activé ou un carbamate activé, un tensioactif de formule (I) dans lequel G comprend un groupe G' représentant un groupe de formule (XII) étant alors formé,
- l'un des G₁ et G₂ représente une amine -NH₂ et l'autre G₁ ou G₂ représente un aldéhyde -CHO, un tensioactif de formule (I) dans lequel G comprend un groupe G' représentant un groupe de formule (XXI) étant alors formé,
- l'un des G₁ et G₂ représente un hydrazide de formule -(C=O)-NH-NH₂ et l'autre G₁ ou G₂ représente un groupe -(C=O)-R₁₀₂, un tensioactif de formule (I) dans lequel G comprend un groupe G' représentant un groupe de formule (XIII) étant alors formé,
- l'un des G₁ et G₂ représente un alcyne et l'autre G₁ ou G₂ représente un azide, un tensioactif de formule (I) dans lequel G comprend un groupe G' représentant un groupe de formule (XVIII) étant alors formé,
- l'un des G₁ et G₂ représente un cyclooctyne et l'autre G₁ ou G₂ représente un azide, un tensioactif de formule (I) dans lequel G comprend un groupe G' représentant un groupe de formule (XVIII') étant alors formé,
- l'un des G₁ et G₂ représente un furane et l'autre G₁ ou G₂ représente un maléimide, un tensioactif de formule (I) dans lequel G comprend un groupe G' représentant un groupe de formule (XVII) étant alors formé,
- l'un des G₁ et G₂ représente un aldéhyde et l'autre G₁ ou G₂ représente une amine, un tensioactif de formule (I) dans lequel G comprend un groupe G' représentant un groupe de formule (XXI) étant alors formé,
- l'un des G₁ et G₂ représente un phosphate de formule -O-P(=O)(OH)₂ et l'autre G₁ ou G₂ représente un hydroxyle, un tensioactif de formule (I) dans lequel G comprend un groupe G' représentant un groupe de formule (XXII) étant alors formé,
- l'un des G₁ et G₂ représente un bon groupe partant LG et l'autre G₁ ou G₂ représente un groupe de formule suivante 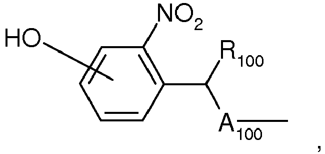 un tensioactif de formule (I) dans lequel G comprend un groupe G' représentant un groupe de formule (XXIV) dans laquelle A₁₀₁ représente O étant alors formé,
- l'un des G₁ et G₂ représente un hydroxyle ou une amine -NH₂ et l'autre G₁ ou G₂ représente un groupe de formule suivante 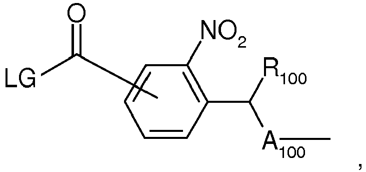 un tensioactif de formule (I) dans lequel G comprend un groupe G' représentant un groupe de formule (XXIV) dans laquelle A₁₀₁ représente respectivement -O-(CO)- ou - NH-(CO) étant alors formé,
- l'un des G₁ et G₂ représente un bon groupe partant LG et l'autre G₁ ou G₂ représente un hydroxyle, un tensioactif de formule (I) dans lequel G comprend un groupe G' représentant un groupe de formule (XXV) étant alors formé,
- l'un des G₁ et G₂ représente un bon groupe partant LG et l'autre G₁ ou G₂ représente une amine -NH₂, un tensioactif de formule (I) dans lequel G comprend un groupe G' représentant un groupe de formule (XXVI) étant alors formé,
- l'un des G₁ et G₂ représente une oxyamine -O-NH₂ et l'autre G₁ ou G₂ représente un aldéhyde, un tensioactif de formule (I) dans lequel G comprend un groupe G' représentant un groupe de formule (XXVII) étant alors formé.

Lorsque le groupe G comprend plusieurs groupes G', le choix des groupes réagissant ensemble : G'₁ et G₁ d'une part et G'₂ et G₂ d'autre part, peut être effectué de la même façon, en remplaçant les groupes G₁ ou G₂ dans les exemples mentionnés ci-dessus par G'₁ ou G'₂.

Le composé de formule (LII) peut être soit un antigène en tant que tel lorsque celui-ci comprend à l'état naturel un groupe -G₂ susceptible de se greffer au tensioactif de formule (LI), soit un antigène modifié chimiquement pour y greffer le groupe G₂ désiré (par l'intermédiaire du groupe de liaison Z₁), cette modification chimique étant réalisée dans des conditions connues de l'homme du métier.

Le procédé peut donc comprendre, préalablement à la mise en contact de l'émulsion prémix et du composé de formule (LII) pour former le tensioactif porteur d'un antigène de formule (I), une étape de préparation du composé de formule (LII) par modification chimique d'un antigène pour y greffer le groupe G₂.

Dans un mode de réalisation, le composé de formule (LII) est un antigène naturellement porteur d'une fonction amine -NH₂. On peut notamment citer les antigènes protéiques comprenant au moins une lysine.

Par exemple, pour préparer une composition immunogène dont le tensioactif de formule (I) a la formule (I') rappelée ci-dessous : 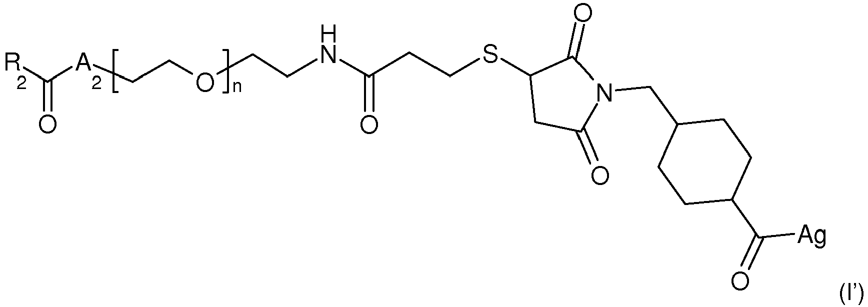 le procédé comprend typiquement :
- la préparation du composé de formule (LII') par modification chimique d'un antigène porteur d'une fonction amine -NH₂ par réaction avec du (Sulfosuccinimidyl-4-N-maleimidomethyl) cyclohexane-1-carboxylate) (Sulfo-SMCC) selon le schéma réactionnel suivant : 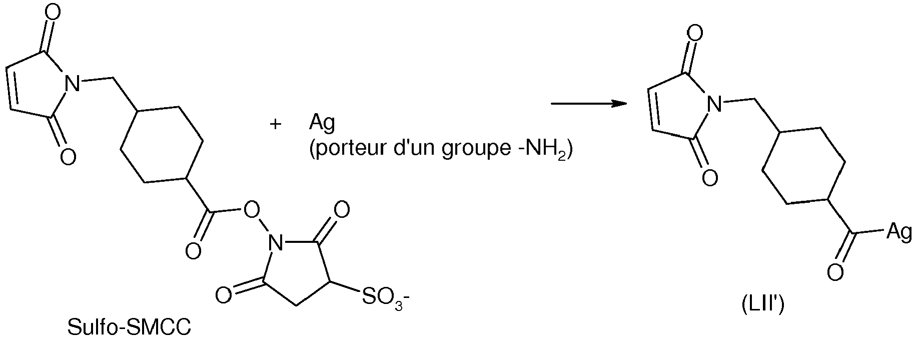
- puis la mise en contact, de préférence réalisée à une température de 0°C à 15°C, notamment de 0 à 10°C, de préférence de 0 à 5°C :
   - d'une émulsion prémix comprenant une phase aqueuse continue et une phase dispersée sous forme de gouttelettes, comprenant un lipide amphiphile, un lipide solubilisant comprenant au moins un glycéride d'acide gras, un co-tensioactif comprenant au moins une chaine composée de motifs d'oxyde d'alkylène et un tensioactif de formule (LI') suivante : 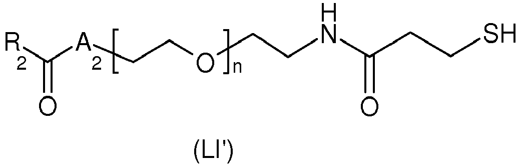 dans laquelle le rapport molaire du tensioactif de formule (LI) sur la somme du co-tensioactif et du tensioactif de formule (LI') est de 0,01% à 5%,
   - avec le composé de formule (LII'),
où R₂, A₂, n et Ag sont tels que définis ci-dessus.

Généralement, le rendement de la réaction entre le tensioactif de formule (LI) et le composé de formule (LII) (c'est-à-dire de la réaction de greffage de l'antigène aux gouttelettes de l'émulsion) est supérieur à 40%, notamment supérieur à 50%, typiquement supérieur à 60%. Des rendements supérieurs à 90% peuvent être observés dans certains modes de réalisation. Ces rendements sont variables selon la réaction chimique mise en oeuvre (et donc de la nature des groupes G₁ et G₂ ou G'₁ et G'₂), selon la nature du tensioactif de formule (LI) (par exemple, au-delà de 200 motifs d'oxyde d'éthylène dans le groupe H₁, la chaîne poly(oxyde d'éthylène) du tensioactif se replie sur elle-même et les groupes G₁ sont moins accessibles pour réagir avec le composé de formule (LII)) et selon la nature de l'antigène Ag (selon sa taille, sa charge, l'accessibilité de la fonction Z₁-G₂ dans l'espace...).

Les composants de la composition immunogène décrits ci-dessus sont disponibles commercialement ou peuvent être préparés en suivant des protocoles décrits dans la littérature.

### • Formation de l'émulsion prémix

L'émulsion prémix peut être préparée aisément par dispersion de quantités appropriées de phase huileuse et de phase aqueuse sous l'effet d'un cisaillement, typiquement par un procédé comportant les étapes consistant à :
(i) préparer une phase huileuse comprenant un lipide amphiphile et un lipide solubilisant comprenant au moins un glycéride d'acide gras;
(ii) préparer une phase aqueuse comprenant un co-tensioactif comprenant au moins une chaine composée de motifs d'oxyde d'alkylène et un tensioactif de formule (LI);
(iii) disperser la phase huileuse dans la phase aqueuse sous l'action d'un cisaillement suffisant pour former une émulsion; et
(iv) récupérer l'émulsion ainsi formée.

Dans ce procédé, on mélange d'abord les différents constituants huileux pour préparer un pré-mélange huileux pour la phase dispersée de l'émulsion. Le mélange des différents constituants huileux peut éventuellement être facilité par mise en solution d'un des constituants ou du mélange complet dans un solvant organique approprié et évaporation subséquente du solvant, pour obtenir un pré-mélange huileux homogène pour la phase dispersée. Le choix du solvant organique dépend de la solubilité des constituants. Les solvants employés peuvent être par exemple le méthanol, l'éthanol, le chloroforme, le dichlorométhane, l'hexane, le cyclohexane, le DMSO, le DMF ou encore le toluène. Lorsqu'il s'agit d'une composition immunogène destinée à être administrée, il s'agit de préférence de solvants organiques volatils et/ou non toxiques pour l'homme. Par ailleurs, il est préféré de réaliser le pré-mélange à une température à laquelle l'ensemble des ingrédients est liquide.

Avantageusement, la phase huileuse est dispersée dans la phase aqueuse à l'état liquide. Si l'une des phases se solidifie à température ambiante, il est préférable de réaliser le mélange avec l'une ou de préférence les deux phases chauffées à une température supérieure ou égale à la température de fusion, les deux phases étant chauffées à une température de préférence inférieure à 80°C, et encore préférentiellement inférieure à 70°C, et encore préférentiellement inférieure à 60°C.

L'émulsification sous l'effet de cisaillement est de préférence réalisée à l'aide d'un sonificateur ou d'un microfluidiseur. De préférence, la phase aqueuse puis la phase huileuse sont introduites dans les proportions souhaitées dans un récipient cylindrique approprié puis le sonificateur est plongé dans le milieu et mis en marche pendant une durée suffisante pour obtenir une émulsion, le plus souvent quelques minutes.

L'émulsion prémix est généralement une nanoémulsion. Par le procédé susmentionné, on obtient une nanoémulsion homogène dans laquelle le diamètre moyen des gouttelettes est supérieur à 20 nm et inférieur à 200 nm, notamment de 50 à 120 nm.

De préférence, le potentiel zêta de l'émulsion obtenue est inférieur à 25 mV en valeur absolue, c'est-à-dire compris entre -25mV et 25 mV.

Avant préparation de la composition immunogène selon l'invention, l'émulsion prémix peut être diluée et/ou stérilisée, par exemple par filtration ou dialyse. Cette étape permet d'éliminer les éventuels agrégats qui pourraient s'être formés au cours de la préparation de l'émulsion.

L'émulsion prémix ainsi obtenue est prête à l'emploi, le cas échéant après dilution.

Dans l'émulsion prémix, le rapport molaire du tensioactif de formule (LI) sur la somme du co-tensioactif et du tensioactif de formule (LI) est de 0,01% à 5%. En effet, il a été mis en évidence que le greffage du composé de formule (LII) sur les gouttelettes de l'émulsion prémix (par réaction avec le tensioactif de formule (LI)) n'est pas efficace pour des rapports molaires (tensioactif de formule (LI)) / (co-tensioactif + tensioactif de formule (LI)) supérieur à 5%.

### [Utilisations de la composition immunogène]

### • Méthode de production d'anticorps

L'invention concerne également une méthode de production d'anticorps monoclonaux ou polyclonaux mettant en oeuvre la composition immunogène définie ci-dessus.

Ainsi, l'invention concerne une méthode de production d'anticorps polyclonaux, comprenant les étapes consistant en :
(a) l'immunisation d'un animal avec une composition immunogène telle que définie dans l'une quelconque des revendications 1 à 7, de manière à induire une réponse immune humorale contre ledit antigène, et
(b) la récolte des anticorps polyclonaux induits dirigés contre ledit antigène.

L'invention concerne également une méthode de production d'anticorps monoclonaux, comprenant les étapes consistant en :
(i) l'immunisation d'un animal avec une composition immunogène selon l'invention,
(ii) récupération et isolement des lymphocytes B de l'animal immunisé à l'étape (i),
(iii) production d'un hybridome et culture dudit l'hybridome afin de produire des anticorps monoclonaux dirigés contre l'antigène présent dans ladite composition immunogène,
(iv) récolte et purification des anticorps monoclonaux produits à l'étape (iii).

L'immunisation aux étapes (a) et (i) est réalisée en injectant la composition immunogène selon l'invention à un animal en une dose efficace pour induire une réponse immune humorale à un antigène tel que défini ci-dessus. L'homme du métier est capable de déterminer les conditions nécessaires à l'immunisation des animaux. Plusieurs protocoles d'immunisation sont ainsi possibles en fonction de l'antigène présent dans la composition immunogène selon l'invention, par exemple en variant les doses, les intervalles des injections, la durée du traitement.

L'animal utilisé dans les méthodes de production d'anticorps selon l'invention est un animal classiquement utilisé pour produire des anticorps, à savoir un rongeur (souris, rat, hamster), un lapin, une chèvre, un mouton, un singe, une poule, un cochon d'inde, ou un cheval.

Optionnellement, dans les méthodes de production d'anticorps selon l'invention, une étape de contrôle, dans le sang de l'animal immunisé, de la présence d'anticorps dirigé contre l'antigène présent dans la composition immunogène selon l'invention est réalisée après les étapes (a) ou (i) d'immunisation. Cette étape de contrôle est réalisée par des techniques classiques connues de l'homme du métier, par exemple en titrant la quantité d'anticorps dans le sérum de l'animal immunisé par ELISA.

Dans la méthode de production d'anticorps polyclonaux selon l'invention, l'étape (b) de récolte des anticorps polyclonaux induits dirigés contre ledit antigène est réalisée par des techniques classiques connues de l'homme du métier. Cette étape comprend notamment une collecte du sang de l'animal immunisé (avec ou sans sacrifice de l'animal), suivie de l'isolement du sérum qui contient les anticorps polyclonaux, et optionnellement de la purification des anticorps polyclonaux.

Dans la méthode de production d'anticorps monoclonaux selon l'invention, la récupération et l'isolement des lymphocytes B à l'étape (iii) est réalisée par des techniques classiques connues de l'homme du métier. Cette étape fait notamment intervenir le sacrifice de l'animal immunisé, suivi du prélèvement de la rate, et de l'isolement des lymphocytes B à partir de la rate prélevée.

La production d'un hybridome à l'étape (iv) est réalisée selon des techniques classique connues de l'homme du métier, et implique notamment la fusion des lymphocytes B isolés à l'étape (iii) avec un myélome, de sorte à produire un hybridome. La fusion est par exemple réalisée en utilisant du polyéthylène glycol ou par électroporation. L'hybridome ainsi obtenu est ensuite cultivé dans des conditions appropriées facilement déterminables par l'homme du métier de sorte à permettre à l'hybridome de sécréter des anticorps. Selon l'échelle de production d'anticorps désirée, cette étape de culture de l'hybridome peut notamment être réalisée en bio-réacteur.

Dans une dernière étape (v), les anticorps ainsi sécrétés sont récoltés et purifiés à l'aide de techniques classique connues de l'homme du métier, comme par exemple par chromatographie liquide haute performance, par chromatographie d'affinité en utilisant la protéine G, ou encore par précipitation à l'ammonium.

### • Utilisation en tant que médicament

L'invention a également pour objet une composition immunogène telle que définie ci-dessus pour son utilisation en tant que médicament.

L'invention concerne également une composition immunogène telle que définie ci-dessus pour son utilisation pour induire une réponse immune contre l'antigène présent dans la composition immunogène. De préférence, la composition immunogène telle que définie ci-dessus est utilisée pour induire une réponse immune contre l'antigène présent dans la composition immunogène chez un individu auquel ladite composition immunogène est administrée.

Dans certains modes de réalisation, ladite réponse immune est une réponse immune humorale contre l'antigène présent dans la composition immunogène.

Dans certains modes de réalisation, la composition immunogène selon l'invention est utilisée pour traiter ou prévenir une infection, un cancer, une maladie inflammatoire ou une allergie, selon la nature de l'antigène présent dans la composition immunogène.

Par « infection », on entend une infection causée par n'importe quel pathogène, i.e. un virus, une bactérie, une levure ou un parasite. De préférence, l'infection est une infection due à un pathogène à partir duquel au moins un antigène tel que défini ci-dessus est dérivé.

Par « allergie », on entend une allergie due à n'importe quel allergène. De préférence, l'allergie est une allergie due à un allergène à partir duquel au moins un antigène tel que défini ci-dessus est dérivé.

Par « maladie inflammatoire », on entend ici une maladie associée à une inflammation. Des exemples de maladies inflammatoires sont bien connus de l'homme du métier et incluent en particulier l'athérosclérose, l'ischémie myocardique, l'acné, l'asthme, les maladies auto-immunes, la prostatite, la glomérulonéphrite, les hypersensibilités, les maladies inflammatoires chroniques intestinales, les maladies inflammatoires pelviques, la polyarthrite rhumatoïde, le rejet de greffe, la vasculite, la cystite interstitielle, les allergies et les myopathies inflammatoires.

Par « cancer », on entend n'importe quel cancer. De préférence, le cancer est un cancer à partir duquel au moins un antigène tumoral tel que défini ci-dessus est dérivé.

La composition immunogène peut être une composition à visée prophylactique ou à visée thérapeutique, ou encore les deux.

De préférence, ladite composition immunogène selon l'invention est un vaccin.

Dans certains modes de réalisation, ladite composition est administrée à un humain, incluant un homme, une femme ou un enfant, ou à un mammifère non humain, incluant un primate (singe), un félin (chat), un canin (chien), un bovin (vache), un ovin (mouton, chèvre), un équin (cheval), un porcin (cochon), un rongeur (rat, souris, hamster, cochon d'inde), ou un lapin.

### • Méthode d'immunisation

L'invention concerne également une méthode d'immunisation contre une maladie chez individu le nécessitant comprenant l'administration à l'individu d'une composition immunogène ou d'un vaccin selon l'invention. De préférence, ladite composition immunogène ou ledit vaccin est administré(e) en une dose immunoprotectrice.

Par « individu le nécessitant », on entend un individu qui développe ou qui risque de développer une maladie. L'individu peut être un humain, incluant un homme, une femme ou un enfant, ou un mammifère non humain, incluant un primate (singe), un félin (chat), un canin (chien), un bovin (vache), un ovin (mouton, chèvre), un équin (cheval), un porcin (cochon), un rongeur (rat, souris, hamster, cochon d'inde), ou un lapin.

Dans certains modes de réalisation, la maladie est une infection, une allergie, une maladie inflammatoire ou un cancer. De préférence, ladite infection est une infection virale, bactérienne, parasitaire causée par un pathogène à partir duquel l'antigène tel que défini ci-dessus est dérivé. De préférence, ladite allergie est une allergie due à un allergène tel que défini ci-dessus. De préférence, ledit cancer est un cancer à partir duquel l'antigène tel que défini ci-dessus est dérivé.

Le mode d'administration peut être n'importe quel mode d'administration utilisé dans le domaine des vaccins. La composition immunogène peut notamment être administrée par voie intradermique, intramusculaire, topique, trans-cutanée, cutanée, mucosale, intranasale. De préférence, lorsque la composition immunogène selon l'invention est sous forme de gel, elle est administrée par voie cutanée ou mucosale.

Par « dose immunoprotectrice », on entend une quantité capable d'induire une réponse immune humorale et/ou cellulaire spécifique. La réponse immune humorale est évaluée par la détection de la présence d'anticorps neutralisants dans le sérum de l'hôte vacciné selon des techniques connues de l'homme du métier. La réponse immune cellulaire est évaluée par la détection de la présence de lymphocytes T CD4+, T CD8+, et/ou les cellules NK dans le sérum de l'hôte vacciné selon des techniques connues de l'homme du métier. La quantité de composition ou de vaccin selon la présente invention ainsi que la fréquence d'administration sera déterminée par des études cliniques, par le médecin ou par le pharmacien. La « dose immunoprotectrice » spécifique à chacun des individus pourra dépendre d'un certain nombre de facteurs comme la nature et la sévérité du désordre à traiter, la composition utilisée, l'âge, le poids, l'état de santé général, le sexe et le régime de l'individu, le mode d'administration, la durée du traitement (en monodose ou en plusieurs doses), les médicaments utilisés en combinaison et d'autres facteurs bien connus des spécialistes médicaux.

Le volume d'administration peut varier selon le mode d'administration. De préférence, lors d'une administration par voie sous-cutanée, le volume peut être compris entre 0.1 ml et 10 ml.

Le moment optimal d'administration de la composition immunogène selon l'invention est d'environ 1 semaine à 6 mois, de préférence 1 mois à 3 mois, avant la première exposition au pathogène. La composition immunogène peut être administrée en tant qu'agent prophylactique chez les hôtes à risque de développer une maladie telle que définie ci-dessus.

La composition immunogène selon l'invention peut être administrée en une dose unique, ou optionnellement, l'administration peut impliquer l'utilisation d'une première dose suivie d'une seconde dose (dose « booster ») qui est administrée, par exemple 2 à 6 mois plus tard, comme déterminé de manière appropriée par l'homme du métier.

L'invention est illustrée au vu des figures et exemples qui suivent.

### FIGURES

La figure 1 représente un schéma illustrant une coupe d'une gouttelette de composition immunogène selon l'invention. Sur la couronne sont représentés des antigènes représentés par 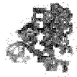 greffés de façon covalente aux gouttelettes (par les tensioactifs de formule (I)), des agents de ciblage représentés par 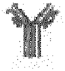 (lipide mannosylé ou anticorps) et des agents immunostimulants représentés par 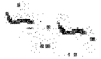
La figure 2 représente le schéma réactionnel de déprotection du précurseur du tensioactif de formule (LI') par clivage du groupe -S-Pyridinyle pour libérer la fonction thiol qui servira ultérieurement à greffer l'antigène ovalbumine (exemple 1 paragraphe 1.1.2.).
La figure 3 représente un schéma illustrant une coupe d'une gouttelette de l'émulsion prémix de l'exemple 1 paragraphe 1.1.2.. Le coeur des gouttelettes (représenté par 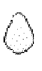 ) comprend l'huile et le lipide solubilisant, et la couronne comprend le lipide amphiphile (représenté par 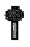 ), le co-tensioactif et le tensioactif de formule (LI') (représentés par 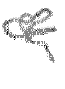 ).
La figure 4 représente le schéma réactionnel de modification chimique de l'ovalbumine pour lui greffer un groupe maléimide (servant ultérieurement à greffer l'ovalbumine modifiée aux gouttelettes de l'émulsion) et un fluorophore (exemple 1 paragraphe 1.2.1.).
La figure 5 représente le schéma réactionnel de greffage de l'ovalbumine modifiée sur une gouttelette de prémix puis blocage des fonctions thiols restantes avec Mal-OH (exemple 1 paragraphe 1.2.2.).
La figure 6 fournit l'intensité de fluorescence (ordonnée) en fonction du volume d'élution en mL (abscisses) pour l'ovalbumine (trait plein) ou pour les gouttelettes (trait en pointillés) (exemple 1 paragraphe 1.2.2.).
La figure 7 fournit le nombre moyen d'ovalbumine greffé par gouttelette (ordonnée) en fonction de la masse en mg de précurseur de tensioactif de formule (LI') dans l'émulsion (abscisses), les carrés correspondant aux points expérimentaux et la ligne en pointillé à l'extrapolation linéaire (exemple 1 paragraphe 1.2.2.).
La figure 8 est une photographie d'un gel de SDS-PAGE de 1) l'ovalbumine fonctionnalisée par le tensioactif de formule (LI') ; 2) l'ovalbumine libre ; 3) les gouttelettes fonctionnalisées par l'ovalbumine et issues d'une émulsion prémix B ; 4) les gouttelettes fonctionnalisées par l'ovalbumine et issues d'une émulsion prémix B' (exemple 1 paragraphe 1.2.2.).
La figure 9 fournit la viabilité d'une lignée de fibroblastes 3T3 en % (ordonnées) en fonction de la proportion en µg/mL d'émulsion (abscisses) (exemple 1 paragraphe 1.2.5.).
   Courbe losanges pleins, trait en pointillés : fibroblastes incubés en présence de gouttelettes possédant 0% molaire de tensioactif fonctionnalisable.
   Courbe carrés pleins, trait plein : fibroblastes incubés en présence de gouttelettes possédant 0,35% molaire de tensioactif fonctionnalisable et dont les fonctions thiols ont été « désactivées » par réaction avec un maléimide-OH.
   Courbe carrés vides, trait plein : fibroblastes incubés en présence de gouttelettes possédant 0,88% molaire de tensioactif fonctionnalisable et dont les fonctions thiols ont été désactivées par réaction avec un maléimide-OH.
   Courbe triangles pleins, trait plein : fibroblastes incubés en présence de gouttelettes possédant 0,35% molaire de tensioactif fonctionnalisable sur lesquels l'ovalbumine a été greffée.
   Courbe triangles vides, trait plein : fibroblastes incubés en présence de gouttelettes possédant 0,88% molaire de tensioactif fonctionnalisable sur lesquels l'ovalbumine a été greffée.

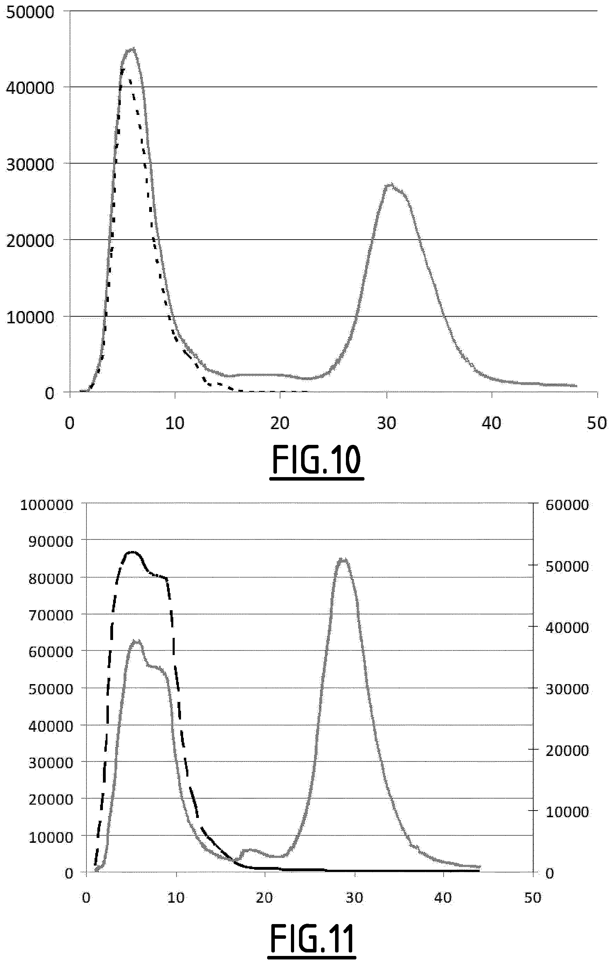

La figure 10 fournit l'intensité de fluorescence (ordonnée) en fonction du volume d'élution en mL (abscisses) pour le peptide (trait plein) ou pour les gouttelettes (trait en pointillés) (exemple 2).
La figure 11 fournit l'intensité de fluorescence (ordonnée) en fonction du volume d'élution en mL (abscisses) pour le peptide (trait plein) ou pour les gouttelettes (trait en pointillés) (exemple 3).

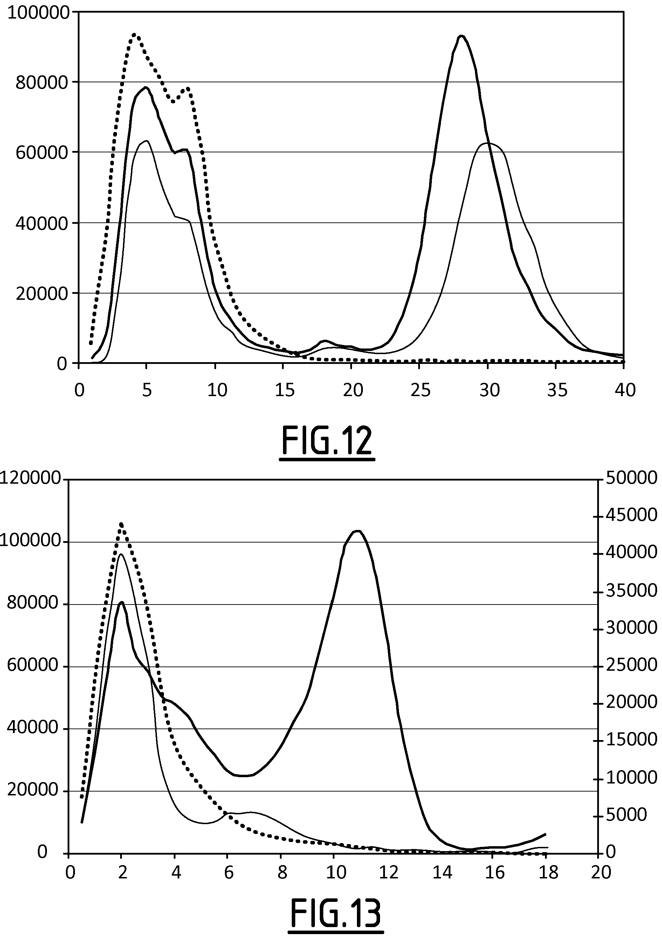

La figure 12 fournit l'intensité de fluorescence (ordonnée) en fonction du volume d'élution en mL (abscisses) pour les peptides (traits pleins) ou pour les gouttelettes (trait en pointillés) (exemple 4).
La figure 13 représente l'intensité de fluorescence (ordonnées) en fonction du volume en mL (abscisses) pour l'ovalbumine (trait plein épais), l'anticorps (trait plein fin) et les gouttelettes (trait en pointillés) (exemple 7).

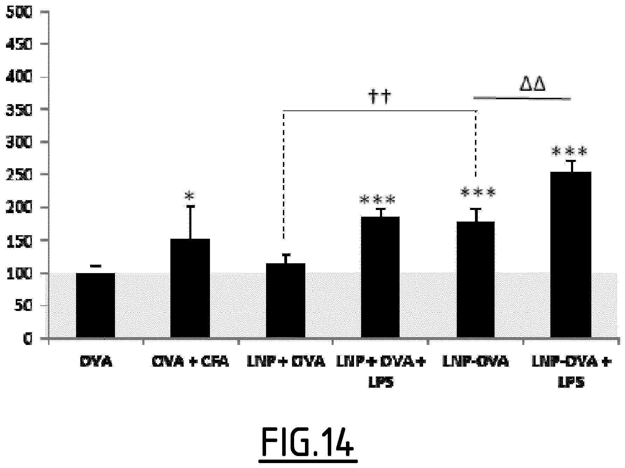

La figure 14 représente les résultats d'immunisation obtenus pour n = 4 individus, à savoir le pourcentage d'anticorps anti-ovalbuline (%OVA) (ordonnées) en fonction de la composition utilisée :
   - OVA pour l'administration d'ovalbumine seule sans vecteur (comparatif),
   - OVA + CFA pour l'administration d'ovalbumine seule sans vecteur et d'adjuvant CFA (comparatif),
   - LNP+OVA pour l'administration d'ovalbumine et de gouttelettes d'émulsion, l'ovalbumine n'étant pas liée aux gouttelettes (comparatif),
   - LNP+OVA+LPS pour l'administration d'ovalbumine, de gouttelettes d'émulsion et d'agent immunostimulant LPS, l'ovalbumine n'étant pas liée aux gouttelettes et le LPS n'étant pas incorporé dans les gouttelettes (comparatif),
   - LNP-OVA pour l'administration d'ovalbumine liée de façon covalente aux gouttelettes d'émulsion (composition immunogène selon l'invention),
   - LNP-OVA+LPS pour l'administration d'ovalbumine liée de façon covalente aux gouttelettes d'émulsion, et le LPS n'étant pas incorporé dans les gouttelettes.
   * p < 0,05 ; *** p < 0,001 comparé aux contrôles OVA ; †† p < 0,01 ; ΔΔ p < 0,01 (exemple 8).

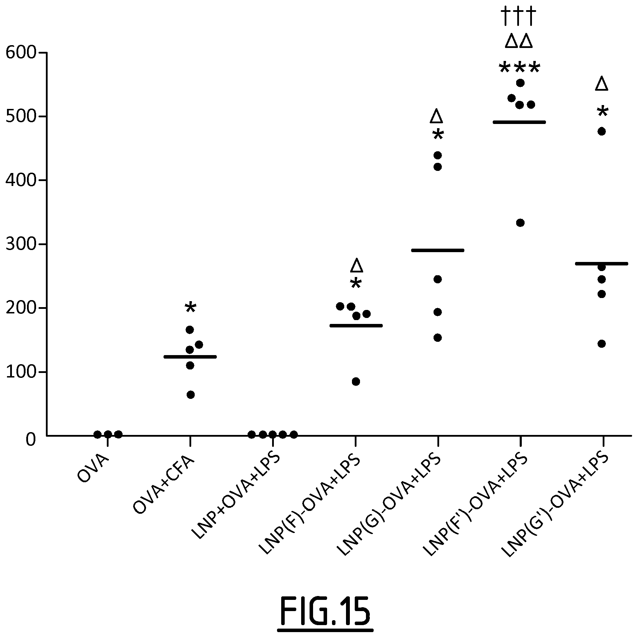

La figure 15 représente les résultats d'immunisation obtenus pour n = 5 individus, à savoir la proportion Ig total anti-OVA (ng/mL) dans le sera des souris immunisées par l'ovalbumine (ordonnées) en fonction de la composition utilisée :
   - OVA pour l'administration d'ovalbumine seule sans vecteur (comparatif),
   - OVA + CFA pour l'administration d'ovalbumine seule sans vecteur et d'adjuvant CFA (comparatif),
   - LNP+OVA+LPS pour l'administration d'ovalbumine, de gouttelettes d'émulsion F à l'exemple 1.1.2 et d'agent immunostimulant LPS, l'ovalbumine n'étant pas liée aux gouttelettes de l'émulsion et le LPS n'étant pas incorporé dans les gouttelettes de l'émulsion (comparatif),
   - LNP(F)-OVA+LPS pour l'administration de gouttelettes d'émulsion F greffées par l'ovalbumine obtenue à l'exemple 1.2.2 (composition immunogène selon l'invention) et d'agent immunostimulant LPS, le LPS n'étant pas incorporé dans les gouttelettes,
   - LNP(G)-OVA+LPS pour l'administration de gouttelettes d'émulsion G greffées par l'ovalbumine obtenue à l'exemple 1.2.2 (composition immunogène selon l'invention) et d'agent immunostimulant LPS, le LPS n'étant pas incorporé dans les gouttelettes,
   - LNP(F')-OVA+LPS pour l'administration de gouttelettes d'émulsion F' greffées par l'ovalbumine obtenue à l'exemple 1.2.2 (composition immunogène selon l'invention) et d'agent immunostimulant LPS, le LPS n'étant pas incorporé dans les gouttelettes,
   - LNP(G')-OVA+LPS pour l'administration de gouttelettes d'émulsion G' greffée par l'ovalbumine obtenue à l'exemple 1.2.2 (composition immunogène selon l'invention) et d'agent immunostimulant LPS, le LPS n'étant pas incorporé dans les gouttelettes.
   test Wilconson: *
   * p<0,05; * * * p<0,001 en comparaison avec OVA
   Δ p<0,05; ΔΔ p<0,01 en comparaison avec OVA+CFA
   ††† p<0,001 en comparaison avec LNP(F)-OVA + LPS
   (exemple 8).

### EXEMPLES

### Exemple 1 : Préparation d'une composition immunogène comprenant un tensioactif porteur d'un antigène (ovalbumine) de formule (I')

### 1.1. Préparation de l'émulsion prémix

Une émulsion prémix comprenant un tensioactif de formule (LI') dans laquelle R₂ représente C₁₇H₃₅, A₂ représente NH et n représente 100, soit de formule suivante : 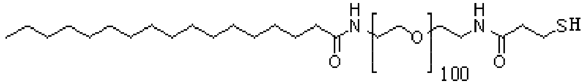 a été préparée comme suit.

### 1.1.1. Préparation d'un précurseur du tensioactif de formule (LI')

Un précurseur du tensioactif de formule (LI') dans lequel la fonction thiol terminale est protégée par un groupe -S-Pyridinyle a été préparé en suivant le schéma réactionnel suivant :

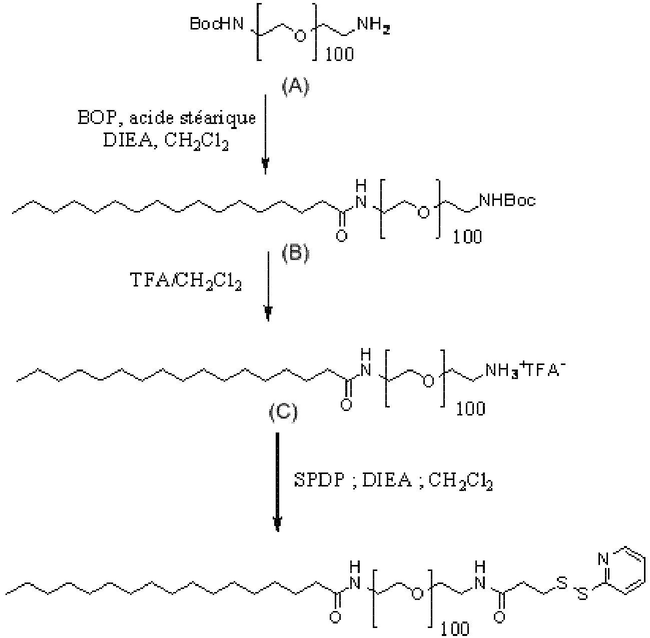

### Synthèse du composé (B)

De l'acide stéarique (2 g ; 0,6 mmol) et du Benzotriazole-1-yl-oxy-tris-(dimethylamino)-phosphonium hexafluorophosphate (BOP) (265,2 mg ; 0,6 mmol) ont été dissous dans du CH₂Cl₂ (15 mL). Après 10 minutes d'agitation, du BocNH-PEG100-NH₂ (PM : 4928 ; 2 g ; 0,4 mmol) (composé (A)) et de la diisopropyléthylamine (DIEA) (104,5 mL ; 0,6 mmol) ont été ajoutés au milieu réactionnel. La disparition de l'amine de départ a été vérifiée par chromatographie sur couche mince (CCM) (CH₂Cl₂/MeOH). Après 2 heures sous agitation, le produit a été précipité dans l'éther à froid, dissous dans un minimum d'eau puis dialyse contre de l'eau milli Q (cut off 1000). La solution a ensuite été récupérée et l'eau a été enlevée soit par évaporation (éthanol comme azéotrope) soit par lyophilisation, pour donner 1,5 g de composé (B) (poudre blanche), soit un rendement de 70 %.

CCM (CH₂Cl₂/ MeOH 9/1): Rf=0,5

RMN ¹H (300 MHz ; CDCl3) : d : 0,87 (t ; *J*=6,5 Hz ; 3H ; C**H₃**-CH₂) ; 1,24 (m ; 28H ; 14C**H₂**) ; 1,44 (s ; 9H ; C(C**H₃**)₃) ; 1,67 (quin ; 2H ; C**H₂**-CH₂-CONH) ; 2,42 (t ; *J*=7,5 Hz ; 2H ; C**H₂**-CONH) ; 3,3 (t ; J=5,0 Hz ; 2H ; BocNH-C**H₂**) ; 3,45-3,8 (m ; 362H ; xC**H₂**(PEG), CH₂CONH-C**H₂**)

### Synthèse du composé (C)

Le composé (B) (1,5 g ; 0,29 mmol) a été dissous dans 10 mL de dichlorométhane et 4 mL d'acide trifluoroacétique (TFA). La conversion en composé (C) a été suivie par CCM (révélateur ninhydrine). Après 1 heure sous agitation, le solvant a été évaporé par coévaporation avec du toluène (qui élimine le TFA). Le produit a été séché sous vide pour donner 1,3 g de composé (C) (poudre blanche), soit un rendement de 86,7 %

CCM (CH₂Cl₂/ MeOH 9/1): Rf=0,27

RMN ¹H (300 MHz ; CDCl3) : d : 0,87 (t ; *J*=6,5 Hz ; 3H ; C**H₃**-CH₂) ; 1,24 (m ; 28H ; 14C**H₂**) ; 1,60 (quin ; 2H ; C**H₂**-CH₂-CONH) ; 2,15 (t ; J=7,5 Hz ; 2H ; C**H₂**-CONH) ; 3,17 (bt ; 2H ; C**H₂**-NH₃⁺) ; 3,4 (m ; 2H ; CH₂CONH-C**H₂**) ; 3,5-3,8 (m ; 360H ; xC**H₂**(PEG)) ; 6,14 (bs ; 1 H ; N**H**CO) ; 7,9 (bs ; 2H ; N**H₂**/N**H₃+**)

### Synthèse du précurseur du tensioactif de formule (LI')

Sous argon, du composé (C) (0,5 g ; 0,1 mmol) et de la DIEA (52 mL ; 0,3 mmol) ont été dissous dans du dichlorométhane (10 mL). Après 5 minutes sous agitation du Succinimidyl 3-(2-pyridyldithio)propionate (SPDP) (93 mg ; 0,3 mmol) ont été ajoutés dans le milieu réactionnel. La disparition de l'amine a été suivie par CCM (CH₂Cl₂/MeOH 9/1). Après 1 heure de réaction, le produit a été précipité à deux reprises dans l'éther pour donner après filtration 400 mg de précurseur (poudre jaunâtre) soit un rendement de 76 % CCM (CH₂Cl₂/ MeOH 9/1): Rf=0,42

RMN 1 H (300 MHz ; CDCl3) : d : 0,88 (t ; *J*=6,5 Hz ; 3H ; C**H₃**-CH₂) ; 1,25 (m ; 28H ; 14C**H₂**) ; 1,63 (quin ; 2H ; C**H₂**-CH₂-CONH) ; 2,17 (t ; *J*=7,5 Hz ; 2H ; C**H₂**-CONH) ; 2,62 (t ; *J*=7 Hz ; 2H ; C**H₂**-SS) ; 3,09 (t ; *J*=7 Hz ; 2H ; NHCO-C**H₂**-CH₂-SS) ; 3,42 (m ; 2H ; C**H₂**-NHCO) ; 3,48-3,8 (m ; 360H ; xC**H₂**(PEG) ; C**H₂**-NHCO) ; 6,11 (bt ; 1H ; NH) ; 6,79 (bt ; 1H ; N**H**) ; 7,11 (m ; 1H ; C**H**pyr) ; 7,67 (m ; 2H ; 2C**H**pyr) ; 8,49 (m ; 1H ; CHpyr)

### 1.1.2. Préparation d'émulsions prémix comprenant le tensioactif de formule (LI')

L'émulsion prémix a été préparée en suivant les procédures décrites dans WO 2010/018223 avec les compositions indiquées dans les tableaux 2 et 3, la dissolution complète du Myrj S40 et du tensioactif de formule (LII) dans la phase aqueuse ayant nécessité de chauffer la solution à 60°C. Les phases aqueuse et huileuse sont alors mélangés puis émulsifiés par sonication selon les paramètres décrits dans le tableau 4.

**Tableau 2 : Formulation des émulsions prémix de diamètre 120 nm comprenant le précurseur du tensioactif de formule (LI')**

| | Lipide amphiphile lécithine S75 (Lipoïd) (mg) | Lipide solubilisant Suppocire® NB (Gattefossé) (mg) | Lipide cationique (DOTAP) (mg) | Huile de soja (Croda) (mg) | PBS 1X (µL) | cotensioactif MYRJ S40 (CRODA) (PEG 40) | | précurseur | | Ratio précurseur / (précurseur + cotensioactif) (%) | |
|---|---|---|---|---|---|---|---|---|---|---|---|
| | | | | | | mg | mmol | mg | mmol | % mol | % mas |
| A | 45 | 450 | 0 | 150 | 1140 | 214 | 107 | 1 | 0.19 | 0,18 | 0,47 |
| B | 45 | 450 | 0 | 150 | 1140 | 213 | 106,5 | 2 | 0.37 | 0,35 | 0,94 |
| C | 45 | 450 | 0 | 150 | 1140 | 210 | 105 | 5 | 0.94 | 0,88 | 2,33 |
| D | 45 | 450 | 0 | 150 | 1140 | 205 | 102,5 | 10 | 1.87 | 1,79 | 4,65 |
| E | 45 | 450 | 0 | 150 | 1140 | 195 | 97,5 | 20 | 3.75 | 3,70 | 9,30 |
| F | 45 | 450 | 0 | 150 | 1140 | 210 | 105 | 5 | 0.94 | 0,88 | 2,33 |
| G | 11 | 450 | 34 | 150 | 1140 | 210 | 105 | 5 | 0.94 | 0,88 | 2,33 |

| | | | | | | | | | | | |
|---|---|---|---|---|---|---|---|---|---|---|---|
| Dans le tableau 2, « précurseur » signifie « précurseur du tensioactif de formule (LI'), « %mol » signifie %molaire et « %mas » signifie %massique. Dans les 7 émulsions prémix, la masse totale de (précurseur du tensioactif de formule (LI') + cotensioactif) est toujours de 215 mg. | | | | | | | | | | | |

**Tableau 3 : Formulation des émulsions prémix de diamètre 80 nm comprenant le précurseur du tensioactif de formule (LI').**

| | Lipide amphiphile lécithine S75 (Lipoïd) (mg) | Lipide solubilisant Suppocire® NB (Gattefossé) (mg) | Lipide cationique (DOTAP) (mg) | Huile de soja (Croda) (mg) | PBS 1X (µL) | cotensioactif MYRJ S40 (CRODA) (PEG 40) | | précurseur | | Ratio Précurseur / (précurseur + cotensioactif) (%) | |
|---|---|---|---|---|---|---|---|---|---|---|---|
| | | | | | | mg | mmol | mg | mmol | % mol | % mass |
| A' | 50 | 307,5 | 0 | 102,5 | 1240 | 299 | 149,5 | 1 | 0,19 | 0,13 | 0,33 |
| B' | 50 | 307,5 | 0 | 102,5 | 1240 | 298 | 149 | 2 | 0,37 | 0,25 | 0,67 |
| C' | 50 | 307,5 | 0 | 102,5 | 1240 | 295 | 147,5 | 5 | 0,94 | 0,63 | 1,67 |
| D' | 50 | 307,5 | 0 | 102,5 | 1240 | 290 | 145 | 10 | 1,87 | 1,28 | 3,33 |
| E' | 50 | 307,5 | 0 | 102,5 | 1240 | 280 | 140 | 20 | 3,75 | 2,61 | 6,67 |
| F' | 50 | 307,5 | 0 | 102,5 | 1240 | 295 | 147,5 | 5 | 0,94 | 0,63 | 1,67 |
| G' | 12 | 307,5 | 38 | 102,5 | 1240 | 295 | 147,5 | 5 | 0,94 | 0,63 | 1,67 |

| | | | | | | | | | | | |
|---|---|---|---|---|---|---|---|---|---|---|---|
| Dans le tableau 3, « précurseur » signifie « précurseur du tensioactif de formule (LI'), « %mol » signifie %molaire et « %mas » signifie %massique. Dans les 7 émulsions prémix, la masse totale de (précurseur du tensioactif de formule (LI') + cotensioactif) est de 300 mg. | | | | | | | | | | | |

L'augmentation de la quantité de précurseur du tensioactif de formule (LI') dans les émulsions n'a pu être faite que pour des rapports molaires tensioactif de formule (LI') + cotensioactif) / (précurseur du tensioactif de formule (LI') + cotensioactif) inférieurs à 5%. Au-delà de ces rapports molaires, les émulsions ne sont pas stables et les gouttelettes s'agrègent pour former un milieu très visqueux qui ne peut pas être utilisé pour le greffage de l'antigène.

**Tableau 4 : Paramètres de sonication utilisés avec un sonificateur AV505® (Sonics, Newtown, USA)**

| Sonde (φ) | Puissance Pmax | Temps de sonication | Pulse on/off |
|---|---|---|---|
| 3 mm | 28% | 20 min | 10s / 30s |

Les émulsions prémix ainsi produites comprennent des gouttelettes comprenant un précurseur du tensioactif de formule (LI') dont la fonction thiol est protégée par le groupe -S-Pyridinile qui ont été déprotégées pour pouvoir greffer l'anticorps de façon covalente aux gouttelettes.

Pour ce faire, les émulsions prémix ont été incubées avec 4 mg de dithiothréitol pendant 1 heure sous agitation magnétique à température ambiante. Les émulsions prémix ont ensuite purifiées par dialyse (seuil de coupure : 12 kDa, contre du PBS 1X, quatre fois 1 h puis une nuit) pour éliminer les composants non-intégrés aux gouttelettes ainsi que les produits secondaires issus de la déprotection, comme illustré sur la figure 2. Une coupe d'une gouttelette d'une émulsion prémix est schématisée sur la figure 3.

Afin de vérifier que les tensioactifs de formule (LI') étaient bien incorporés aux gouttelettes et porteurs de fonctions thiols libres, un fluorophore-maléimide (Fluoprobe 647-H maleimide d'Interchim) a été greffé sur ces fonctions thiols afin de les doser. Les résultats, fournis au tableau 5, ont démontré plus de 95% des tensioactifs de formule (LI') sont incorporés dans les gouttelettes sous leur forme -SH.

**Tableau 5 : Dosage des fonctions -SH sur les gouttelettes**

| Emulsions prémix | A | B | C | D | A' | B' | C' | D' |
|---|---|---|---|---|---|---|---|---|
| Quantité de précurseur du tensioactif de formule (LI') introduits initialement dans la formulation (mg) | 1 | 2 | 5 | 10 | 2,5 | 5 | 7,5 | 10 |
| %age de précurseur du tensioactif de formule (LI') réellement incorporé dans les gouttelettes | 96,8 | 96,3 | 97,4 | 98,4 | 40,2 | 40,1 | 59,7 | 71,5 |
| Nombre moyen de fonctions -SH par gouttelettes théorique pour une incorporation de 100% | 125 | 250 | 625 | 1260 | 104 | 209 | 313 | 418 |
| Nombre moyen de fonctions -SH par gouttelettes réel tenant compte de l'incorporation réelle | 121 | 241 | 609 | 1238 | 42 | 84 | 187 | 299 |
| Taille moyenne (nm) après déprotection | 116,6 ± 2,4 | 118,4 ± 1,8 | 115,1 ± 2,7 | 112,2 ± 2,5 | 78,9 ± 0,7 | 75,9 ± 4,9 | 77,5 ± 0,5 | 74,1 ± 5,2 |

De plus, les tailles des gouttelettes des 8 émulsions prémix A, B, C, D, A', B', C' et D' du tableau 5 ont été mesurées par DLS (appareil Zetasizer Nano ZS de chez Malvern Instruments, UK). Les gouttelettes des 4 émulsions prémix A, B, C et D ont un diamètre de l'ordre de 120 nm. Les gouttelettes des 4 émulsions prémix A', B', C' et D'ont un diamètre de l'ordre de 80 nm.

### 1.2. Greffage de l'ovalbumine sur les gouttelettes des émulsions prémix préparées selon 1.1.

### 1.2.1. Préparation du composé de formule (LII') par modification chimique de l'ovalbumine pour y greffer le groupe G₂ de type maléimide

L'ovalbumine a été choisie comme antigène modèle à greffer sur les gouttelettes car elle est connue pour posséder deux épitopes de classes différentes connues pour produire une réponse cellulaire (MHC-I) et une réponse humorale (MHC-II) :
- OVA 257-264 : SIINFEKL (SEQ ID N° 1),
- OVA 323-339: ISQAVHAAHAEINEAGR (SEQ ID N°2).
C'est une protéine globulaire de 45 kDa avec un pH isoélectrique de 4.5. Elle possède 6 fonctions cystéine dont aucune n'est accessible chimiquement sans dénaturation préalable de la protéine et 20 fonctions lysine dont 3 seulement sont accessibles chimiquement sans dénaturation préalable de la protéine (Steven et al., Biochem J., 1958, 70, 179-182).

Afin de greffer l'ovalbumine sur les fonctions thiols présentes en surface des gouttelettes des émulsions prémix préparées selon le paragraphe 1.1., il a été nécessaire d'introduire une ou plusieurs fonctions maléimide sur la protéine, via un linker bifonctionnel : le Sulfosuccinimidyl 4-(N-maleimidomethyl)cyclohexane-1-carboxylate (Sulfo-SMCC) selon le schéma réactionnel suivant. Pour cela, on a fait réagir l'ovalbumine en solution dans le PBS 1X avec 10 à 50 équivalents de Sulfo-SMCC sous agitation magnétique pendant 1 h à température ambiante.

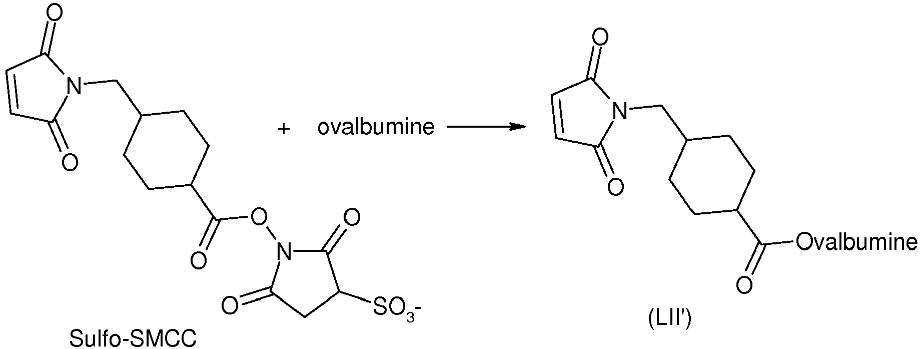

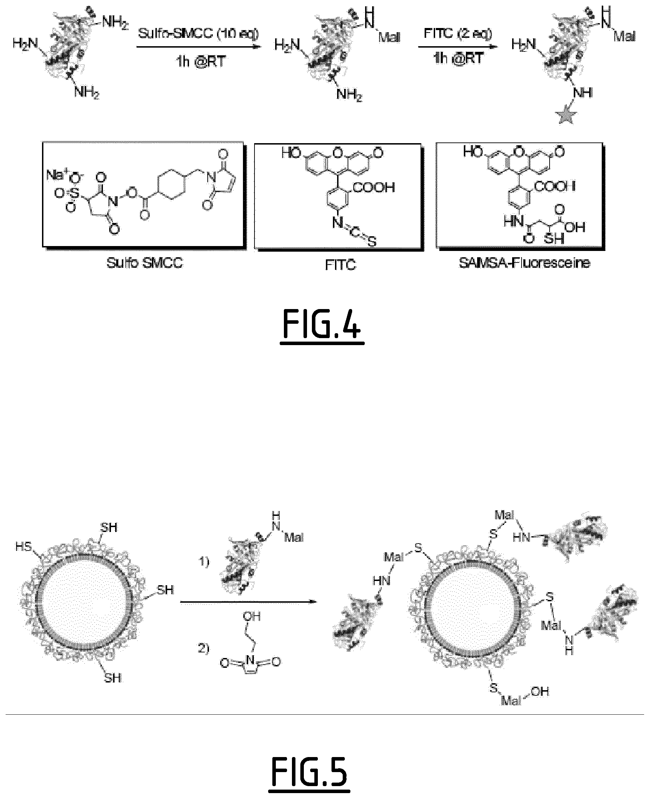

Afin de pouvoir quantifier ultérieurement le rendement de la réaction de modification chimique de l'ovalbumine, un échantillon a été prélevé et l'ovalbumine a été marquée avec un fluorophore de type NHS-ester ou isothiocyanate, par exemple du FITC, en ajoutant ce dernier au milieu réactionnel pour une heure supplémentaire.
La protéine a ensuite été séparée de l'excès de réactifs par chromatographie d'exclusion stérique sur colonne PD-10.
Afin de quantifier le nombre de fonctions maléimides réactives introduites sur la protéine, on a dosé ces dernières en fluorescence par réaction avec un fluorophore porteur d'une fonction thiol tel que la SAMSA-Fluoresceine comme illustré sur la figure 4.

Ce dosage en fluorescence a donné les résultats suivants pour 10 et 50 équivalents de Sulfo-SMCC :
- 10 eq. : 0,73 maléimide par ovalbumine (soit 24 % de rendement de fonctionnalisation)
- 50 eq. : 1,23 maléimide par ovalbumine (soit 41 % de rendement de fonctionnalisation) Ces conditions semblent donc idéales pour obtenir une moyenne de un maléimide par ovalbumine et donc éviter la formation de liaisons covalentes entre les gouttelettes : gouttelette-ovalbumine-gouttelette lors du greffage.

### 1.2.2. Procédé de greffage - Réaction entre le tensioactif de formule (LI') porteur d'un groupe G₁ de type -SH et le composé de formule (LII') porteur d'un groupe G₂ de type maléimide.

Le composé de formule (LII') (ovalbumine fonctionnalisée par un malémide) a été purifié, en solution dans le PBS 1X, et a été placé sous agitation magnétique à 0-4°C dans un bain d'eau glacée. L'émulsion prémix a alors été ajoutée lentement au goutte à goutte avec un ratio composé de formule (LII')/thiol contenu dans l'émulsion prémix de 1,1/1. Le greffage a lieu par formation du tensioactif de formule (I) suivante :

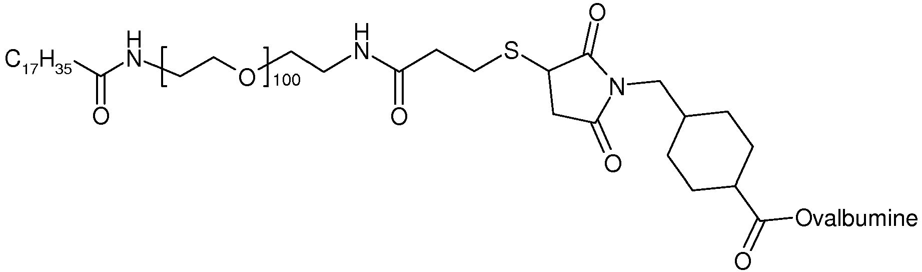

L'agitation a été maintenue plusieurs heures jusqu'à ce que la température remonte à 20°C puis on a ajouté de la 1-(2-hydroxyethyl)-1H-pyrrole-2,5-dione (Mal-OH) avec un ratio Mal-OH/thiol de 3/1 de façon à consommer les fonctions thiols n'ayant pas réagi, comme illustré sur la figure 5.

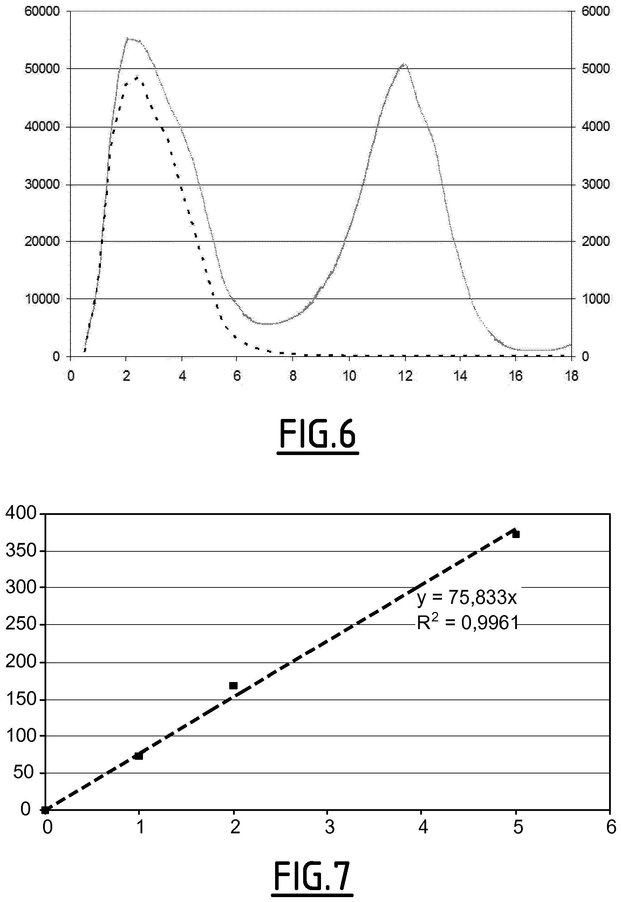

Les émulsions obtenues ont alors été purifiées par chromatographie d'exclusion stérique sur résine Superdex 200 en récoltant des fractions de 500 µL après le passage du volume mort de la colonne. Les profils d'élution des gouttelettes chargées en fluorophores et de la protéine marquée ont été suivis en fluorescence. Un exemple de résultat de fluorescence est donné en Figure 6.
Le signal de fluorescence de l'ovalbumine greffée est corrélé au signal de fluorescence des gouttelettes. Le pourcentage ovalbumine greffée/ovalbumine libre a été obtenu en faisant le rapport entre les aires sous la courbe pour l'ovalbumine greffée et non greffée. On a alors calculé le nombre de moles d'ovalbumine greffée, le rendement de fonctionnalisation et le nombre moyen d'ovalbumine par gouttelette. Pour une même émulsion prémix, la relation entre le nombre moyen d'ovalbumine par gouttelette et la quantité de thiols disponibles est linéaire, comme le montre l'exemple de l'émulsion B indiqué en figure 7.

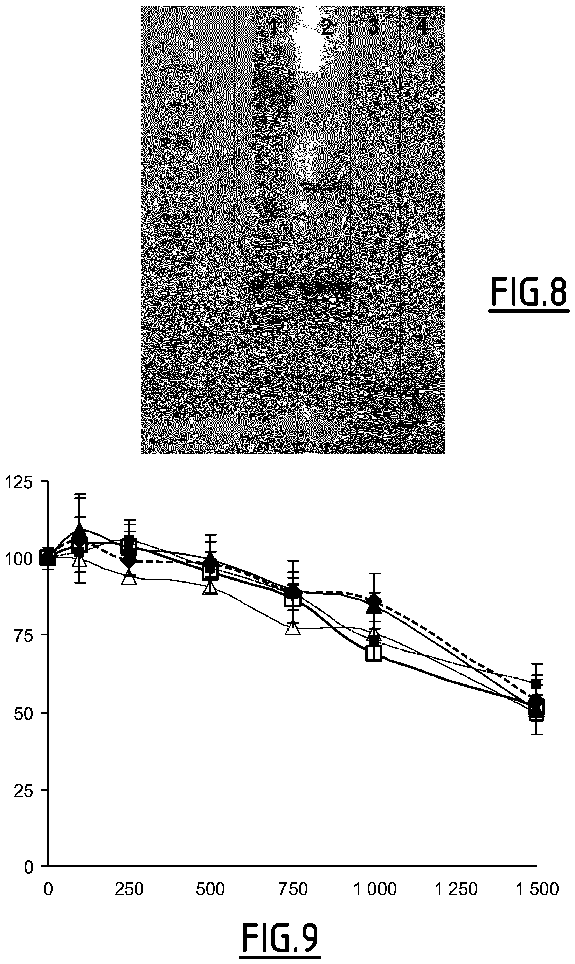

En considérant que l'ovalbumine est une protéine globulaire de diamètre 6 nm (d'après Malvern) il a été possible de calculer le pourcentage de surface d'une gouttelette couverte par l'ovalbumine greffée. On a obtenu une couverture de surface maximale de 23%.
Les gouttelettes porteuses d'ovalbumine ont été analysées en SDS-PAGE après purification par chromatographie d'exclusion stérique. Aucune raie spécifique à l'ovalbumine n'apparait dans les gouttelettes purifiées, ce qui montre l'efficacité de la séparation par chromatographie d'exclusion stérique (figure 8).

### 1.2.3. Caractérisation physicochimique des émulsions obtenues

Le diamètre hydrodynamique et le potentiel de surface des gouttelettes sur lesquelles l'ovalbumine a été greffée ont été déterminés par DLS/ELS (appareil Zetasizer Nano ZS de chez Malvern Instruments, UK).
Une augmentation du diamètre hydrodynamique a été observée après greffage de l'ovalbumine (Tableau 6). Cette augmentation dépend du nombre d'ovalbumine greffé sur les gouttelettes et semble atteindre un plateau.

**Tableau 6 : Propriétés physiques des gouttelettes avant et après greffage de l'ovalbumine.**

| Emulsion prémix utilisée | A | B | C | B' |
|---|---|---|---|---|
| Quantité de précurseur de tensioactif de formule (LI') introduit initialement dans l'émulsion prémix (mg) | 1 | 2 | 5 | 5 |
| Nombre moyen d'ovalbumine par gouttelette | 74 | 168 | 373 | 59 |
| Diamètre hydrodynamique avant greffage (nm) | 113,0 ± 0,6 | 115,0 ± 2,1 | 112,4 ± 2,3 | 81,2 ± 2,1 |
| Diamètre hydrodynamique après greffage (nm) | 133,4 ± 3,3 | 139,3 ± 5,3 | 161,2 ± 5,2 | 106,2 ± 2,6 |
| Pdl avant greffage | 0,104 ± 0,016 | 0,087 ± 0,007 | 0,099 ± 0,003 | 0,170 ± 0,005 |
| Pdl après greffage | 0,107 ± 0,002 | 0,103 ± 0,012 | 0,192 ± 0,016 | 0,233 ± 0,005 |

| Emulsion prémix utilisée | F | G | F' | G' |
|---|---|---|---|---|
| Quantité de précurseur de tensioactif de formule (Ll') introduit initialement dans l'émulsion prémix (mg) | 5 | 5 | 5 | 5 |
| Nombre moyen d'ovalbumine par gouttelette | 373 | 373 | 60 | 60 |
| Diamètre hydrodynamique avant greffage (nm) | 119 ± 1 | 123 ± 3 | 81 ± 2 | 82 ± 3 |
| Diamètre hydrodynamique après greffage (nm) | 157 ± 2 | 160 ± 4 | 98 ± 3 | 103 ± 2 |
| Pdl avant greffage | 0,129 ± 0,014 | 0,114 ± 0,015 | 0,170 ± 0,005 | 0,146 ± 0,010 |
| Pdl après greffage | 0,196 ± 0,013 | 0,198 ± 0,017 | 0,208 ± 0,011 | 0,187 ± 0,006 |
| Potentiel zêta (Zp) avant greffage | -6 ± 1 | + 6 ± 1 | -7 ± 3 | + 6 ± 1 |
| Potentiel zêta (Zp) après greffage | - 8 ± 1 | + 4 ± 1 | - 8 ± 1 | + 4 ± 1 |

Malgré l'augmentation de taille observée après le greffage de l'ovalbumine, le diamètre hydrodynamique est resté dans une gamme de taille intéressante pour les applications en vaccination, c'est-à-dire une taille de gouttelette inférieure à 200 nm pour favoriser l'internalisation cellulaire.

En ce qui concerne le potentiel de surface des gouttelettes, il est resté le même avant et après greffage, à savoir compris entre -7 et -10 mV (mesuré dans le PBS 0.1 X).

### 1.2.4. Stabilité colloïdale des émulsions obtenues

La stabilité des gouttelettes sur lesquelles l'ovalbumine a été greffée possédant le taux de charge en ovalbumine le plus élevé (émulsion C greffée par l'ovalbumine obtenue en 1.2.2.) a été vérifiée en observant l'évolution du diamètre hydrodynamique, du potentiel de surface Pdl et du potentiel zeta des gouttelettes placées à 4°C pendant 150 jours. Aucune évolution de ces paramètres dans le temps n'a été observée, ce qui démontre la stabilité des émulsions.

### 1.2.5. Toxicité des gouttelettes sur lesquelles l'ovalbumine a été greffée

Des gouttelettes sur lesquelles l'ovalbumine a été greffée (émulsion A greffée par l'ovalbumine et émulsion C greffée par l'ovalbumine obtenue en 1.2.2., Figure 9 courbes avec les triangles) ont été soumises à un test de toxicité afin de vérifier leur impact sur une lignée de fibroblastes 3T3. Des émulsions exemptes de tensioactif de formule (I) ou (LI') (sans ovalbumine, Figure 9 courbes avec les carrés, et sans tensioactif fonctionnalisable, Figure 9 courbe trait en pointillés, losanges) ont été utilisées comme contrôle. La concentration en gouttelettes (en µg/mL) est indiquée en abscisses et la viabilité cellulaire (en % de cellules vivantes) est indiquée en coordonnées. L'IC50 déterminé par cette méthode était le même pour toutes les gouttelettes sur lesquelles l'ovalbumine a été greffée et identique à celui des émulsions contrôles. Ceci démontre que le greffage en surface de l'ovalbumine n'affecte en rien la bonne tolérance des fibroblastes 3T3 à ces dernières.

### Exemple 2 : Préparation d'une composition immunogène comprenant un tensioactif porteur d'un antigène (peptide) de formule (I')

Le peptide qui a été utilisé dans ces expériences constitue l'épitope à réponse cellulaire (MHC-I) de l'ovalbumine, soit la séquence OVA 257-264 : S-I-I-N-F-E-K-L (8 mers) SEQ ID NO :1. Ce peptide a été modifié de façon à y greffer un fluorophore : la 6-carboxy-fluorescine (Fam™), par ajout d'une lysine à l'extrémité C-terminale, et une fonction maléimide (Mal) sur l'extrémité N-terminale par le sous-traitant Smartox Biotechnology. Le peptide obtenu possède donc la séquence suivante : Mal-S-I-I-N-F-E-K-L-K-6-carboxy- fluorescine (SEQ ID NO :3).
Ce peptide a été mis à réagir avec une émulsion prémix B telle que préparée dans l'exemple 1 (voir 1.1.2.) mais encapsulant en plus un agent d'intérêt : un fluorophore, le DiD. La purification du mélange réactionnel par chromatographie d'exclusion stérique sur gel a alors montré un signal de fluorescence du peptide (Figure 10, trait plein) corrélé à un signal de fluorescence des gouttelettes (Figure 10, trait en pointillés). Selon les résultats de la Figure 10, on estime que chaque gouttelette porte en moyenne 181 peptides (rendement de couplage de 60%).

### Exemple 3 : Préparation d'une composition immunogène comprenant un tensioactif porteur d'un antigène (peptide) de formule (I')

Le peptide qui a été utilisé dans ces expériences constitue l'épitope à réponse humorale (MHC-II) de l'ovalbumine, soit la séquence OVA 323-339 : I-S-Q-A-V-H-A-A-H-A-E-I-N-E-A-G-R (17 mers) (SEQ ID NO :2). Le peptide Ova 323-339 a été modifié de façon à y greffer un fluorophore : la carboxytetramethylrhodamine (Tamra™) sur l'extrémité N-terminale et une fonction maléimide (Mal) sur l'extrémité C-terminale par le sous-traitant Smartox Biotechnology. Le peptide obtenu possède donc la séquence suivante : Mal-I-S-Q-A-V-H-A-A-H-A-E-I-N-E-A-G-R-carboxytetramethylrhodamine (SEQ ID NO:4). Ce peptide a été mis à réagir avec une émulsion prémix B telle que préparée dans l'exemple 1 (voir 1.1.2.) mais encapsulant un fluorophore, le DiD. La purification du mélange réactionnel par chromatographie d'exclusion stérique sur gel a alors montré un signal de fluorescence du peptide (Figure 11, trait plein) corrélé à un signal de fluorescence des gouttelettes (Figure 11, trait en pointillés). Selon les résultats de la Figure 11, on estime que chaque gouttelette porte en moyenne 99 peptides (rendement de couplage de 40%).

### Exemple 4 : Préparation d'une composition immunogène comprenant un tensioactif porteur de deux antigènes (peptides) de formule (I')

Les peptides qui ont été utilisés dans ces expériences constituent l'épitope à réponse cellulaire (MHC-I) de l'ovalbumine, soit la séquence OVA 257-264 S-I-I-N-F-E-K-L (8 mers) (SEQ ID NO :1) et l'épitope à réponse humorale (MHC-II) de l'ovalbumine, soit la séquence OVA 323-339 I-S-Q-A-V-H-A-A-H-A-E-I-N-E-A-G-R (17 mers) (SEQ ID NO :2). Le peptide OVA 257-264 a été modifié de façon à y greffer un fluorophore : la 6-carboxy-fluorescine (Fam™), par ajout d'une lysine à l'extrémité C-terminale, et une fonction maléimide (Mal) sur l'extrémité N-terminale par le sous-traitant Smartox Biotechnology. Le peptide obtenu possède donc la séquence suivante : Mal-S-I-I-N-F-E-K-L-K-6-carboxy-fluorescine (SEQ ID NO :3). Le peptide Ova 323-339 a été modifié de façon à y greffer un fluorophore : la carboxytetramethylrhodamine (Tamra™) à l'extrémité N-terminale et une fonction maléimide (Mal) à l'extrémité C-terminale par le sous-traitant Smartox Biotechnology. Le peptide obtenu possède donc la séquence suivante : Mal-I-S-Q-A-V-HA-A-H-A-E-I-N-E-A-G-R-carboxytetramethylrhodamine (SEQ ID NO :4). Ces peptides sont alors mis à réagir avec une émulsion prémix B telle que préparée dans l'exemple 1 (voir 1.1.2.) mais encapsulant un fluorophore, le DiD. La purification du mélange réactionnel par chromatographie d'exclusion stérique sur gel a alors montré un signal de fluorescence des peptides (Figure 12, deux courbes en traits pleins) corrélé à un signal de fluorescence des gouttelettes (Figure 12, trait en pointillés). Selon les résultats de la Figure 12, on estime que chaque gouttelette porte en moyenne 76 peptides OVA 257-264 et 51 peptides OVA 323-339 (rendement de couplage de 43% pour chacun des peptides).

### Exemple 5 : Préparation d'une composition immunogène comprenant un tensioactif porteur d'un antigène de formule (I') et un agent immunostimulant : le MPLA

Le second volet consiste, après avoir greffé de façon covalente l'antigène ovalbumine dans les gouttelettes, à incorporer dans les gouttelettes un immuno-adjuvant MPLA qui va augmenter la réponse immunitaire. Cet adjuvant a été inséré dans la couronne des gouttelettes.

### 5.1. Encapsulation du MPLA dans des émulsions prémix comprenant le tensioactif de formule (LI')

Le MPLA (ou lipide A) est un lipide immunoadjuvant, autorisé depuis 2009 par la FDA pour une utilisation chez l'homme (Cervarix, Papillomavirus, GSK). Il est l'un des constituants principaux du Lipopolysaccharide (LPS) qui constitue lui même en partie la paroi des bactéries.
Une fois purifié, le lipide A présente toujours une activité immunostimulante comme le LPS mais avec une toxicité moindre, ce qui en fait un adjuvant de premier choix. De par sa nature lipidique, il s'insère très bien à la surface des nano-objets hydrophobes tels que les nanoparticules de PLGA, ou encore les liposomes, avec des rendements d'encapsulation allant de corrects à très bons. Le MPLA est généralement encapsulé à des taux de charge massiques allant de 0,1 à 1 %, taux de charges qui suffisent à observer un effet positif sur l'activation du système immunitaire.
Pour encapsuler le MPLA dans les gouttelettes, ce dernier a été dissous dans la phase huileuse fondue avant sonication. Le MPLA a été encapsulé à des taux de charge massiques théoriques de 0,1, 0,4 et 1,3 % pour lesquels peu de modifications des propriétés physicochimiques ont été observées. La taille des gouttelettes contenant le MPLA n'augmente significativement que pour un taux de charge massique théorique de 1.3 % (Tableau 7).

**Tableau 7 : caractérisation physicochimique des gouttelettes comprenant du MPLA encapsulé (dites « LNP(MPLA) ») à différents taux de charge.**

| DLE théorique MPLA (% m/m) | Diamètre hydrodynamique (nm) | Pdl |
|---|---|---|
| 0 | 109,2 ± 0,8 | 0,104 ± 0,027 |
| 0,1 | 109,1 ± 0,4 | 0,096 ± 0,011 |
| 0,4 | 124,7 ± 4,8 | 0,154 ± 0,007 |
| 1,3 | 133,6 ± 1,1 | 0,181 ± 0,013 |

De ce fait, le taux de charge de 0,4 % a été conservé par la suite. Le dosage du MPLA par des dosages d'endotoxines via le kit LAL (Lysat de Limulus Amebocyte) montre un rendement d'incorporation de 95% soit un taux de charge de 0,38 %.

### 5.2. Greffage de l'ovalbumine sur les gouttelettes des émulsions prémix préparées selon 5.1.

Par la suite, des gouttelettes contenant le MPLA à 0,4 % (m/m) et le précurseur du tensioactif de formule (LI') (porteur du groupe terminal SPDP) (5 mg introduits dans la formulation) ont été préparées, activées et purifiées comme décrit dans l'exemple 1. Le greffage et la purification ont été réalisés dans les mêmes conditions. Les émulsions obtenues présentent des propriétés physicochimiques identiques aux émulsions préparées sans MPLA.
Le greffage de l'ovalbumine sur les gouttelettes comprenant du MPLA encapsulé (dites « LNP(MPLA) ») a alors été réalisé comme décrit dans l'exemple 1 et les gouttelettes comprenant du MPLA et de l'ovalbumine (dites « LNP-MPLAOva ») obtenues possèdent des propriétés physicochimiques proches des LNP-Ova.
La réaction de greffage a conduit à un nombre moyen d'ovalbumine par LNP(MPLA) de 413, proche des 373 obtenus dans les mêmes conditions pour les LNP sans MPLA selon l'exemple 1.

### 5.3. Stabilité colloïdale des LNP(MPLA)-Ova

L'étude de la stabilité à 4°C de ces LNP(MPLA)-Ova n'a pas montré de modification particulière au niveau des caractéristiques physicochimiques et confirme que la modification de surface par l'ovalbumine ainsi que l'intercalation du lipide A au sein de la couronne des gouttelettes ne diminue pas leur stabilité colloïdale (Tableau 8).

**Tableau 8 : Stabilité des LNP(MPLA)-Ova conservées à 4°C**

| Temps à 4°C (jours) | Diamètre hydrodynamique (nm) | Pdl |
|---|---|---|
| 0 | 148,6 ± 3,0 | 0,120 ± 0,011 |
| 50 | 151,1 ± 4,1 | 0,147 ± 0,005 |

### Exemple 6 : Ciblage des cellules immunes par une composition comprenant un agent biologique de ciblage : le Mannan (LNP-Mannan)

Le mannan est un polymère linéaire de mannose. Il est produit par *Saccharomyces Cerevisiae* et ne possède pas de masse molaire moyenne définie.

Le mannan a été fonctionnalisé de la même façon que l'ovalbumine (voir 1.2.1) et mis à réagir avec une émulsion prémix B (voir 1.1). Les gouttelettes obtenues possèdent un taux de charge en Mannan de 0,5% et ont été testées. Pour ce taux de charge, aucune modification de taille ou de surface significative n'est apparue.
Des macrophages ont été incubés avec des LNP-Mannan contenant un fluorophore (DiD) puis la fluorescence au sein des cellules a été mesurée par cytométrie en flux après une heure et trois heures d'incubation. Les résultats montrent que les gouttelettes porteuses de Mannan sont significativement plus captées par les macrophages après une heure d'incubation (Figure 9 et tableau 9). De plus, au bout de 3h d'incubation, l'intensité moyenne de fluorescence dans les cellules en contact avec les LNP-Mannan est significativement plus importante, ce qui montre que ces cellules ont captés plus de gouttelettes que les cellules mises en contact avec les gouttelettes contrôles.

**Tableau 9 : résultats de capture cellulaire sur les macrophages**

| | Proportion de cellules positives après 1h d'incubation | Proportion de cellules positives après 3h d'incubation | Intensité moyenne de fluorescence par cellule après 1h d'incubation | Intensité moyenne de fluorescence par cellule après 3h d'incubation |
|---|---|---|---|---|
| Contrôle | 0,3 ± 0,01 % | 73,2 ± 0,7 % | 1873 ± 225 | 1843 ± 25 |
| Mannan | 43,5 ± 0,6 % | 85,2 ± 1,5 % | 1442 ± 44 | 2593 ± 36 |

### Exemple 7 : Préparation d'une composition immunogène comprenant un tensioactif porteur d'un antigène (ovalbumine) de formule (I') et un tensioactif porteur d'un anticorps de formule (I')

Une possibilité pour cibler les cellules du système immunitaire a été de réaliser un greffage covalent d'anticorps sur les fonctions thiols du tensioactif de formule (LI') tout en maintenant la possibilité de greffer l'ovalbumine de façon covalente aux gouttelettes par la suite.
Pour se faire, un anticorps modèle fonctionnalisé par le Sulfo-SMCC, le Cetuximab, et portant un fluorophore A, l'Alexa700-NHS, a été mis à réagir avec une émulsion prémix telle que préparée dans l'exemple 1 mais encapsulant en plus un fluorophore B, le DiO, dans un rapport stoechiométrique Cetuximab/SH 1/10, tel que seule une faible proportion des fonctions thiols sera occupée par l'anticorps. Le milieu réactionnel a alors été mis à réagir, sans purification préalable, avec l'ovalbumine modifiée chimiquement pour porter :
- un groupe maléimide permettant le greffage aux fonctions thiols du tensioactif de formule (LI') restant (modification chimique comme dans l'exemple 1 (voir 1.2.1.)) et
- un fluorophore C, le Cy5-NHS.
Les fluorophores A, B et C ont été choisis de telle manière qu'il n'était pas possible d'avoir de transfert d'énergie entre eux. La purification du mélange réactionnel final par chromatographie d'exclusion stérique sur gel a alors montré l'existence de gouttelettes bi-fonctionnalisées porteuses à la fois d'anticorps et d'ovalbumine (signal de fluorescence A, Figure 13, courbe en trait plein fin) corrélé à un signal de fluorescence B, Figure 13, courbe en trait en pointillés et à un signal de fluorescence C, Figure 13, courbe en trait plein épais), comme illustré en figure 13. Selon les résultats de la Figure 13, on estime que chaque gouttelette porte en moyenne 44 anticorps (rendement de couplage de 80 %) et 244 ovalbumines (rendement de couplage de 42 %).

### Exemple 8 : Utilisation biologique de la composition immunogène selon l'exemple 1 Immunisation - validation in vivo des gouttelettes sur lesquelles l'ovalbumine a été greffée

Ci-après, les gouttelettes sur lesquelles l'ovalbumine a été greffée obtenues dans l'exemple 1 sont nommées « LNP-OVA ».

Des souris BALB de 8 semaines ont reçu une première injection de LNP-Ova avec ou sans LPS. Les contrôles négatifs sont obtenus en injectant l'ovalbumine libre avec ou sans LPS ainsi que l'ovalbumine libre accompagné de gouttelettes LNP « nues » (c'est à dire sans protéine greffée à leur surface) avec ou sans LPS. Au bout de 21 jours, une deuxième injection de rappel est effectuée et les souris sont sacrifiées au 28ème jour. Les taux d'anticorps Anti-Ova dans les *sera* sont alors déterminés par ELISA.

### Pourcentage d'anticorps anti-ovalbuline (%OVA) en fonction de la composition utilisée (figure 14)

Les gouttelettes LNP sont celles de l'émulsion greffée par l'ovalbumine obtenue à l'exemple 1.2.2
L'immunisation avec l'ovalbumine libre sans LPS donne une réponse immunitaire faible normalisée à 100% (OVA, Figure 14). L'ajout d'un adjuvant vétérinaire classique (CFA : adjuvant complet de Freund) à l'injection d'ovalbumine donne une réponse immunitaire plus forte mais avec une forte hétérogénéité interindividuelle (OVA+CFA, Figure 14). Un contrôle négatif a été réalisé en injectant des gouttelettes nues avec l'ovalbumine libre, avec ou sans adjuvant (LNP+OVA, LNP+OVA+LPS, Figure 14). Les réponses observées sont les mêmes que pour OVA et OVA+CFA, ce qui montre que les gouttelettes nues sont très bien tolérées et n'activent pas le système immunitaire. Finalement, l'injection de - LNP-Ova conduit à une réponse immunitaire significativement plus importante que l'ovalbumine libre (LNP-OVA vs OVA, Figure 14) et du même ordre de grandeur que les réponses obtenues avec l'ovalbumine libre et adjuvants. Ce résultat démontre que la simple vectorisation de l'ovalbumine par les gouttelettes produit un effet similaire à l'ajout d'un adjuvant. Pour rappel, les LNP-Ova ne contiennent aucun adjuvant. L'ajout d'un adjuvant en plus des LNP-Ova augmente encore plus et de façon significative la réponse (LNP-OVA+LPS, Figure 14). En conclusion, ces expériences d'immunisation valident l'intérêt de la vectorisation de l'antigène ovalbumine par l'émulsion selon l'invention.

Proportion Ig total anti-OVA (ng/mL) en fonction de la composition utilisée (figure 15) Pour chaque composition, 50 µg d'ovalbumine (greffée ou non aux gouttelettes d'émulsion) ont été injectés. La quantité d'ovalbumine injectée est donc la même pour toutes les compositions injectées.
L'immunisation avec l'ovalbumine libre sans LPS a donné une réponse immunitaire faible (OVA, Figure 15).
L'ajout d'un adjuvant utilisé classiquement dans le domaine vétérinaire (CFA : adjuvant complet de Freund) à l'injection d'ovalbumine a induit une réponse immunitaire plus importante que celle de la protéine administrée seule, caractérisée par un taux plus important d'anticorps circulants anti-ovalbumine (OVA+CFA, Figure 15).
Dans tous les cas, l'injection de LNP-OVA (c'est-à-dire les émulsions selon l'invention présentant de l'ovalbumine greffée de façon covalente en surface) a provoqué une réponse immunitaire significativement plus importante que celles induites par l'ovalbumine libre ou par l'ovalbumine dans le CFA (Figure 15).
En particulier, le taux d'anticorps anti-ovalbumine le plus élevé pour une même dose administrée d'ovalbumine est observé suite à l'injection de l'émulsion F' greffée par l'ovalbumine obtenue à l'exemple 1.2.2, caractérisée notamment par une moindre variabilité inter-individuelle de réponse.
Pour les formulations de gouttelettes neutres (émulsions F et F' greffées par l'ovalbumine obtenue à l'exemple 1.2.2, exemptes de lipide cationique), la taille semble être un critère prépondérant dans le niveau de réponse induite, dans la mesure où l'émulsion F greffée par l'ovalbumine obtenue à l'exemple 1.2.2 (diamètre 157 nm) a induit une réponse significativement :
- plus importante que OVA+CFA et
- moins importante que celle obtenue avec l'émulsion F' greffée par l'ovalbumine obtenue à l'exemple 1.2.2 (diamètre 98 nm).
En revanche, les formulations de gouttelettes cationiques (émulsions G et G' greffées par l'ovalbumine obtenue à l'exemple 1.2.2, comprenant le lipide cationique DOTAP), induisent une réponse humorale de même ordre, qui est toutefois plus importante que celle obtenue avec OVA+CFA (Figure 15). Dans ce cas précis, la taille ne semble pas être un facteur déterminant.
Ces résultats démontrent que la vectorisation de l'ovalbumine par les gouttelettes des émulsions potentialise les réponses immunes. En effet, il est important d'observer que les réponses immunes induites sont très faibles lorsque la protéine antigénique OVA est administrée dans une solution contenant des gouttelettes nues (sur lesquelles OVA n'est pas greffée de façon covalente) et le LPS (LNP+OVA+LPS, figure 15), ce qui souligne l'importance de l'existence de la liaison covalente entre l'ovalbumine et les gouttelettes.

En outre, les rates des souris ont été prélevés au sacrifice, dissociées et les splénocytes ainsi récoltés ont été remis en culture et réexposés à la protéine ovalbumine. Puis, les surnageants ont été collectées et les cytokines ont été dosées par la technique CBA (pour cytometry beads assay).
Les résultats ont montré que l'émulsion F' greffée par l'ovalbumine obtenue à l'exemple 1.2.2 utilisée dans le protocole d'immunisation est celle qui induit la plus forte sécrétion de cytokines IL-17, INFγ, IL10 et TNFα, comparée aux autres d'émulsion F, G et G' greffées par l'ovalbumine obtenue à l'exemple 1.2.2 et à l'ovalbumine seule (OVA) ou ovalbumine dans le CFA (OVA+CFA) (tableau 10).
Il est intéressant de noter que la sécrétion d'IFNγ est également plus importante, comparativement à l'ovalbumine dans le CFA (OVA+CFA) lorsque l'émulsion F greffée par l'ovalbumine obtenue à l'exemple 1.2.2 a été utilisée dans le protocole d'immunisation. De façon similaire, le taux de TNFα est plus élevé lorsque l'émulsion G greffée par l'ovalbumine obtenue à l'exemple 1.2.2 a été utilisée dans le protocole d'immunisation.

**Tableau 10: Proportions de cytokines IL-17, INFγ, IL10 et TNFα mesurées dans les rates des souris**

| | OVA (comparatif) | OVA+CFA (comparatif) | OVA + LNP + LPS (comparatif) | LNP(F)-OVA + LPS | LNP(F')-OVA + LPS | LNP(G)-OVA + LPS | LNP(G')-OVA + LPS |
|---|---|---|---|---|---|---|---|
| IL-10 (pg/m L) | 22 ± 21 | 31 ± 11 | 16 ± 7 | 27 ± 11 | 54 ± 26 | 34 ± 25 | 21 ± 13 |
| IL-17 (pg/m L) | 133 ± 31 | 217 ± 177 | 16 ± 8 | 275 ± 161 | 415 ± 209 | 196 ± 144 | 180 ± 69 |
| TNFα (pg/m L) | 73 ± 36 | 151 ± 54 | 150 ± 66 | 174 ± 38 | 315 ± 126 | 324 ± 148 | 161 ± 45 |
| IFN-γ (pg/m L) | 22 ± 34 | 19 ± 20 | 4 ± 5 | 79 ± 41 | 213 ± 120 | 41 ± 31 | 35 ± 28 |

## Revendications

1. Composition immunogène comprenant une phase aqueuse continue et une phase dispersée sous forme de gouttelettes et comprenant :
- un lipide amphiphile,
- un lipide solubilisant comprenant au moins un glycéride d'acide gras,
- un co-tensioactif comprenant au moins une chaine composée de motifs d'oxyde d'alkylène,
- un tensioactif porteur d'un antigène de formule (I) suivante :
(L₁-X₁-H₁-Y₁)ᵥ-G-Z₁-Ag (I),
dans laquelle :
- L₁ représente un groupe lipophile,
- X₁, Y₁, Z₁ et G représentent indépendamment un groupe de liaison,
- H₁ représente un groupe hydrophile comprenant une chaîne polyalcoxylée,
- v est un nombre entier de 1 à 8, et
- Ag représente un antigène,
dans laquelle le rapport molaire du tensioactif porteur d'un antigène de formule (I) sur la somme du co-tensioactif et du tensioactif porteur d'un antigène de formule (I) est de 0,01% à 5%.

2. Composition immunogène selon la revendication 1, dans laquelle, dans la formule (I) :
- L₁ est choisi parmi :
- un groupe R ou R-(C=O)-, où R représente une chaîne hydrocarbonée linéaire comprenant de 7 à 23 atomes de carbone,
- un ester ou un amide d'acides gras comprenant de 8 à 24 atomes de carbone et de phosphatidyléthanolamine, tel que le distéaryl phosphatidyléthanolamine (DSPE), et
- un poly(oxyde de propylène), et/ou
- X₁, Y₁ et Z₁ sont indépendamment choisis parmi :
- une liaison simple,
- un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH,
- un groupe Alk étant un alkylène comprenant de 1 à 8 atomes de carbone comprenant éventuellement un cycle (par exemple un radical 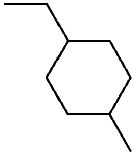 ), et
- un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents, et/ou
- H₁ est choisi parmi un poly(oxyde d'éthylène) comprenant typiquement de 3 à 500 unités oxyde d'éthylène, de préférence de 20 à 200 unités oxyde d'éthylène, et/ou
- G comprend au moins un groupe G' ayant l'une des formules suivantes : 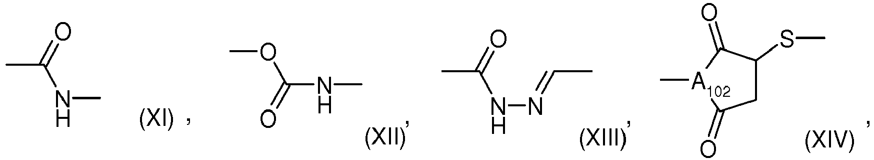 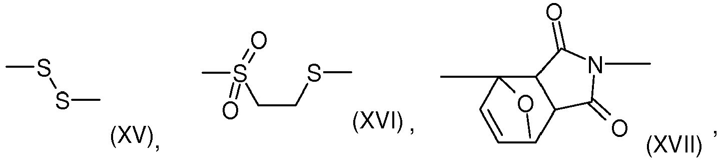 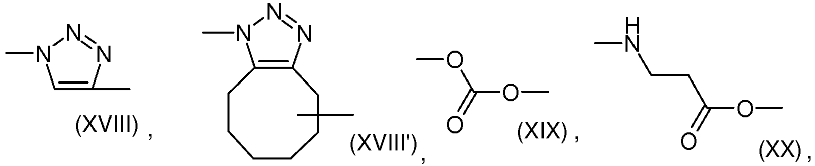 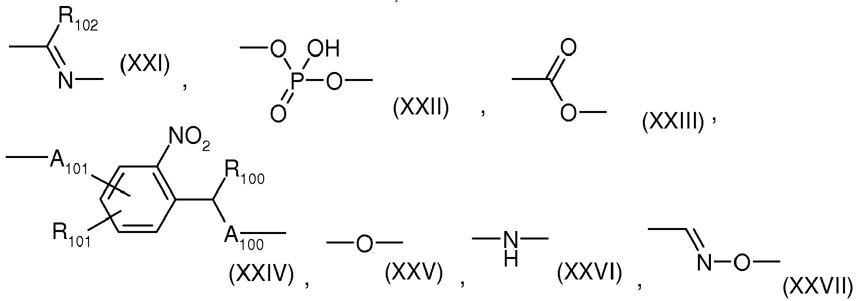 où A₁₀₂ représente CH ou N, R₁₀₂ représente H ou une chaîne hydrocarbonée linéaire comprenant de 1 à 6 atomes de carbone, A₁₀₁ représente -O-, -NH-(CO)- ou -O-(CO)-, R₁₀₀ représente H ou un méthyle, A₁₀₀ représente -O- ou -NH- et R₁₀₁ représente H, Me ou -OMe.

3. Composition immunogène selon la revendication 1 ou 2, dans laquelle, dans la formule (I), le radical L₁-X₁-H₁- consiste en l'un des groupes de formules suivantes : 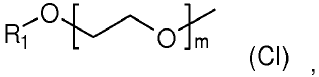 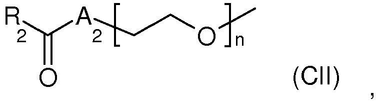 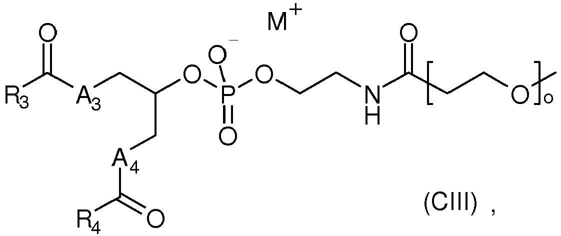 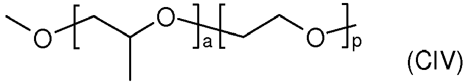 dans lesquelles :
- R₁, R₂, R₃ et R₄ représentent indépendamment une chaîne hydrocarbonée linéaire comprenant de 7 à 23 atomes de carbone,
- A₁, A₂, A₃ et A₄ représentent O ou NH,
- m, n, o et p représentent indépendamment des nombres entiers de 3 à 500, de préférence 20 à 200, et
- a représente un nombre entier de 20 à 120,
- M représente H ou un cation.

4. Composition immunogène selon l'une quelconque des revendications 1 à 3, dans laquelle le tensioactif de formule (I) a la formule (I') suivante : 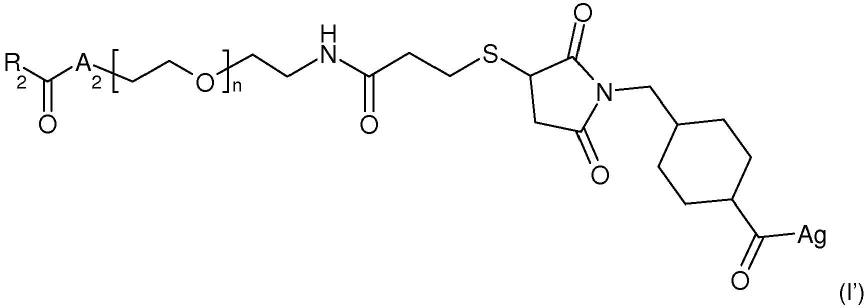 dans laquelle :
- R₂ représente une chaîne hydrocarbonée linéaire comprenant de 7 à 23 atomes de carbone, de préférence 17,
- A₂ représente O ou NH, de préférence NH, et
- n représente un nombre entier de 3 à 500, de préférence de 20 à 200, notamment 100,
- Ag représente un antigène.

5. Composition immunogène selon l'une quelconque des revendications 1 à 4, dans laquelle :
- le lipide amphiphile est un phospholipide, et/ou
- le lipide solubilisant est constitué d'un mélange de glycérides d'acides gras saturés comportant au moins 10% en poids d'acides gras en C12, au moins 5% en poids d'acides gras en C14, au moins 5% en poids d'acides gras en C16 et au moins 5% en poids d'acides gras en C18, et/ou
- le co-tensioactif est choisi parmi les composés conjugués polyéthylèneglycol/phosphatidyl-éthanolamine, les éthers d'acide gras et de polyéthylèneglycol, les esters d'acide gras et de polyéthylèneglycol et les copolymères blocs d'oxyde d'éthylène et d'oxyde de propylène, et la chaîne polyalcoxylée du co-tensioactif comprend de 10 à 200 motifs oxyde d'éthylène/oxyde de propylène.

6. Composition immunogène selon l'une quelconque des revendications 1 à 5, comprenant un agent immunostimulant.

7. Composition immunogène selon l'une quelconque des revendications 1 à 6, comprenant :
- un ligand biologique de ciblage greffé ou non sur le co-tensioactif, et/ou
- un agent d'intérêt choisi parmi un agent optique tel que un colorant, un chromophore, un fluorophore ou un agent physique, tel qu'un isotope radioactif ou un photo-sensibilisateur, et/ou
- un tensioactif cationique, notamment choisi parmi choisi parmi le *N*[1-(2,3-dioléyloxy) propyl]-*N*,*N*,*N*triméthylammonium, le 1,2-dioleyl-3-trimethylamonium-propane, le *N*-(2-hydroxyethyl)-*N*,*N*-dimethyl-2,3-bis(tetradecyloxy-1-propananium), le 1-[2-(oleoyloxy)ethyl]-2-oleyl-3-(2-hydroxyethyl)imidazolinium, et le dioctadecylamidoglycylspermine.

8. Composition immunogène selon l'une quelconque des revendications 1 à 7, dont la viscosité est supérieure à 0,1 Pa.s.

9. Procédé de préparation de la composition immunogène telle que définie dans l'une quelconque des revendications 1 à 8, comprenant la mise en contact :
- d'une émulsion prémix comprenant une phase aqueuse continue et une phase dispersée sous forme de gouttelettes, comprenant un lipide amphiphile, un lipide solubilisant comprenant au moins un glycéride d'acide gras, un co-tensioactif comprenant au moins une chaine composée de motifs d'oxyde d'alkylène et un tensioactif de formule (LI) suivante :
L₁-X₁-H₁-Y₁-G₁ (LI),
dans laquelle le rapport molaire du tensioactif de formule (LI) sur la somme du co-tensioactif et du tensioactif de formule (LI) est de 0,01% à 5%,
- avec un composé de formule (LII) suivante :
G₂-Z₁-Ag (LII)
où L₁, X₁, H₁, Y₁, Z₁ et Ag sont tels que définis dans l'une quelconque des revendications 1 à 3, et G₁ et G₂ sont des groupes susceptibles de réagir ensemble pour former le groupe G tel que défini dans la revendications 1 ou 2,
dans des conditions permettant la réaction du tensioactif de formules (LI) avec le composé de formule (LII) pour former le tensioactif porteur d'un antigène de formule (I) tel que défini dans l'une quelconque des revendications 1 à 4.

10. Procédé de préparation selon la revendication 9, comprenant préalablement à la mise en contact de l'émulsion prémix et du composé de formule (LII) pour former le tensioactif porteur d'un antigène de formule (I), une étape de préparation du composé de formule (LII) par modification chimique d'un antigène pour y greffer le groupe G₂.

11. Procédé de préparation selon la revendication 10 d'une composition immunogène selon la revendication 4, comprenant :
- la préparation du composé de formule (LII') par modification chimique d'un antigène porteur d'une fonction amine -NH₂ par réaction avec du (Sulfosuccinimidyl-4-N-maleimidomethyl) cyclohexane-1-carboxylate) (Sulfo-SMCC) selon le schéma réactionnel suivant : 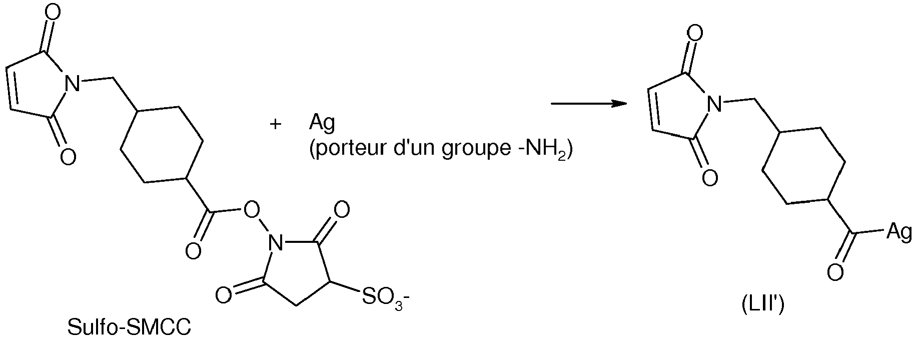
- puis la mise en contact :
- d'une émulsion prémix comprenant une phase aqueuse continue et une phase dispersée sous forme de gouttelettes, comprenant un lipide amphiphile, un lipide solubilisant comprenant au moins un glycéride d'acide gras, un co-tensioactif comprenant au moins une chaine composée de motifs d'oxyde d'alkylène et un tensioactif de formule (LI') suivante : 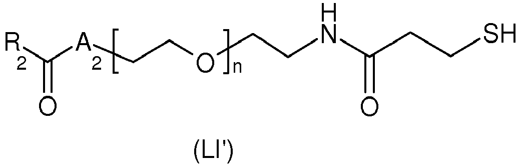 dans laquelle le rapport molaire du tensioactif de formule (LI') sur la somme du co-tensioactif et du tensioactif de formule (LI') est de 0,01% à 5%,
- avec le composé de formule (LII'),
où R₂, A₂, n et Ag sont tels que définis dans la revendication 4.

12. Composition immunogène selon l'une quelconque des revendications 1 à 8 pour son utilisation en tant que médicament.

13. Composition immunogène selon l'une quelconque des revendications 1 à 8 pour induire une réponse immune contre ledit antigène.

14. Composition immunogène pour son utilisation selon la revendication 13, pour traiter ou prévenir une infection, un cancer, une maladie inflammatoire ou une allergie.

15. Composition immunogène pour son utilisation selon la revendication 14, dans laquelle ladite infection est une infection virale, bactérienne ou parasitaire.

16. Méthode de production d'anticorps polyclonaux, comprenant les étapes consistant en :
(a) l'immunisation d'un animal avec une composition immunogène telle que définie dans l'une quelconque des revendications 1 à 8, de manière à induire une réponse immune humorale contre ledit antigène, et
(b) la récolte des anticorps polyclonaux induits dirigés contre ledit antigène.

17. Méthode de production d'anticorps monoclonaux, comprenant les étapes consistant en :
(i) l'immunisation d'un animal avec une composition immunogène telle que définie dans l'une quelconque des revendications 1 à 8,
(ii) récupération et isolement des lymphocytes B de l'animal immunisé à l'étape (i),
(iii) production d'un hybridome et culture dudit l'hybridome afin de produire des anticorps monoclonaux dirigés contre l'antigène présent dans ladite composition immunogène,
(iv) récolte et purification des anticorps monoclonaux produits à l'étape (iii).

## Patentansprüche

1. Immunogene Zusammensetzung umfassend eine kontinuierliche wässrige Phase und eine disperse Phase als Tröpfchen und umfassend:
- ein amphiphiles Lipid,
- ein löslich machendes Lipid umfassend mindestens ein Fettsäureglycerid,
- ein Co-Tensid umfassend mindestens ein Kette bestehend aus Alkylenoxid-Einheiten,
- ein Tensid, welches ein Antigen der folgenden Formel (I) trägt:
(L₁-X₁-H₁-Y₁)ᵥ-G-Z₁-Ag (I),
wobei:
- L₁ für eine lipophile Gruppe steht,
- X₁, Y₁, Z₁ und G unabhängig voneinander für eine bindende Gruppe stehen,
- H₁ für eine hydrophile Gruppe, umfassend eine polyalkoxylierte Gruppe, steht,
- v eine ganze Zahl von 1 bis 8 ist, und
- Ag für ein Antigen steht,
wobei das Molverhältnis des Tensids, welches ein Antigen der Formel (I) trägt, zu der Summe aus Co-Tensid und Tensid, welches ein Antigen der Formel (I) trägt, von 0.01% bis 5% ist.

2. Immunogene Zusammensetzung gemäß Anspruch 1, wobei, in Formel (I):
- L₁ ausgewählt ist aus:
- eine Gruppe R oder R-(C=O)-, wobei R einer linearen Kohlenwasserstoffkette entspricht umfassend 7 bis 23 Kohlenstoffatome,
- ein Fettsäureester oder -amid umfassend 8 bis 24 Kohlenstoffatome und Phosphatidylethanolamin, wie zum Beispiel Distearylphosphatidylethanolamin (DSPE), und
- ein Poly(propylenoxid), und/oder
- X₁, Y₁ und Z₁ unabhängig voneinander auswählt sind aus:
- einer Einfachbindung;
- einer Gruppe Z ausgewählt aus -O-, -NH-, -O(OC)-, -(CO)O-, -(CO)NH-, - NH(CO)-, -O-(CO)-O-, -NH-(CO)-O-, -O-(CO)-NH- und -NH-(CO)-NH,
- einer Alk Gruppe bestehend aus einem Alkylen umfassend 1-8 Kohlenstoffatome, die gegebenenfalls einen Ring umfassen (z.B. ein Radikal 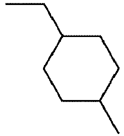 ), und
- einer Gruppe Z-Alk, Alk-Z, Alk-Z-Alk oder Z-Alk-Z, wobei Alk und Z so wie oben definiert sind, und wobei die beiden Gruppen Z der Gruppe Z-Alk-Z identisch oder verschieden sind, und/oder
- H₁ ausgewählt ist aus einem Poly(Ethylenoxid) umfassend typischerweise von 3 bis 500 Einheiten Ethylenoxid, vorzugsweise von 20 bis 200 Einheiten Ethylenoxid, und/oder
- G mindestens eine Gruppe G' mit einer der folgenden Formeln umfasst: 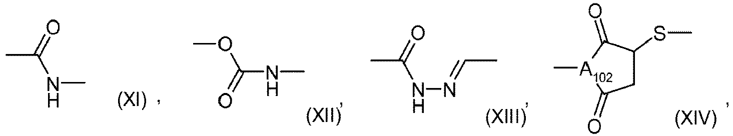 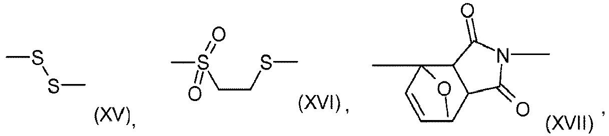 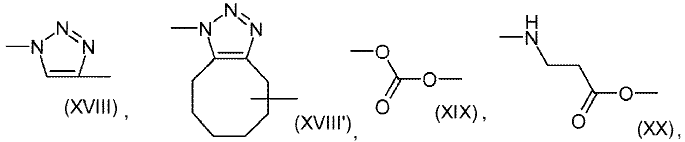 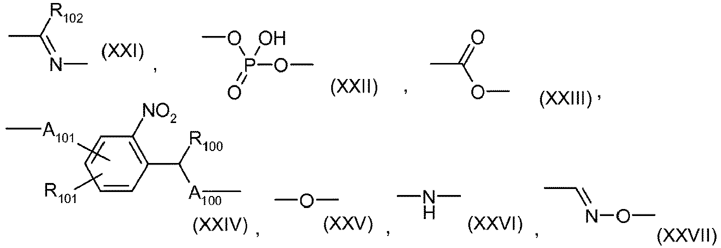 wobei A₁₀₂ CH oder N entspricht, R₁₀₂ H oder einer linearen Kohlenwasserstoffkette umfassend 1 bis 6 Kohlenstoffatomen entspricht, A₁₀₁-O-, NH-(CO)- oder -O-(CO)- entspricht, R₁₀₀ H oder Methyl entspricht, A₁₀₀ -O- oder -NH- entspricht, und R₁₀₁ H, Me oder -OMe entspricht.

3. Die immunogene Zusammensetzung gemäß Anspruch 1 oder 2, wobei, in Formel (I), das Radikal L₁-X₁-H₁- aus einer der Gruppen der folgenden Formel besteht: 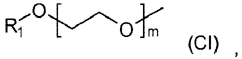 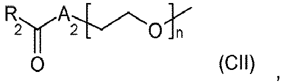 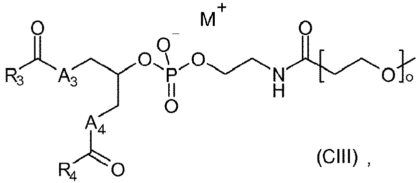 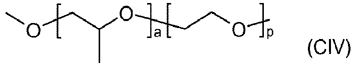 wobei:
- R₁, R₂, R₃ und R₄ unabhängig voneinander für eine lineare Kohlenwasserstoffkette umfassend 7 bis 23 Kohlenstoffatome stehen,
- A₁, A₂, A₃ und A₄ für O oder NH stehen,
- m, n, o und p unabhängig voneinander für ganze Zahlen von 3 bis 500, vorzugsweise von 20 bis 200, stehen, und
- a für eine ganze Zahl von 20 bis 120 steht,
- M für H oder ein Kation steht.

4. Die immunogene Zusammensetzung gemäß einem der Ansprüche 1 bis 3, wobei das Tensid der Formel (I) die folgende Formel (I') hat: 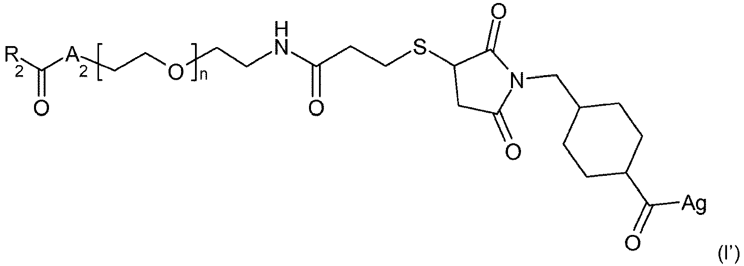 wobei:
- R₂ für eine lineare Kohlenwasserstoffkette steht, umfassend 7 bis 23, vorzugsweise 17, Kohlenstoffatome,
- A₂ für O oder NH steht, vorzugsweise NH, und
- n für eine ganze Zahl von 3 bis 500, vorzugsweise 20 bis 200, steht, insbesondere 100,
- Ag für ein Antigen steht.

5. Die immunogene Zusammensetzung gemäß einem der Ansprüche 1 bis 4, wobei:
- das amphiphile Lipid ein Phospholipid ist, und/oder
- das löslich machende Lipid aus einer Mischung von gesättigten Fettsäureglyceriden besteht, die mindestens 10 Gewichtsprozente an C₁₂-Fettsäuren, mindestens 5 Gewichtsprozente C₁₄-Fettsäuren, mindestens 5 Gewichtsprozente C₁₆-Fettsäuren und mindestens 5 Gewichtsprozente C₁₈-Fettsäuren enthalten, und/oder
- das Co-Tensid ausgewählt ist aus konjugierten Polyethylenglycol/PhosphatidylEthanolamin-Konjugatverbindungen, Fettsäure- und Polyethylenglycol-Ethern, Fettsäure- und Polyethylenglycol-Estern und Blockcopolymeren von Ethylenoxid und Propylenoxid, und die polyalkoxylierte Kette des Co-Tensids 10 bis 200 Ethylenoxid/Propylenoxid-Einheiten umfasst.

6. Die immunogene Zusammensetzung gemäß einem der Ansprüche 1 bis 5, umfassend ein immunstimulierendes Mittel.

7. Die immunogene Zusammensetzung gemäß einem der Ansprüche 1 bis 6, umfassend:
- einen auf das Co-Tensid aufgepfropften oder nicht aufgepfropften biologischen Ziel-Ligand, und/oder
- ein Mittel von Interesse, ausgewählt aus einem optischen Mittel wie z.B. ein Färbemittel, ein Chromophor, ein Fluorophor oder einem physikalischen Mittel, wie zum Beispiel einem radioaktiven Isotop oder einem Photosensibilisator, und/oder
- ein kationisches Tensid, ausgewählt insbesondere aus *N*[1-(2,3-Dioleyloxy) propyl]-*N, N, N*-trimethylammonium, 1,2-Dioleyl-3-trimethylamoniumpropan, *N-*(2-Hydroxyethyl)-*N,N*-dimethyl-2,3-bis(tetradecyloxy-1-propananium), 1-[2-(Oleoyloxy)ethyl]-2-oleyl-3-(2-hydroxyethyl)imidazolinium, und Dioctadecylamidoglycylspermin.

8. Die immunogene Zusammensetzung gemäß einem der Ansprüche 1 bis 7, deren Viskosität mehr als 0,1 Pa.s beträgt.

9. Ein Verfahren zur Herstellung der immunogenen Zusammensetzung wie definiert in einem der Ansprüche 1 bis 8, umfassend das in Kontakt bringen:
- einer Prämix-Emulsion, umfassend eine kontinuierliche wässrige Phase und eine disperse Phase in Form von Tröpfchen, umfassend ein amphiphiles Lipid, ein löslich machendes Lipid, umfassend mindestens ein Fettsäureglycerid, ein Co-Tensid umfassend mindestens eine Kette zusammengesetzt aus Alkylenoxidelementen und einem Tensid mit folgender Formel (LI):
L₁-X₁-H₁-Y₁-G₁ (LI),
wobei das Molverhältnis des Tensids der Formel (LI) zu der Summe aus Co-Tensid und Tensid der Formel (LI) 0,01 % bis 5 % beträgt,
- mit einer Verbindung der folgenden Formel (LII):
G₂-Z₁-Ag (LII),
- wobei L₁, X₁, H₁, Y₁, Z₁ und Ag definiert sind wie in einem der Ansprüche 1 bis 3, und G₁ und G₂ Gruppen sind, die geeignet sind, miteinander zu reagieren und die Gruppe G bilden können, definiert wie in Anspruch 1 oder 2,
- unter Bedingungen, die die Reaktion des Tensids der Formel (LI) mit dem Stoff der Formel (LII) erlauben, zur Bildung des Tensids, welches ein Antigen der Formel (I) trägt, wie in einem der Ansprüche 1 bis 4 definiert.

10. Verfahrens zur Herstellung gemäß Anspruch 9, umfassend einen Schritt der Herstellung der Verbindung der Formel (LII) durch chemische Modifizierung eines Antigens, um darauf Gruppe G₂ zu pfropfen, vor dem In-Kontakt-bringen der Prämix-Emulsion und der Verbindung der Formel (LII) zur Bildung des Tensids, welches ein Antigen der Formel (I) trägt.

11. Verfahren zur Herstellung gemäß Anspruch 10 einer immunogenen Zusammensetzung gemäß Anspruch 4, umfassend:
- die Vorbereitung der Verbindung der Formel (LII') durch chemische Modifizierung eines Antigens, welches eine Aminofunktion -NH₂ trägt, durch Reaktion mit (Sulfosuccinimidyl-4-N-maleimidomethyl)cyclohexan-1-carboxylat) (Sulfo-SMCC) gemäß folgendem Reaktionsschema: 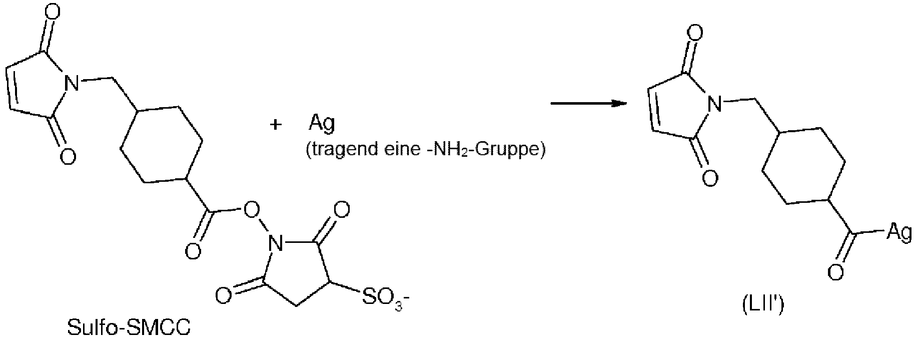
- dann das In-Kontaktbringen
- einer Prämix-Emulsion, umfassend eine kontinuierliche wässrige Phase und eine disperse Phase in Form von Tröpfchen, umfassend ein amphiphiles Lipid, ein löslich machendes Lipid, umfassend mindestens ein Fettsäureglycerid, ein Co-Tensid, umfassend mindestens eine Kette bestehend aus Alkylenoxidelementen und ein Tensid mit folgender Formel (LI'): 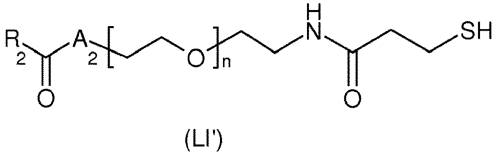 wobei das Molverhältnis des Tensids der Formel (LI') zu der Summe aus Co-Tensids und Tensid der Formel (LI') 0,01 % bis 5 % beträgt,
- mit einer Verbindung der Formel (LII'):
G₂-Z₁-Ag (LII),
wobei R₂, A₂, n und Ag definiert sind wie in Anspruch 4.

12. Immunogene Zusammensetzung gemäß einem der Ansprüche 1 bis 8 zur Verwendung als Medikament.

13. Immunogene Zusammensetzung gemäß einem der Ansprüche 1 bis 8, um eine Immunreaktion gegen das Antigen hervorzurufen.

14. Immunogene Zusammensetzung zur Verwendung gemäß Anspruch 13, um eine Infektion, Krebs, eine entzündliche Krankheit oder eine Allergie zu behandeln oder zu verhüten.

15. Immunogene Zusammensetzung zur Verwendung gemäß Anspruch 14, wobei die Infektion eine Virusinfektion, eine bakterielle Infektion oder Parasitenbefall ist.

16. Verfahren zur Herstellung polyklonaler Antikörper, umfassend die Schritte bestehend aus:
(a) Immunisierung eines Tieres mit einer immunogenen Zusammensetzung, definiert wie in einem der Ansprüche 1 bis 8, um eine humorale Immunreaktion gegen das Antigen herbeizuführen, und
(b) Gewinnen der induzierten polyklonalen Antikörper, die gegen das Antigen gerichtet sind.

17. Verfahren zur Herstellung monoklonaler Antikörper, umfassend die Schritte bestehend aus:
(i) Immunisierung eines Tieres mit einer immunogenen Zusammensetzung, definiert wie in einem der Ansprüche 1 bis 8,
(ii) Rückgewinnung und Isolierung der B-Lymphozyten des in Schritt (i) immunisierten Tieres,
(iii) Herstellung eines Hybridoms und Kultur des Hybridoms für die Produktion von monoklonalen Antikörpern, die gegen das Antigen gerichtet sind, welches in der immunogenen Zusammensetzung vorhanden ist,
(iv) Gewinnung und Reinigung der in Schritt (iii) produzierten monoklonalen Antikörper.

## Claims

1. An immunogenic composition comprising a continuous aqueous phase and a dispersed phase as droplets and comprising:
- an amphiphilic lipid,
- a solubilizing lipid comprising at least one fatty acid glyceride,
- a co-surfactant comprising at least one chain consisting of alkylene oxide units,
- a surfactant bearing an antigen of the following formula (I):
(L₁-X₁-H₁-Y₁)ᵥ-G-Z₁-Ag (I),
wherein:
- L₁ represents a lipophilic group,
- X₁, Y₁, Z₁ and G represent independently a binding group,
- H₁ represents a hydrophilic group comprising a polyalkoxylated chain,
- v is an integer from 1 to 8, and
- Ag represents an antigen,
wherein the molar ratio of the surfactant bearing an antigen of formula (I) over the sum of the co-surfactant and of the surfactant bearing an antigen of formula (I) is from 0.01% to 5%.

2. The immunogenic composition according to claim 1, wherein, in formula (I):
- L₁ is selected from:
- a group R or R-(C=O)-, wherein R represents a linear hydrocarbon chain comprising from 7 to 23 carbon atoms,
- an ester or amide of fatty acids comprising from 8 to 24 carbon atoms and phosphatidylethanolamine, such as distearyl phosphatidylethanolamine (DSPE), and
- a poly(propylene oxide), and/or
- X₁, Y₁ and Z₁ are independently selected from:
- a single bond,
- a group Z selected from -O-, -NH-, -O(OC)-, -(CO)O-, -(CO)NH-, -NH(CO)-, -O-(CO)-O-, -NH-(CO)-O-, -O-(CO)-NH- and -NH-(CO)-NH,
- a group Alk being an alkylene comprising from 1 to 8 carbon atoms optionally comprising a ring (for example a radical 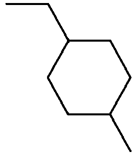 ), and
- a group Z-Alk, Alk-Z, Alk-Z-Alk or Z-Alk-Z wherein Alk and Z are as defined above and wherein both groups Z of the group Z-Alk-Z are identical or different, and/or
- H₁ is selected from a poly(ethylene oxide) typically comprising from 3 to 500 ethylene oxide units, preferably from 20 to 200 ethylene oxide units, and/or
- G comprises at least one group G' having one of the following formulae: 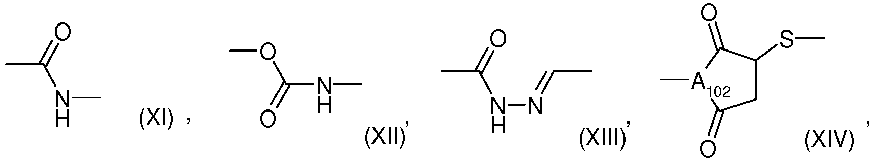 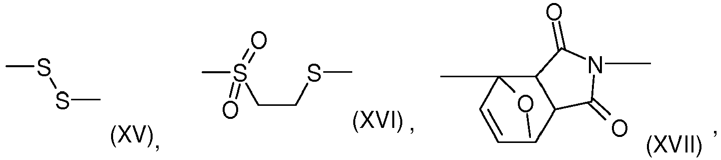 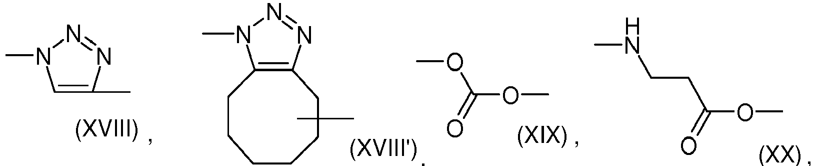 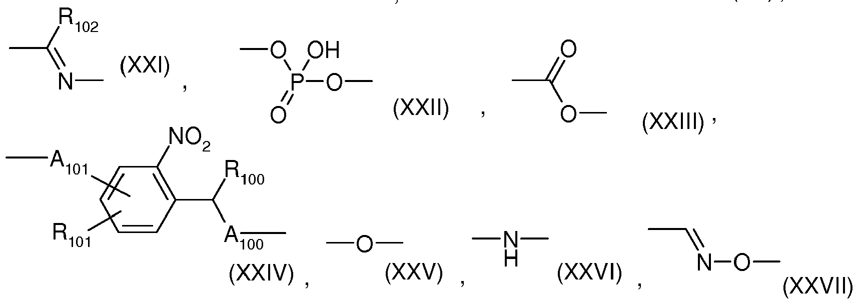 wherein A₁₀₂ represents CH or N, R₁₀₂ represents H or a linear hydrocarbon chain comprising from 1 to 6 carbon atoms, A₁₀₁ represents -O-, -NH-(CO)- or -O-(CO)-, R₁₀₀ represents H or a methyl, A₁₀₀ represents -O- or -NH- and R₁₀₁ represents H, Me or -OMe.

3. The immunogenic composition according to claim 1 or 2, wherein, in formula (I), the radical L₁-X₁-H₁- consists in one of the groups of the following formulae: 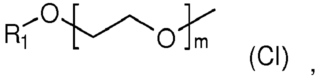 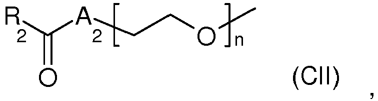 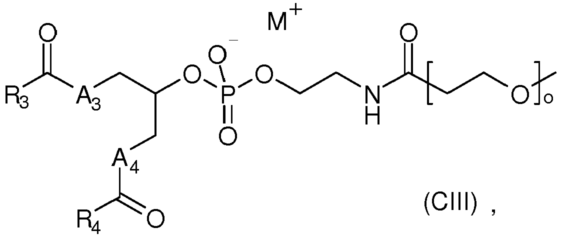 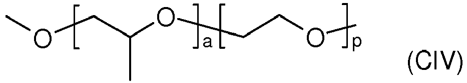 wherein:
- R₁, R₂, R₃ and R₄ represent independently a linear hydrocarbon chain comprising from 7 to 23 carbon atoms,
- A₁, A₂, A₃ and A₄ represent O or NH,
- m, n, o and p represent independently integers from 3 to 500, preferably 20 to 200, and
- a represents an integer from 20 to 120,
- M represents H or a cation.

4. The immunogenic composition according to any of claims 1 to 3, wherein the surfactant of formula (I) has the following formula (I'): 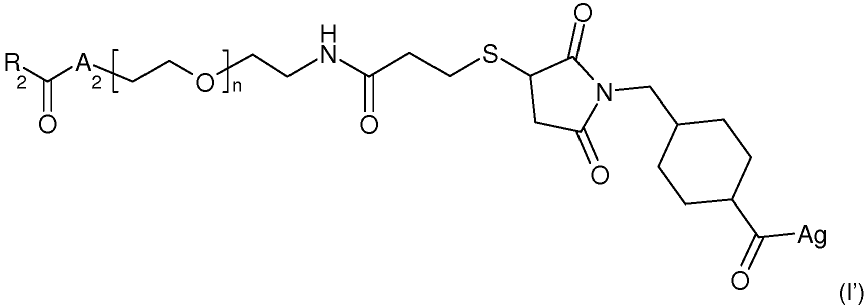 wherein:
- R₂ represents a linear hydrocarbon chain comprising from 7 to 23 carbon atoms, preferably 17,
- A₂ represents O or NH, preferably NH, and
- n represents an integer from 3 to 500, preferably from 20 to 200, notably 100,
- Ag represents an antigen.

5. The immunogenic composition according to any of claims 1 to 4, wherein:
- the amphiphilic lipid is a phospholipid, and/or
- the solubilizing lipid consists of a mixture of saturated fatty acid glycerides including at least 10% by weight of C₁₂ fatty acids, at least 5% by weight of C₁₄ fatty acids, at least 5% by weight of C₁₆ fatty acids and at least 5% by weight of C18 fatty acids, and/or
- the co-surfactant is selected from polyethyleneglycol/phosphatidyl-ethanolamine conjugate compounds, fatty acid and polyethyleneglycol ethers, fatty acid and polyethyleneglycol esters and block copolymers of ethylene oxide and propylene oxide, and the polyalkoxylated chain of the co-surfactant comprises from 10 to 200 ethylene oxide/propylene oxide units.

6. The immunogenic composition according to any of claims 1 to 5, comprising an immunostimulating agent.

7. The immunogenic composition according to any of claims 1 to 6, comprising:
- a biological targeting ligand either grafted or not on the co-surfactant, and/or
- an agent of interest selected from an optical agent such as a coloring agent, a chromophore, a fluorophore or a physical agent, such as a radio-active isotope or a photo-sensitizer, and/or
- a cationic surfactant, notably selected from among *N*[1-(2,3-dioleyloxy)propyl]-*N*,*N*,*N-*trimethylammonium, 1,2-dioleyl-3-trimethylamonium-propane, *N*-(2-hydroxyethyl)-*N*,*N*-dimethyl-2,3-bis(tetradecyloxy-1-propananium), le 1-[2-(oleoyloxy)ethyl]-2-oleyl-3-(2-hydroxyethyl)imidazolinium, and dioctadecylamidoglycylspermine.

8. The immunogenic composition according to any of claims 1 to 7, for which the viscosity is more than 0,1 Pa.s.

9. A method for preparing the immunogenic composition as defined in any of claims 1 to 8, comprising the putting into contact:
- of a premix emulsion comprising a continuous aqueous phase and a dispersed phase as droplets, comprising an amphiphilic lipid, a solubilizing lipid comprising at least one fatty acid glyceride, a co-surfactant comprising at least one chain consisting of alkylene oxide units and a surfactant of the following formula (LI):
L₁-X₁-H₁-Y₁-G₁ (LI),
wherein the molar ratio of the surfactant of formula (LI) over the sum of the co-surfactant and of the surfactant of formula (LI) is from 0.01% to 5%,
- with a compound of the following formula (LII):
G₂-Z₁-Ag (LII)
wherein L₁, X₁, H₁, Y₁, Z₁ and Ag are as defined in any of claims 1 to 3, and G₁ and G₂ are groups which may react together in order to form the group G as defined in claims 1 or 2, under conditions allowing the reaction of the surfactant of formulae (LI) with the compound of formula (LII) in order to form the surfactant bearing an antigen of formula (I) as defined in any of claims 1 to 4.

10. The preparation method according to claim 9, comprising prior to the contacting of the premix emulsion and of the compound of formula (LII) in order to form the surfactant bearing an antigen of formula (I), a step for preparing the compound of formula (LII) by chemically modifying an antigen for grafting thereon the group G₂.

11. The method according to claim 10 for preparing the immunogenic composition according to claim 4, comprising:
- the preparation of the compound of formula (LII') by chemical modification of an antigen bearing a amine function -NH₂ by reaction with (sulfosuccinimidyl-4-N-maleimidomethyl)cyclohexane-1-carboxylate) (Sulfo-SMCC) according to the following reaction scheme: 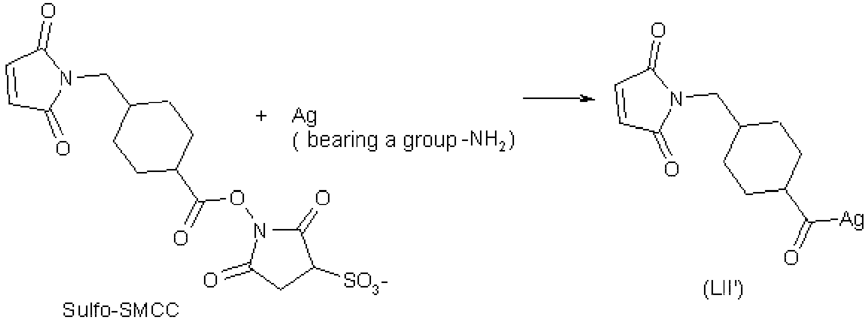
- and then the contacting:
- of a premix emulsion comprising a continuous aqueous phase and a dispersed phase as droplets, comprising an amphiphilic lipid, a solubilizing lipid comprising at least one fatty acid glyceride, a co-surfactant comprising at least one chain consisting of alkylene oxide units and a surfactant of the following formula (LI'): 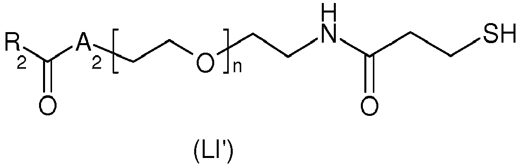 wherein the molar ration of the surfactant of formula (LI') over the sum of the co-surfactant and of the surfactant of formula (LI') is from 0.01% to 5%,
- with the compound of formula (LII'),
wherein R₂, A₂, n and Ag are as defined in claim 4.

12. The immunogenic composition according to any of claims 1 to 8 for its use as a drug.

13. The immunogenic composition according to any of claims 1 to 8 for inducing an immune response against said antigen.

14. The immunogenic composition for its use according to claim 13, for treating or preventing an infection, a cancer, an inflammatory disease or an allergy.

15. The immunogenic composition for its use according to claim 14, wherein said infection is a viral, bacterial or parasite infection.

16. A method for producing polyclonal antibodies, comprising the steps consisting in:
(a) immunizing an animal with an immunogenic composition as defined according to any of claims 1 to 8, so as to induce a humoral immune response against said antigen, and
(b) harvesting the induced polyclonal antibodies directed against said antigen.

17. A method for producing monoclonal antibodies, comprising the steps consisting in:
(i) immunizing an animal with an immunogenic composition as defined according to any of claims 1 to 8,
(ii) recovering and isolating the B lymphocytes of the immunized animal in step (i),
(iii) producing a hybridoma and cultivating said hybridoma in order to produce monoclonal antibodies directed against the antigen present in said immunogenic composition,
(iv) harvesting and purifying the monoclonal antibodies produced in step (iii).


### SEQUENCE LISTING

<110> Commissariat à l'Energie Atomique et aux Energies Alternatives (C.E.A.)
<120> Composition immunogène sous forme d'émulsion
<130> BET14P0286
<160> 4
<170> PatentIn version 3.5
<210> 1
   <211> 8
   <212> PRT
   <213> Artificial Sequence
<220>
   <223> Acides aminés 257 à 264 de l'ovalbumine
<400> 1
   Ser Ile Ile Asn Phe Glu Lys Leu
   1               5
<210> 2
   <211> 17
   <212> PRT
   <213> Artificial Sequence
<220>
   <223> Acides aminés 323 à 339 de l'ovalbumine
<400> 2
   Ile Ser Gln Ala Val His Ala Ala His Ala Glu Ile Asn Glu Ala Gly
   1               5                   10                  15
   Arg
<210> 3
   <211> 9
   <212> PRT
   <213> Artificial Sequence
<220>
   <223> Peptide de l'ovalbumine modifié
<220>
   <221> MISC_FEATURE
   <222> (1)..(1)
   <223> Xaa est une maléimide-sérine
<220>
   <221> MISC_FEATURE
   <222> (9)..(9)
   <223> Xaa est une lysine-6carboxyfluorescine
<400> 3
   Xaa Ile Ile Asn Phe Glu Lys Leu Xaa
   1               5
<210> 4
   <211> 17
   <212> PRT
   <213> Artificial Sequence
<220>
   <223> Peptide de l'ovalbumine modifié
<220>
   <221> MISC_FEATURE
   <222> (1)..(1)
   <223> Xaa est une maléimide-isoleucine
<220>
   <221> MISC_FEATURE
   <222> (17)..(17)
   <223> Xaa est une arginine-carboxytetramethylrhodamine
<400> 4
   Xaa Ser Gln Ala Val His Ala Ala His Ala Glu Ile Asn Glu Ala Gly
   1               5                   10                  15
   Xaa